(19) Europäisches Patentamt
European Patent Office
Office européen des brevets

(11) **EP 4 674 480 A2**

(12) # EUROPEAN PATENT APPLICATION

(43) Date of publication:
**07.01.2026 Bulletin 2026/02**

(21) Application number: **25196854.1**

(22) Date of filing: **01.10.2021**

(51) International Patent Classification (IPC):
**A61P 9/00** *(2006.01)*

(52) Cooperative Patent Classification (CPC):
**A61K 38/1796; A61P 9/00; C07K 14/71;**
C07K 2319/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 02.10.2020 US 202063086858 P
02.10.2020 US 202063086894 P
02.10.2020 US 202063086860 P
09.11.2020 US 202063111337 P
09.11.2020 US 202063111476 P
09.11.2020 US 202063111460 P
04.03.2021 US 202163156870 P
19.03.2021 US 202163163655 P

(62) Document number(s) of the earlier application(s) in accordance with Art. 76 EPC:
**21876630.1 / 4 221 736**

(71) Applicant: **Keros Therapeutics, Inc.**
**Lexington, MA 02421 (US)**

(72) Inventors:
• **SEEHRA,, Jasbir, S.**
**Lexington, 02421 (US)**
• **ROVALDI,, Christopher, R.**
**Swampscott, 01907 (US)**

(74) Representative: **Carpmaels & Ransford LLP**
**One Southampton Row**
**London WC1B 5HA (GB)**

Remarks:
•The complete document including Reference Table(s) and the Sequence Listing(s) can be downloaded from the EPO website
•This application was filed on 19-08-2025 as a divisional application to the application mentioned under INID code 62.
•Claims filed after the date of receipt of the divisional application (Rule 68(4) EPC)

(54) **METHODS OF USING ACTIVIN RECEPTOR TYPE II VARIANTS**

(57) The invention features polypeptides that include an extracellular ActRII variant, such as an ActRIIA variant, ActRIIB variant, or ActRII chimera. In some embodiments, a polypeptide of the invention includes an extracellular ActRII variant fused to an Fc domain monomer or moiety. The invention also features pharmaceutical compositions containing said polypeptides and methods of using the polypeptides to treat diseases and conditions that can be treated with erythropoietin or an erythropoiesis-stimulating agent.

EP 4 674 480 A2

**Description**

**SEQUENCE LISTING**

The instant application contains a Sequence Listing which has been submitted electronically in ASCII format and is hereby incorporated by reference in its entirety. The ASCII copy, created on October 1, 2021, is named 51184-026WO4_Sequence_Listing_10_1_21_ST25 and is 546,175 bytes in size.

**BACKGROUND OF THE INVENTION**

Erythropoietin (EPO) is a hormone secreted primarily by the kidney, typically in response to low oxygen levels. It is known to promote the production of erythrocytes, but has also been found to stimulate the mobilization, proliferation, and differentiation of endothelial progenitor cells, to improve gastrointestinal dysmotility, to have broad neuroprotective and anti-inflammatory capabilities, and to play a central role in tissue protection and restoration. Recombinant EPO and EPO mimetics, such as epoetin alfa and epoetin beta, which are often referred to as erythropoiesis-stimulating agents (ESAs), are currently used to treat anemia associated with chronic kidney disease and anemia associated with cancer or chemotherapy. However, recombinant EPO therapy requires intravenous administration one to three times per week for up to twelve weeks, a treatment regimen that is inconvenient for the patient. In addition, treatment with high doses of EPO may lead to the proliferation of cancer cells in subjects with cancer or to tumor recurrence in subjects who have previously had cancer. Therefore, there exists a need for alternative therapies to EPO.

**SUMMARY OF THE INVENTION**

The present invention features polypeptides that include an extracellular activin receptor type II (ActRII) variant, such as an extracellular ActRIIA variant, an extracellular ActRIIB variant, or an extracellular ActRII chimera. In some embodiments, a polypeptide of the invention includes an extracellular ActRII variant fused to the N- or C-terminus of an Fc domain monomer or moiety. Such moieties may be attached by amino acid or other covalent bonds and may increase stability of the polypeptide. A polypeptide including an extracellular ActRII variant fused to an Fc domain monomer may also form a dimer (e.g., a homodimer or heterodimer) through the interaction between two Fc domain monomers. The polypeptides of the invention may be used in the place of EPO to treat a subject having a disease or condition that can be treated with EPO or an ESA, such as end-stage renal disease, renal insufficiency, polycythemia, a disease associated with a dysfunction of endothelial progenitor cells, a neurological disorder or inflammatory brain disease, gastrointestinal dysmotility, ischemia (e.g., central nervous system (CNS), liver, renal, or cardiac ischemia), hypoxia, or a disease or condition having an inflammatory or autoimmune component, or to treat a subject receiving kidney dialysis, a subject who is going to undergo surgery, or a subject who has recently received a stem cell transplant. The polypeptides of the invention may also be used to increase EPO levels and EPO receptor levels in a subject in need thereof. Further, the polypeptides of the invention may also be used to affect myostatin, activin (activin A and/or activin B), and/or bone morphogenetic protein 9 (BMP9) signaling in a subject having a risk of developing or having a disease or condition described herein.

Exemplary embodiments of the invention are described in the enumerated paragraphs below.

E1.    A method of treating a subject having or at risk of developing a disease or condition that can be treated with erythropoietin or an erythropoiesis-stimulating agent by administering to the subject a therapeutically effective amount of a composition of Table 10, a composition of Table 11, or a composition of Table 12.

E2.    A method of affecting myostatin, activin A, activin B, and/or BMP9 signaling (e.g., reducing or inhibiting the binding of myostatin, activin A, activin B, and/or BMP9 to their endogenous receptors) in a subject having or at risk of developing a disease or condition that can be treated with erythropoietin or an erythropoiesis-stimulating agent by administering to the subject a therapeutically effective amount of a composition of Table 10, a composition of Table 11, or a composition of Table 12.

E3.    The method of E1 or E2, in which the disease or condition that can be treated with erythropoietin or an erythropoiesis-stimulating agent is anemia due to dialysis or anemia of prematurity.

(continued)

E4.   The method of E1 or E2, in which the disease or condition that can be treated with erythropoietin or an erythropoiesis-stimulating agent is end-stage renal disease, renal insufficiency, polycythemia, hemochromatosis, a disease or condition associated with dysfunction of endothelial progenitor cells, a disease or condition having an autoimmune or inflammatory component, a neurological disorder or inflammatory brain disease, gastrointestinal dysmotility, a disease of the endocrine system, a disease of the reproductive system, aging, pregnancy (e.g., a hematologic irregularity associated with pregnancy), a menstrual disorder, ischemia or an ischemic disorder or condition, hypoxia or a hypoxic disorder or condition, an ulcer, a burn, a wound (e.g., a chronic wound), ischemia-reperfusion injury, asthma, hypertension, a viral disease or infection, a systemic microbial infection, a gastrointestinal disease, arterial sclerosis, cancer, psychosis, a genetic disease, an inflammatory disease, graft-versus-host disease, cardiovascular disease, an allergy, or arthritis.

E5.   A method of increasing erythropoietin levels in a subject in need thereof by administering to the subject a therapeutically effective amount of a composition of Table 10, a composition of Table 11, or a composition of Table 12.

E6.   A method of increasing erythropoietin receptor levels in a subject in need thereof by administering to the subject a therapeutically effective amount of a composition of Table 10, a composition of Table 11, or a composition of Table 12.

E7.   The method of E5 or E6, in which the subject has or is at risk of developing anemia due to dialysis or anemia of prematurity.

E8.   The method of E5 or E6, in which the subject has or is at risk of developing of end-stage renal disease, renal insufficiency, polycythemia, hemochromatosis, a disease or condition associated with dysfunction of endothelial progenitor cells, a disease or condition having an autoimmune or inflammatory component, a neurological disorder or inflammatory brain disease, gastrointestinal dysmotility, a disease of the endocrine system, a disease of the reproductive system, aging, pregnancy (e.g., a hematologic irregularity associated with pregnancy), a menstrual disorder, ischemia or an ischemic disorder or condition, hypoxia or a hypoxic disorder or condition, an ulcer, a burn, a wound (e.g., a chronic wound), ischemia-reperfusion injury, asthma, hypertension, a viral disease or infection, a systemic microbial infection, a gastrointestinal disease, arterial sclerosis, cancer, psychosis, a genetic disease, an inflammatory disease, graft-versus-host disease, cardiovascular disease, an allergy, or arthritis.

E9.   The method of E4 or E8, in which the in which the disease or condition that can be treated with erythropoietin or an erythropoiesis-stimulating agent is ischemia or in which the subject has or is at risk of developing ischemia.

E10.   The method of E9, in which the ischemia is central nervous system ischemia, liver ischemia, renal ischemia, or cardiac ischemia.

E11.   The method of E4 or E8, in which the disease or condition that can be treated with erythropoietin or an erythropoiesis-stimulating agent is an ischemic disorder or condition or in which the subject has or is at risk of developing an ischemic disorder or condition.

E12.   The method of E11, in which the ischemic disorder or condition is occlusive arterial disease, chronic venous insufficiency, circulatory shock (e.g., hemorrhagic, septic, or cardiogenic shock), pulmonary embolism, myocardial infarction, ischemic stroke, acute respiratory failure, chronic heart failure, atherosclerosis, cardiac cirrhosis, macular degeneration, sleep apnea, Raynaud's disease, systemic sclerosis, nonbacterial thrombotic endocarditis, a transient ischemic attack, or ischemia resulting from general anesthesia.

E13.   The method of E4 or E8, in which the disease or condition that can be treated with erythropoietin or an erythropoiesis-stimulating agent is a hypoxic disorder or condition or in which the subject has or is at risk of developing a hypoxic disorder or condition.

E14.   The method of E13, in which the hypoxic disorder or condition is a pulmonary disorder (e.g., chronic obstructive pulmonary disease), perinatal hypoxia, severe pneumonia, pulmonary edema, hyaline membrane disease, liver disease, renal disease, cancer, or altitude sickness.

E15.   The method of E4 or E8, in which the disease or condition that can be treated with erythropoietin or an erythropoiesis-stimulating agent is a viral disease or infection or in which the subject has or is at risk of developing a viral disease or infection.

E16.   The method of E15, in which the viral disease or infection is a Hepatitis C virus infection or an HIV infection.

(continued)

E17. The method of E4 or E8, in which the disease or condition that can be treated with erythropoietin or an erythropoiesis-stimulating agent is a disease or condition associated with dysfunction of endothelial progenitor cells or in which the subject has or is at risk of developing a disease or condition associated with dysfunction of endothelial progenitor cells.

E18. The method of E17, in which the disease or condition associated with dysfunction of endothelial progenitor cells is heart failure, angina pectoris, endotheliosis, reticuloendotheliosis, age-related cardiovascular disorder, coronary heart disease, atherosclerosis, myocardial ischemia, hypercholesterolemia, an ischemic disorder of the extremities, Raynaud's disease, preeclampsia, pregnancy induced hypertension, an endothelium-mediated chronic inflammatory disorder (e.g., inflammation of the vessels), wound healing, chronic renal failure (chronic kidney disease), or acute renal failure (acute kidney failure).

E19. The method of E18, in which the disease or condition associated with dysfunction of endothelial progenitor cells is chronic renal failure (chronic kidney disease).

E20. The method of E4 or E8, in which the disease or condition that can be treated with erythropoietin or an erythropoiesis-stimulating agent is an autoimmune or inflammatory disease or condition or in which the subject has or is at risk of developing an autoimmune or inflammatory disease or condition.

E21. The method of E20, in which the autoimmune or inflammatory disease or condition is acute cerebrovascular injury, acute brain injury, acute cardiovascular injury, arthritis, an autoimmune disease, a stroke, a neurological injury, or immune-mediated inflammation.

E22. The method of E4 or E8, in which the disease or condition that can be treated with erythropoietin or an erythropoiesis-stimulating agent is a neurological disorder or inflammatory brain disease or in which the subject has or is at risk of developing a neurological disorder or inflammatory brain disease.

E23. The method of E22, in which the neurological disorder or inflammatory brain disease is a demyelinating disease, epilepsy, spinal cord injury (e.g., an acute spinal cord injury), a complication following traumatic brain injury (e.g., to treat a symptom of the traumatic brain injury, such as hypotension, hypoxemia, brain swelling, headache, neck pain, difficulty remembering, difficulty concentrating, difficulty making decisions, fatigue, a mood change, nausea, photophobia, blurred vision, ear ringing, a loss of sense of taste, and a loss of sense of smell, seizures, coma, muscle weakness, paralysis, or a progressive decline in neurologic function), a chronic inflammatory brain disease, or a neurological disorder associated with a surgery (e.g., thoracoabdominal aortic surgery).

E24. The method of E23, in which the chronic inflammatory brain disease is a neurodegenerative disease.

E25. The method of E24, in which the neurodegenerative disease is Alzheimer's disease, Parkinson's disease, Huntington's disease, amyotrophic lateral sclerosis (ALS), or age-related macular degeneration (AMD).

E26. The method of E23, in which the demyelinating disease is multiple sclerosis, neuromyelitis optica, acute disseminated encephalomyelitis, or transverse myelitis.

E27. The method of E4 or E8, in which the disease or condition that can be treated with erythropoietin or an erythropoiesis-stimulating agent is gastrointestinal dysmotility or in which the subject has or is at risk of developing gastrointestinal dysmotility.

E28. The method of E27, in which the gastrointestinal dysmotility is associated with an intestinal injury, abdominal trauma, an intestinal inflammatory condition, an intestinal infection, slow transit constipation, post-operative ileus, a neurodegenerative injury, a neurotraumatic injury, a congenital problem, or a malnutrition-malabsorption problem.

E29. The method of E28, in which the intestinal infection is a bacterial infection (e.g., an infection that leads to sepsis or bacteremia), peritonitis, or ascites.

E30. The method of E28, in which the intestinal inflammatory condition is inflammatory bowel disease, Crohn's disease, or ulcerative colitis.

E31. The method of E28, in which the slow transit constipation is chronic constipation, idiopathic constipation, constipation due to post-operative ileus, or constipation caused by opiate use.

E32. The method of E28, in which the congenital problem is gastroschisis, omphalocele, aganglionic megacolon, Hirschsprung's disease, chronic intestinal pseudo-obstruction, small left colon syndrome, anorectal anomalies, esophageal dysplasia and atresia, ectopic anus, congenital hernias, or internal anal sphincter achalasia.

(continued)

E33. The method of E28, in which the malnutrition-malabsorption problem is associated with an intestinal injury, an abdominal trauma, an intestinal inflammatory condition, an intestinal infection, constipation, post-operative ileus, a neurodegenerative injury, a neurotraumatic injury, a congenital problem, Gaucher disease, refeeding syndrome, extremely low birth weight, cancer cachexia, infection, cancer, spinal cord dysfunction, spinal dysraphism, bifida, a tumor, central nervous system dysfunction, peripheral neuropathy, removal part of the gastrointestinal tract, hemorrhage, liver dysfunction, celiac disease, cystic fibrosis, muscular dystrophies, or cerebral palsy.

E34. The method of E4 or E8, in which the disease or condition that can be treated with erythropoietin or an erythropoiesis-stimulating agent is end-stage renal disease or in which the subject has or is at risk of developing end-stage renal disease.

E35. The method of E4 or E8, in which the disease or condition that can be treated with erythropoietin or an erythropoiesis-stimulating agent is polycythemia or in which the subject has or is at risk of developing polycythemia.

E36. The method of any one of E1-E35, in which the composition of Table 10, Table 11, or Table 12 is administered to the subject prior to surgery (e.g., to increase red blood cell count prior to surgery), after stem cell transplantation, prior to or during a space flight, during or after tissue or organ transplantation, to promote the growth of new blood vessels, for granulation tissue formation, for trauma treatment, or for post-vascular graft treatment.

E37 The method of any one of E1-E36, in which the subject is receiving kidney dialysis.

E38. The method of any one of E1, E2, E4-E6, and E8-E37, in which the subject does not have anemia.

E39. The method of any one of E1, E2, E4-E6, and E8-E38, in which the subject has normal hematopoiesis.

E40. The method of any one of E1-E39, in which the subject has low serum erythropoietin.

E41. A method of preparing a tissue or organ for transplantation by contacting the tissue or organ (e.g., when in the tissue or organ donor or after removal from the tissue or organ donor) with a therapeutically effective amount of a composition of Table 10, a composition of Table 11, or a composition of Table 12.

E42. The method of any one of E1-E41, wherein the method comprises administering to the subject a therapeutically effective amount of a composition of Table 10.

E43. The method of any one of E1-E41, wherein the method comprises administering to the subject a therapeutically effective amount of a composition of Table 11.

E44. The method of any one of E1-E41, wherein the method comprises administering to the subject a therapeutically effective amount of a composition of Table 12.

E45. The method of any one of E1-E44, wherein the composition is administered in an amount sufficient to increase EPO levels, increase EPO receptor levels, promote the growth of new blood vessels and/or the replacement of damaged vascular regions, promote granulation tissue formation, reduce infiltration of mononuclear cells into the brain of the subject, improve a neurological deficit, reduce axonal damage, reduce neuronal cell death, reduce glial cell death, affect myostatin, activin A, activin B, and/or BMP9 signaling in the subject, or reduce or inhibit the binding of activin A, activin B, and/or myostatin to their receptors.

E46. The method of any one of E1-E45, wherein the method does not cause a vascular complication in the subject.

E47. The method of E46, wherein the method does not increase vascular permeability or leakage.

E48. The method of any one of E1-E47, wherein the subject is a human.

**Definitions**

To facilitate the understanding of this invention, a number of terms are defined below. Terms defined herein have meanings as commonly understood by a person of ordinary skill in the areas relevant to the invention. Terms such as "a", "an," and "the" are not intended to refer to only a singular entity but include the general class of which a specific example may be used for illustration. The terminology herein is used to describe specific embodiments of the invention, but their usage does not limit the invention, except as outlined in the claims.

As used herein, the term "about" refers to a value that is within 10% above or below the value being described.

As used herein, any values provided in a range of values include both the upper and lower bounds, and any values contained within the upper and lower bounds.

As used herein, the terms "extracellular activin receptor type IIA (ActRIIA) variant" and "ActRIIA variant" refer to a peptide including a soluble, extracellular portion of the single transmembrane receptor, ActRIIA, that has at least one

**EP 4 674 480 A2**

amino acid substitution relative to a wild-type extracellular ActRIIA (e.g., bold portion of the sequence of SEQ ID NO: 75 shown below) or an extracellular ActRIIA having any one of the sequences of SEQ ID NOs: 76-96. The sequence of the wild-type, human ActRIIA precursor protein is shown below (SEQ ID NO: 75), in which the signal peptide is italicized and the extracellular portion is bold.

Wild-type, human ActRIIA precursor protein (SEQ ID NO: 75):

> *MGAAAKLAFAVFLISCSS***GAILGRSETQECLFFNANWEKDRTNQTGVEPCYGDKDKRRHCFAT WKNISGSIEIVKQGCWLDDINCYDRTDCVEKKDSPEVYFCCCEGNMCNEKFSYFPEMEVTQPT S**NPVTPKPPYYNILLYSLVPLMLIAGIVICAFWVYRHHKMAYPPVLVPTQDPGPPPPSPLLGLKPL QLLEVKARGRFGCVWKAQLLNEYVAVKIFPIQDKQSWQNEYEVYSLPGMKHENILQFIGAEKRG TSVDVDLWLITAFHEKGSLSDFLKANVVSWNELCHIAETMARGLAYLHEDIPGLKDGHKPAISHR DIKSKNVLLKNNLTACIADFGLALKFEAGKSAGDTHGQVGTRRYMAPEVLEGAINFQRDAFLRID MYAMGLVLWELASRCTAADGPVDEYMLPFEEEIGQHPSLEDMQEVVVHKKKRPVLRDYWQKH AGMAMLCETIEECWDHDAEARLSAGCVGERITQMQRLTNIITTEDIVTVVTMVTNVDFPPKESSL

An extracellular ActRIIA variant may have a sequence of any one of SEQ ID NOs: 1-72. In particular embodiments, an extracellular ActRIIA variant has a sequence of any one of SEQ ID NOs: 6-72 (Table 2). In some embodiments, an extracellular ActRIIA variant may have at least 85% (e.g., at least 85%, 87%, 90%, 92%, 95%, 97%, or greater) amino acid sequence identity to the sequence of a wild-type extracellular ActRIIA (SEQ ID NO: 73).

As used herein, the terms "extracellular activin receptor type IIB (ActRIIB) variant" and "ActRIIB variant" refer to a peptide including a soluble, extracellular portion of the single transmembrane receptor, ActRIIB, that has at least one amino acid substitution relative to a wild-type extracellular ActRIIB (e.g., bold portion of the sequence of SEQ ID NO: 173 shown below). The sequence of the wild-type, human ActRIIB is shown below (SEQ ID NO: 173), in which the signal peptide is italicized and the extracellular portion is bold.

Wild-type human ActRIIB (SEQ ID NO: 173):

> *MTAPWVALALLWGSLCAGS***GRGEAETRECIYYNANWELERTNQSGLERCEGEQDKRLHCY ASWRNSSGTIELVKKGCWLDDFNCYDRQECVATEENPQVYFCCCEGNFCNERFTHLPEAG GPEVTYEPPPTAPT**LLTVLAYSLLPIGGLSLIVLLAFWMYRHRKPPYGHVDIHEDPGPPP PSPLVGLKPLQLLEIKARGRFGCVWKAQLMNDFVAVKIFPLQDKQSWQSEREIFSTPGMK HENLLQFIAAEKRGSNLEVELWLITAFHDKGSLTDYLKGNIITWNELCHVAETMSRGLSY LHEDVPWCRGEGHKPSIAHRDFKSKNVLLKSDLTAVLADFGLAVRFEPGKPPGDTHGQVG TRRYMAPEVLEGAINFQRDAFLRIDMYAMGLVLWELVSRCKAADGPVDEYMLPFEEEIGQ HPSLEELQEVVVHKKMRPTIKDHWLKHPGLAQLCVTIEECWDHDAEARLSAGCVEERVSL IRRSVNGTTSDCLVSLVTSVTNVDLPPKESSI

An extracellular ActRIIB variant may have a sequence of any one of SEQ ID NOs: 157-171. In particular embodiments, an extracellular ActRIIB variant has a sequence of any one of SEQ ID NOs: 158-171 (Table 5). In some embodiments, an extracellular ActRIIB variant may have at least 85% (e.g., at least 85%, 87%, 90%, 92%, 95%, 96%, 97%, 98%, 99%, or greater) amino acid sequence identity to the sequence of a wild-type extracellular ActRIIB (SEQ ID NO: 74). The extracellular ActRIIB variant may also have an N-terminal truncation of 1-7 amino acids relative to the extracellular portion of ActRIIB.

As used herein, the terms "extracellular activin receptor type II (ActRII) chimera," "extracellular ActRII chimera," and "ActRII chimera" refer to a peptide including a soluble, extracellular portion of the single transmembrane receptor ActRIIB and a soluble, extracellular portion of the single transmembrane receptor ActRIIA. The ActRII chimeras described herein result from joining an N-terminal portion of extracellular ActRIIB to a C-terminal portion of extracellular ActRIIA such that the sequences are contiguous (e.g., the ActRIIA sequence continues where the ActRIIB sequence left off, starting with the next the amino acid located in the corresponding position of ActRIIA). The extracellular ActRII chimera may also include one or more amino acid substitutions in the portion of the chimera that corresponds to the sequence of ActRIIB compared to a wild-type extracellular ActRIIB (e.g., bold portion of the sequence of SEQ ID NO: 173 shown above), and one or more amino acid substitutions in the portion of the chimera that corresponds to the sequence of ActRIIA compared to a wild-type extracellular ActRIIA (e.g., bold portion of the sequence of SEQ ID NO: 75 shown above). The extracellular ActRII chimera may also have an N-terminal truncation of 1-9 amino acids relative to the extracellular portion of ActRIIB or ActRIIA. The sequences of wild-type, human ActRIIB (SEQ ID NO: 173) and wild-type, human ActRIIA (SEQ ID NO: 75) are shown in the definitions above, in which the signal peptide is italicized and the extracellular portion is bold. An extracellular ActRII chimera may have the sequence of any one of SEQ ID NOs: 174-216. In particular embodiments, an extracellular ActRII chimera has the sequence of any one of SEQ ID NOs: 195-216 (Table 7).

As used herein, the term "extracellular activin receptor type II (ActRII) variant" refers to an extracellular ActRIIA variant, an extracellular ActRIIB variant, or an extracellular ActRII chimera described herein.

As used herein, the term "N-terminal truncation" refers to a deletion of 1-7 amino acids (e.g., 1, 2, 3, 4, 5, 6, or 7 amino acids) from the N-terminus of an extracellular ActRIIB variant (e.g., an extracellular ActRIIB variant having the sequence of any one of SEQ ID NOs: 157-171 (e.g., SEQ ID NOs: 158-171)) or a deletion of 1-9 amino acids (e.g., 1, 2, 3, 4, 5, 6, 7, 8, or 9 amino acids) from the N-terminus of an extracellular ActRII chimera (e.g., an extracellular ActRII chimera having the sequence of any one of SEQ ID NOs: 174-216 (e.g., SEQ ID NOs: 195-216)). The N-terminal truncation can remove amino acids up two to amino acids before the first cysteine (e.g., the two amino acids before the first cysteine (RE) are retained in an N-terminally truncated extracellular ActRIIB variant and the two amino acids before the first cystine (RE or QE) are retained in an N-terminally truncated extracellular ActRII chimera).

As used herein, the term "linker" refers to a linkage between two elements, e.g., peptides or protein domains. A polypeptide described herein may include an extracellular ActRII variant (e.g., an extracellular ActRIIA variant (e.g., an extracellular ActRIIA variant having a sequence of any one of SEQ ID NOs: 1-72 (e.g., SEQ ID NOs: 6-72)), an extracellular ActRIIB variant (e.g., an extracellular ActRIIB variant having the sequence of any one of SEQ ID NOs: 157-171 (e.g., SEQ ID NOs: 158-171)), or an extracellular ActRII chimera (e.g., an extracellular ActRII chimera having a sequence of any one of SEQ ID NOs: 174-216 (e.g., SEQ ID NOs: 195-216)) fused to a moiety. The moiety may increase stability or improve pharmacokinetic properties of the polypeptide. The moiety (e.g., Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin) may be fused to the polypeptide by way of a linker. A linker can be a covalent bond or a spacer. The term "bond" refers to a chemical bond, e.g., an amide bond or a disulfide bond, or any kind of bond created from a chemical reaction, e.g., chemical conjugation. The term "spacer" refers to a moiety (e.g., a polyethylene glycol (PEG) polymer) or an amino acid sequence (e.g., a 1-200 amino acid sequence) occurring between two elements, e.g., peptides or protein domains, to provide space and/or flexibility between the two elements. An amino acid spacer is part of the primary sequence of a polypeptide (e.g., fused to the spaced peptides via the polypeptide backbone). The formation of disulfide bonds, e.g., between two hinge regions that form an Fc domain, is not considered a linker.

As used herein, the term "Fc domain" refers to a dimer of two Fc domain monomers. An Fc domain has at least 80% sequence identity (e.g., at least 85%, 90%, 95%, 97%, or 100% sequence identity) to a human Fc domain that includes at least a $C_H2$ domain and a $C_H3$ domain. An Fc domain monomer includes second and third antibody constant domains ($C_H2$ and $C_H3$). In some embodiments, the Fc domain monomer also includes a hinge domain. An Fc domain does not include any portion of an immunoglobulin that is capable of acting as an antigen-recognition region, e.g., a variable domain or a complementarity determining region (CDR). In the wild-type Fc domain, the two Fc domain monomers dimerize by the interaction between the two $C_H3$ antibody constant domains, as well as one or more disulfide bonds that form between the hinge domains of the two dimerizing Fc domain monomers. In some embodiments, an Fc domain may be mutated to lack effector functions, typical of a "dead Fc domain." In certain embodiments, each of the Fc domain monomers in an Fc domain includes amino acid substitutions in the $C_H2$ antibody constant domain to reduce the interaction or binding between the Fc domain and an Fcy receptor. In some embodiments, the Fc domain contains one or more amino acid substitutions that reduce or inhibit Fc domain dimerization. An Fc domain can be any immunoglobulin antibody isotype, including IgG, IgE, IgM, IgA, or IgD. Additionally, an Fc domain can be an IgG subtype (e.g., IgG1, IgG2a, IgG2b, IgG3, or IgG4). The Fc domain can also be a non-naturally occurring Fc domain, e.g., a recombinant Fc domain.

As used herein, the term "albumin-binding peptide" refers to an amino acid sequence of 12 to 16 amino acids that has affinity for and functions to bind serum albumin. An albumin-binding peptide can be of different origins, e.g., human, mouse, or rat. In some embodiments, an albumin-binding peptide has the sequence DICLPRWGCLW (SEQ ID NO: 151).

As used herein, the term "fibronectin domain" refers to a high molecular weight glycoprotein of the extracellular matrix, or a fragment thereof, that binds to, e.g., membrane-spanning receptor proteins such as integrins and extracellular matrix components such as collagens and fibrins. In some embodiments, a fibronectin domain is a fibronectin type III domain (SEQ ID NO: 152) having amino acids 610-702 of the sequence of UniProt ID NO: P02751. In other embodiments, a fibronectin domain is an adnectin protein.

As used herein, the term "human serum albumin" refers to the albumin protein present in human blood plasma. Human serum albumin is the most abundant protein in the blood. It constitutes about half of the blood serum protein. In some embodiments, a human serum albumin has the sequence of UniProt ID NO: P02768 (SEQ ID NO: 153).

As used herein, the term "endogenous" describes a molecule (e.g., a polypeptide, nucleic acid, or cofactor) that is found naturally in a particular organism (e.g., a human) or in a particular location within an organism (e.g., an organ, a tissue, or a cell, such as a human cell, e.g., a human red blood cell, platelet, neutrophil, or muscle cell).

As used herein, the term "fused" is used to describe the combination or attachment of two or more elements, components, or protein domains, e.g., peptides or polypeptides, by means including chemical conjugation, recombinant means, and chemical bonds, e.g., amide bonds. For example, two single peptides in tandem series can be fused to form one contiguous protein structure, e.g., a polypeptide, through chemical conjugation, a chemical bond, a peptide linker, or any other means of covalent linkage. In some embodiments of a polypeptide described herein, an extracellular ActRII variant (e.g., an extracellular ActRIIA variant (e.g., an extracellular ActRIIA variant having a sequence of any one of SEQ ID NOs: 1-72 (e.g., SEQ ID NOs: 6-72)), an extracellular ActRIIB variant (e.g., an extracellular ActRIIB variant having the sequence of any one of SEQ ID NOs: 157-171 (e.g., SEQ ID NOs: 158-171)), or an extracellular ActRII chimera (e.g., an extracellular ActRII chimera having a sequence of any one of SEQ ID NOs: 174-216 (e.g., SEQ ID NOs: 195-216)) may be fused in tandem series to the N- or C-terminus of a moiety (e.g., Fc domain monomer (e.g., the sequence of SEQ ID NO: 97), an Fc domain (e.g., a wild-type Fc domain (e.g., the sequence of SEQ ID NO: 150 or SEQ ID NO: 155)), an Fc domain with amino acid substitutions (e.g., one or more substitutions that reduce dimerization), an albumin-binding peptide (e.g., the sequence of SEQ ID NO: 151), a fibronectin domain (e.g., the sequence of SEQ ID NO: 152), or a human serum albumin (e.g., the sequence of SEQ ID NO: 153)) by way of a linker. For example, an extracellular ActRII variant is fused to a moiety (e.g., an Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin) by way of a peptide linker, in which the N-terminus of the peptide linker is fused to the C-terminus of the extracellular ActRII variant through a chemical bond, e.g., a peptide bond, and the C-terminus of the peptide linker is fused to the N-terminus of the moiety (e.g., Fc domain monomer, Fc domain (e.g., wild-type Fc domain), Fc domain with amino acid substitutions (e.g., one or more substitutions that reduce dimerization), albumin-binding peptide, fibronectin domain, or human serum albumin) through a chemical bond, e.g., a peptide bond.

As used herein, the term "C-terminal extension" refers to the addition of one or more amino acids to the C-terminus of an extracellular ActRIIA variant (e.g., an extracellular ActRIIA variant having the sequence of any one of SEQ ID NOs: 1-70 (e.g., SEQ ID NOs: 6-70)) or at the C-terminus of a an extracellular ActRII chimera (e.g., an extracellular ActRII chimera having the sequence of any one of SEQ ID NOs: 174-216 (e.g., SEQ ID NOs: 195-216)). The C-terminal extension can be one or more amino acids, such as 1-6 amino acids (e.g., 1, 2, 3, 4, 5, 6 or more amino acids). The C-terminal extension may include amino acids from the corresponding position of wild-type ActRIIA (for an ActRIIA variant) or from the corresponding position of wild-type ActRIIA or ActRIIB (for an ActRII chimera). Exemplary C-terminal extensions are the amino acid sequence NP (a two amino acid C-terminal extension) and the amino acid sequence NPVTPK (SEQ ID NO: 154) (a six amino acid C-terminal extension). Any amino acid sequence that does not disrupt the activity of the polypeptide can be used. SEQ ID NO: 71, which is the sequence of SEQ ID NO: 69 with a C-terminal extension of NP, and SEQ ID NO: 72, which is the sequence of SEQ ID NO: 69 with a C-terminal extension of NPVTPK, represent two of the possible ways that a polypeptide of the invention can be modified to include a C-terminal extension.

As used herein, the term "percent (%) identity" refers to the percentage of amino acid (or nucleic acid) residues of a candidate sequence, e.g., an extracellular ActRIIA variant, an extracellular ActRIIB variant, or an extracellular ActRII chimera, that are identical to the amino acid (or nucleic acid) residues of a reference sequence, e.g., a wild-type extracellular ActRIIA (e.g., SEQ ID NO: 73) or wild-type extracellular ActRIIB (e.g., SEQ ID NO: 74), after aligning the sequences and introducing gaps, if necessary, to achieve the maximum percent identity (i.e., gaps can be introduced in one or both of the candidate and reference sequences for optimal alignment and non-homologous sequences can be disregarded for comparison purposes). Alignment for purposes of determining percent identity can be achieved in various ways that are within the skill in the art, for instance, using publicly available computer software such as BLAST, ALIGN, or Megalign (DNASTAR) software. Those skilled in the art can determine appropriate parameters for measuring alignment, including any algorithms needed to achieve maximal alignment over the full length of the sequences being compared. In some embodiments, the percent amino acid (or nucleic acid) sequence identity of a given candidate sequence to, with, or against a given reference sequence (which can alternatively be phrased as a given candidate sequence that has or includes a certain percent amino acid (or nucleic acid) sequence identity to, with, or against a given reference sequence) is calculated as follows:

$$100 \times (\text{fraction of } A/B)$$

where A is the number of amino acid (or nucleic acid) residues scored as identical in the alignment of the candidate sequence and the reference sequence, and where B is the total number of amino acid (or nucleic acid) residues in the reference sequence. In some embodiments where the length of the candidate sequence does not equal to the length of the reference sequence, the percent amino acid (or nucleic acid) sequence identity of the candidate sequence to the reference sequence would not equal to the percent amino acid (or nucleic acid) sequence identity of the reference sequence to the candidate sequence.

In particular embodiments, a reference sequence aligned for comparison with a candidate sequence may show that the candidate sequence exhibits from 50% to 100% identity across the full length of the candidate sequence or a selected portion of contiguous amino acid (or nucleic acid) residues of the candidate sequence. The length of the candidate sequence aligned for comparison purpose is at least 30%, e.g., at least 40%, e.g., at least 50%, 60%, 70%, 80%, 90%, or 100% of the length of the reference sequence. When a position in the candidate sequence is occupied by the same amino acid (or nucleic acid) residue as the corresponding position in the reference sequence, then the molecules are identical at that position.

As used herein, the term "serum half-life" refers to, in the context of administering a therapeutic protein to a subject, the time required for plasma concentration of the protein in the subject to be reduced by half. The protein can be redistributed or cleared from the bloodstream, or degraded, e.g., by proteolysis. Serum half-life comparisons can be made by comparing the serum half-life of Fc fusion proteins.

As used herein, the term "affinity" or "binding affinity" refers to the strength of the binding interaction between two molecules. Generally, binding affinity refers to the strength of the sum total of non-covalent interactions between a molecule and its binding partner, such as an extracellular ActRIIA variant, an extracellular ActRIIB variant, or an extracellular ActRII chimera and BMP9 or activin A. Unless indicated otherwise, binding affinity refers to intrinsic binding affinity, which reflects a 1:1 interaction between members of a binding pair. The binding affinity between two molecules is commonly described by the dissociation constant ($K_D$) or the affinity constant ($K_A$). Two molecules that have low binding affinity for each other generally bind slowly, tend to dissociate easily, and exhibit a large $K_D$. Two molecules that have high affinity for each other generally bind readily, tend to remain bound longer, and exhibit a small $K_D$. The $K_D$ of two interacting molecules may be determined using methods and techniques well known in the art, e.g., surface plasmon resonance. $K_D$ is calculated as the ratio of $k_{off}/k_{on}$.

As used herein, the phrase "affecting myostatin, activin A, activin B, and/or BMP9 signaling" means changing the binding of myostatin, activin A, activin B, and/or BMP9 to their receptors, e.g., ActRIIA, ActRIIB, and/or BMPRII (e.g., ActRIIA or ActRIIB, e.g., endogenous ActRIIA or ActRIIB). In some embodiments, a polypeptide including an extracellular ActRIIA variant, an extracellular ActRIIB variant, or an extracellular ActRll chimera described herein reduces or inhibits the binding of myostatin, activin A, activin B, and/or BMP9 to their receptors, e.g., ActRIIA, ActRIIB, and/or BMPRII (e.g., ActRIIA or ActRIIB, e.g., endogenous ActRIIA or ActRIIB).

As used herein, the terms "increasing" and "decreasing" refer to modulating resulting in, respectively, greater or lesser amounts, of function, expression, or activity of a metric relative to a reference. For example, subsequent to administration of a polypeptide of the invention including an extracellular ActRIIA variant, an extracellular ActRIIB variant, or an extracellular ActRII chimera in a method described herein, the amount of a marker of a metric (e.g., EPO or EPO receptor levels) as described herein may be increased in a subject relative to the amount of the marker prior to administration. Generally, the metric is measured subsequent to administration at a time that the administration has had the recited effect, e.g., at least one week, one month, 3 months, or 6 months, after a treatment regimen has begun.

As used herein, the term "anemia" refers to any abnormality in hemoglobin or red blood cells that leads to reduced oxygen levels in the blood. Anemia can be associated with abnormal production, processing, or performance of erythrocytes and/or hemoglobin. The term anemia refers to any reduction in the number of red blood cells and/or level of hemoglobin in blood relative to normal blood levels and can be diagnosed using routine tests, such as a complete blood count.

As used herein, the term "normal hematopoiesis" refers to the process by which the components of blood and blood plasma are produced, which includes the formation of red blood cells (erythrocytes), white blood cells (leukocytes, which includes the formation of lymphocytes, neutrophils, eosinophils, basophils, and macrophages), and platelets (thrombocytes). A subject has normal hematopoiesis if the production of these cells is not impaired and the number of these cells in the blood falls within a range accepted as normal by a medical professional. For example, the normal red blood cell (RBC) range for men is 4.7 to 6.1 million cells per microliter (mcL), the normal RBC range for women who are not pregnant is 4.2 to 5.4 million mcL, and the normal RBC range for children is 4.0 to 5.5 million mcL.

As used herein, the term "a disease or condition that can be treated with EPO or an ESA" refers to a disease or condition that is currently treated by administering EPO, recombinant EPO, an EPO mimetic, or another agent that increases EPO or EPO receptor levels, a disease or condition that could be expected to benefit from increasing EPO or EPO receptor levels based on studies performed in cell culture conditions, animal models, or human trials, or a disease or condition that is associated with low serum EPO. Such diseases and conditions include end-stage renal disease, renal insufficiency, kidney dialysis, spinal cord injury, an iron overload disorder (e.g., hemochromatosis), an inflammatory brain disease, gastrointestinal dysmotility, ischemia, and other diseases and conditions as described in U.S. Patent Nos. 5,013,718, 7,745,387, 8,466,172, 8,729,030, and 10,695,402 and U.S. Patent Application Publication Nos. US20180303903A1 and US20170312268A1, each of which is hereby incorporated by reference.

As used herein, the term "low serum erythropoietin" refers to a level of serum erythropoietin that is below the normal range. Normal levels of erythropoietin range from 4 to 26 milliunits per liter (mU/mL).

"Hypercholesterolemia" is characterized by elevated concentrations of cholesterol in the blood. By far the commonest form of primary hypercholesterolemia is polygenic hypercholesterolemia. Secondary hypercholesterolemias frequently occur in association with diabetes mellitus, nephrotic syndrome, hypothyroidism and hepatic disorders.

"Endothelium-mediated chronic inflammatory disorders" are disorders or conditions of a human or animal body that derive from a defense response of the body and its tissues to harmful stimuli, with certain signal molecules altering the properties of endothelial cells so that, in concert with the activation of other cell types, leukocytes remain adherent to endothelial cells, finally penetrate into the tissue and there initiate inflammation. One example of an endothelium-mediated inflammation is leukocytic vasculitis. A central part is played in the activation of an endothelium-mediated inflammatory event by the transcription factor NF-κB. Another system leading to the development of endothelial cell-mediated chronic inflammations is the AGE-RAGE system.

"Endotheliosis" refers to degenerative and proliferative endothelial changes associated with nonthrombopenic purpura. "Reticuloendotheliosis" refers to diseases of the reticulohistiocytic system, such as reticulum, reticulosis, reticulohistiocytosis and Hand-Schüller-Christian disease.

"Myocardial ischemia" refers to bloodlessness or hypoperfusion, that is an impairment of the blood supply, of the muscular wall of the heart as a result of inadequate or absent arterial supply of blood.

A "cardiac infarct" or "myocardial infarct" is a necrosis of a localized region of the myocardium, which usually occurs as an acute event complicating chronic coronary heart disease.

"Coronary heart disease" or "ischemic heart disease" is a degenerative coronary disorder which, owing to a constriction or a closure of coronary vessels of the heart, leads to a reduced blood supply to the myocardium.

"Angina pectoris" refers to an acute coronary insufficiency or stenocardia which may be induced by an imbalance of the oxygen supply and oxygen demand associated with coronary heart disease, coronary spasms, impairments of blood flow, cardiac arrhythmias, hypertension or hypotension.

"Raynaud's disease" refers to ischemic states which are caused by vasoconstriction, that is vessel spasms, and occurs episodically, usually in the arteries of the fingers. Primary Raynaud's disease is a purely functional impairment of the small vessels supplying the distal parts of the extremities, whereas secondary Raynaud's disease has another disease underlying it, for example an inflammation of vessels.

"Preeclampsia" is an endothelial and vascular disease of the maternal body and appears to be the effect of endotheliotropic substances from the placenta. Preeclampsia is a multisystem disorder which may lead to disturbances of function of numerous organs and be manifested by diverse symptoms. The impairments of blood supply which are typical of the disorder are the result of an increased vascular resistance, possibly with local variations in severity. It is regarded as confirmed that an endothelial dysfunction is the central component of the pathogenesis of preeclampsia.

"Renal failure" refers to the restricted ability of the kidneys to excrete substances normally excreted in the urine, and in advanced stages there is also loss of the ability to regulate the electrolyte, water and acid-base balance. Terminal renal failure is characterized by a collapse of the excretory and endocrine function of the kidneys.

"Heart failure" refers to a pathological state that is also referred to as myocardial insufficiency or weakness of the heart muscle. Heart failure is characterized by inadequate functioning of the heart, the heart no longer being capable of efficient delivery to comply with the requirements. Heart failure can be categorized according to various aspects. For example, according to the affected segment of the heart it is classified as right heart failure, left heart failure and failure on both sides (global failure). According to the stability of an equilibrium influenced by physiological and therapeutic mechanisms, a distinction is made between compensated and decompensated heart failure. Classification takes place into acute and chronic heart failure according to the time course. Causes of heart failure are, inter alia, myocardial infarction, cardiomyopathy, inborn or acquired cardiac defects, essential or pulmonary hypertension, cardiac arrhythmias, coronary heart disease or myocarditis.

The term "ischemia" refers to a reduction in blood flow. Ischemia is associated with a reduction in nutrients, including oxygen, delivered to tissues. Ischemia may arise due to conditions such as atherosclerosis, formation of a thrombus in an artery or vein, or blockage of an artery or vein by an embolus, vascular closure due to other causes, e.g., vascular spasm, etc. Such conditions may reduce blood flow, producing a state of hypoperfusion to an organ or tissue, or block blood flow completely. Other conditions that can produce ischemia include tissue damage due to trauma or injury, such as, e.g., spinal cord injury; viral infection, which can lead to, e.g., congestive heart failure, etc. The terms "ischemic conditions" and "ischemic disorders" refer to acute ischemic conditions including myocardial infarction, ischemic stroke, pulmonary embolism, perinatal hypoxia, circulatory shock including, e.g., hemorrhagic, septic, cardiogenic, etc., acute respiratory failure, etc., chronic ischemic conditions including atherosclerosis, chronic venous insufficiency, chronic heart failure, cardiac cirrhosis, macular degeneration, sleep apnea, Raynaud's disease, systemic sclerosis, nonbacterial thrombotic endocarditis, occlusive artery disease, angina pectoris, TIAs, chronic alcoholic liver disease, etc. Ischemic conditions may also result when individuals are placed under general anesthesia, and can cause tissue damage in organs prepared for transplant.

The terms "hypoxia" and "hypoxic" refer to an environment with levels of oxygen below normal. Hypoxia may be induced in cells by culturing the cells in a reduced oxygen environment, or cells may be treated with compounds that mimic hypoxia. Determining oxygen levels that define hypoxia in cell culture is well within the skill in the art. The terms "hypoxic conditions" and "hypoxic disorders" include ischemic disorders (ischemic hypoxia) such as those listed above, wherein hypoxia results from reduced circulation; pulmonary disorders (hypoxic hypoxia) such as chronic obstructive pulmonary disease (COPD), severe pneumonia, pulmonary edema, hyaline membrane disease, and the like, wherein hypoxia results from reduced oxygenation of the blood in the lungs; liver or renal disease, cancer or other chronic illness, and altitude sickness, etc.

"Wound healing" refers to the physiological processes for regenerating damaged tissue and for closing a wound, especially formation of new connective tissue and capillaries. The wound healing may be primary wound healing (first intention healing), which is characterized by rapid and complication-free closure and substantially complete recovery as a result of minimal formation of new connective tissue between the edges of a wound, which have a good blood supply and are approximated where appropriate, of a clean wound. Wounds where the edges of the wound are further apart and, in particular, crushed or necrotic, and infected wounds, undergo delayed secondary wound healing (second intention healing) in which, as a result of an (a)bacterial inflammation, there is filling of the tissue defect with granulation tissue and extensive formation of scar tissue. Epithelialization starting from the edge terminates the wound healing. The wound healing is divided into three phases, namely latency phase, proliferative phase and repair phase. The latency phase in turn is divided into the oxidative phase with scab formation, especially in the first few hours after the wound occurred, and the absorptive phase with catabolic autolysis, which extends over a period of from one to three days after the wound occurred. The proliferative phase is characterized by anabolic repair with production of collagen by fibroblasts and occurs on the fourth to seventh day after the wound occurred. The repair phase occurs after the eighth day after the wound occurred and is characterized by transformation of the granulation tissue into a scar.

A "wound" refers to an interruption of the coherence of body tissues with or without loss of substance and caused by mechanical injury or physically caused cell damage. Types of wound are mechanical wounds, thermal wounds, chemical wounds, radiation wounds and disease-related wounds. Mechanical wounds arise through traumatic violence and occur in particular as incision and puncture wounds, crushing, lacerating, tearing and abrading wounds, scratch and bite wounds and projective wounds. Thermal wounds arise through exposure to heat or cold. Chemical wounds arise in particular through the action of acids or alkalis. Radiation wounds arise for example through exposure to actinic and ionizing radiation. Wounds occurring in relation to disease are in particular congestion-related wounds, traumatic wounds, diabetic wounds etc.

The term "inflammatory brain disease or disorder" as used herein refers to a brain disease or disorder caused by acute or chronic inflammatory responses in the central nervous system. Acute inflammatory responses in the brain include activation of microglia, appearance of dendritic cells, and the release of pro-inflammatory cytokines and chemokines in the central nervous system. Chronic inflammatory responses include long-standing activation of microglia and subsequent sustained release of inflammatory mediators. Such long-standing activation of microglia results in activation and proliferation of additional microglia, and further release of inflammatory factors. Examples of chronic inflammatory brain diseases or disorders include demyelinating diseases, such as multiple sclerosis, and neurodegenerative diseases, such as Alzheimer's disease (AD), Parkinson's disease (PD), Huntington's disease, amyotrophic lateral sclerosis (ALS), and age-related macular degeneration (AMD).

As used herein, the term "vascular complication" refers to a vascular disorder or any damage to the blood vessels, such as damage to the blood vessel walls. Damage to the blood vessel walls may cause an increase in vascular permeability or leakage. The term "vascular permeability or leakage" refers to the capacity of the blood vessel walls to allow the flow of small molecules, proteins, and cells in and out of blood vessels. An increase in vascular permeability or leakage may be caused by an increase in the gaps (e.g., an increase in the size and/or number of the gaps) between endothelial cells that line the blood vessel walls and/or thinning of the blood vessel walls.

As used herein, the term "polypeptide" describes a single polymer in which the monomers are amino acid residues which are covalently conjugated together through amide bonds. A polypeptide is intended to encompass any amino acid sequence, either naturally occurring, recombinant, or synthetically produced.

As used herein, the term "homodimer" refers to a molecular construct formed by two identical macromolecules, such as proteins or nucleic acids. The two identical monomers may form a homodimer by covalent bonds or non-covalent bonds. For example, an Fc domain may be a homodimer of two Fc domain monomers if the two Fc domain monomers contain the same sequence. In another example, a polypeptide described herein including an extracellular ActRIIA variant, an extracellular ActRIIB variant, or an extracellular ActRII chimera fused to an Fc domain monomer may form a homodimer through the interaction of two Fc domain monomers, which form an Fc domain in the homodimer.

As used herein, the term "heterodimer" refers to a molecular construct formed by two different macromolecules, such as proteins or nucleic acids. The two monomers may form a heterodimer by covalent bonds or non-covalent bonds. For example, a polypeptide described herein including an extracellular ActRIIA variant, an extracellular ActRIIB variant, or an extracellular ActRII chimera fused to an Fc domain monomer may form a heterodimer through the interaction of two Fc domain monomers, each fused to a different extracellular ActRIIA variant, extracellular ActRIIB variant, or extracellular ActRII chimera, respectively, which form an Fc domain in the heterodimer (e.g., a heterodimer can contain two different extracellular ActRIIA variants, two different extracellular ActRIIB variants, or two different extracellular ActRII chimeras).

As used herein, the term "host cell" refers to a vehicle that includes the necessary cellular components, e.g., organelles, needed to express proteins from their corresponding nucleic acids. The nucleic acids are typically included in nucleic acid vectors that can be introduced into the host cell by conventional techniques known in the art (transformation, transfection, electroporation, calcium phosphate precipitation, direct microinjection, etc.). A host cell may be a prokaryotic cell, e.g., a bacterial cell, or a eukaryotic cell, e.g., a mammalian cell (e.g., a CHO cell or a HEK293 cell).

As used herein, the term "therapeutically effective amount" refers an amount of a polypeptide, nucleic acid, or vector of the invention or a pharmaceutical composition containing a polypeptide, nucleic acid, or vector of the invention effective in achieving the desired therapeutic effect in treating a patient having or at risk of developing a disease or condition, such as a disease or condition that can be treated with EPO or an ESA (e.g., end-stage renal disease, renal insufficiency, kidney dialysis, spinal cord injury, an iron overload disorder (e.g., hemochromatosis), an inflammatory brain disease, ischemia, or gastrointestinal dysmotility). In particular, the therapeutically effective amount of the polypeptide, nucleic acid, or vector avoids adverse side effects.

As used herein, the term "pharmaceutical composition" refers to a medicinal or pharmaceutical formulation that includes an active ingredient as well as excipients and diluents to enable the active ingredient suitable for the method of administration. The pharmaceutical composition of the present invention includes pharmaceutically acceptable components that are compatible with the polypeptide, nucleic acid, or vector. The pharmaceutical composition may be in tablet or capsule form for oral administration or in aqueous form for intravenous or subcutaneous administration.

As used herein, the term "pharmaceutically acceptable carrier or excipient" refers to an excipient or diluent in a pharmaceutical composition. The pharmaceutically acceptable carrier must be compatible with the other ingredients of the formulation and not deleterious to the recipient. In the present invention, the pharmaceutically acceptable carrier or excipient must provide adequate pharmaceutical stability to the polypeptide including an extracellular ActRIIA variant, an extracellular ActRIIB variant, or an extracellular ActRII chimera, the nucleic acid molecule(s) encoding the polypeptide, or a vector containing such nucleic acid molecule(s). The nature of the carrier or excipient differs with the mode of administration. For example, for intravenous administration, an aqueous solution carrier is generally used; for oral administration, a solid carrier is preferred.

As used herein, the term "treating and/or preventing" refers to the treatment and/or prevention of a disease or condition, e.g., a disease or condition that can be treated with EPO or an ESA, such as end-stage renal disease, renal insufficiency, kidney dialysis, spinal cord injury, an iron overload disorder (e.g., hemochromatosis), an inflammatory brain disease, ischemia, or gastrointestinal dysmotility, using methods and compositions of the invention. Generally, treating a disease or condition that can be treated with EPO or an ESA occurs after a subject has developed the disease or condition. Preventing a disease or condition that can be treated with EPO or an ESA refers to steps or procedures taken when a subject is at risk of developing the disease or condition. The subject may show signs or mild symptoms that are judged by a physician to be indications or risk factors for developing a disease or condition that can be treated with EPO or an ESA, have another disease or condition associated with the development of a disease or condition that can be treated with EPO or an ESA, be undergoing treatment that may cause a disease or condition that can be treated with EPO or an ESA, or have a family history or genetic predisposition of developing a disease or condition that can be treated with EPO or an ESA, but has not yet developed the disease or condition.

As used herein, the term "subject" refers to a mammal, e.g., preferably a human. Mammals include, but are not limited to, humans and domestic and farm animals, such as monkeys (e.g., a cynomolgus monkey), mice, dogs, cats, horses, and cows, etc.

## DESCRIPTION OF THE DRAWINGS

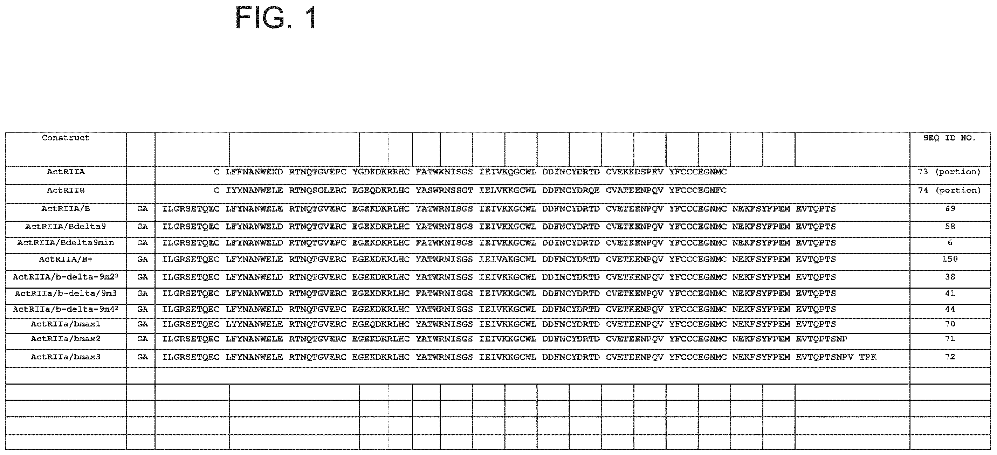

**FIG. 1** is a sequence alignment showing the wild-type sequences of extracellular ActRIIA and ActRIIB and the sequences of exemplary extracellular ActRIIA variants.

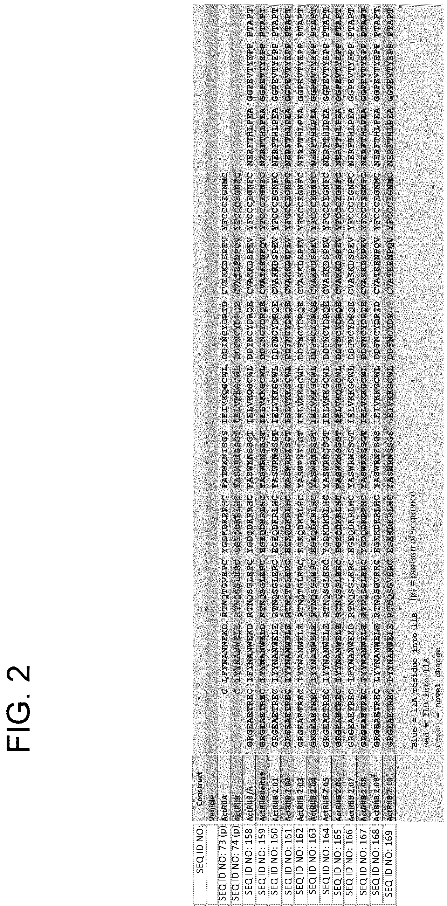

**FIG. 2** is a sequence alignment showing the wild-type sequences of extracellular ActRIIA and ActRIIB and the amino acid substitutions in exemplary extracellular ActRIIB variants.

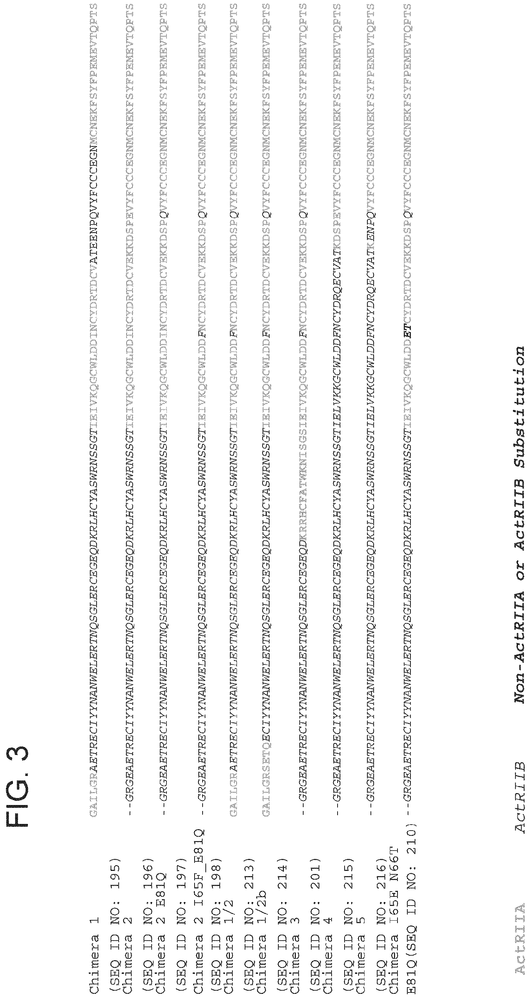

**FIG. 3** is a sequence alignment showing the sequences of exemplary extracellular ActRII chimeras.

**FIG. 4** is a series of graphs showing that a single 10 mg/kg dose of ActRIIA/B-mFc (SEQ ID NO: 69 linked to a mouse Fc domain) administered by intraperitoneal (IP) injection to 11-week-old male mice increased serum EPO levels 4, 7, and 14 days after injection compared to vehicle. Red blood cells were increased at each time point. $*p<0.05$, $**p<0.01$, $****p<0.0001$ between ActRIIA/B-mFc-treated and vehicle at each time point with 2-way ANOVA followed by a Sidak post-test. Data are shown as the mean $\pm$ SEM.

**FIG. 5** is a graph showing that a single 10 mg/kg dose of ActRIIA/B-mFc administered by intraperitoneal (IP) injection to 11-week-old male mice had a long lasting effect on EPO and increased serum EPO levels by day 4 through day 37.

*p≤0.05; **p≤0.01; p****≤0.0001 between ActRIIA/B-mFc-treated and vehicle at each time point by t-test. Data are shown as the mean ± SEM.

FIG. 6 is a series of graphs showing that a single 10mg/kg dose of ActRIIA/B-mFc administered by IP injection to 11-week-old male mice increased EPO receptor levels in bone marrow cells on day 7 and day 14 after injection compared to vehicle. Error bars represent SEM.

## DETAILED DESCRIPTION OF THE INVENTION

The invention features polypeptides that include an extracellular activin receptor type II (ActRII) variant, such as an extracellular ActRIIA variant, an extracellular ActRIIB variant, or an extracellular ActRII chimera. In some embodiments, a polypeptide of the invention includes an extracellular ActRII variant fused to a moiety (e.g., Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin). A polypeptide including an extracellular ActRII variant fused to an Fc domain monomer may also form a dimer (e.g., homodimer or heterodimer) through the interaction between two Fc domain monomers. The ActRII variants described herein have weak binding affinity or no binding affinity to bone morphogenetic protein 9 (BMP9) compared to activins (e.g., activin A and/or activin B) and myostatin. The invention also includes methods of treating diseases and conditions that can be treated with EPO or an ESA (e.g., a disease or condition that can be treated by increasing EPO or EPO receptor levels) by administering to the subject a polypeptide including an extracellular ActRII variant described herein.

### I. Extracellular activin receptor type II (ActRII) variants

Activin type II receptors are single transmembrane domain receptors that modulate signals for ligands in the transforming growth factor β (TGF-β) superfamily. Ligands in the TGF-β superfamily are involved in a host of physiological processes, such as muscle growth, vascular growth, cell differentiation, homeostasis, hematopoiesis, and osteogenesis. Examples of ligands in the TGF-β superfamily include, e.g., activin A, activin B, inhibin, growth differentiation factors (GDFs) (e.g., GDF8, also known as myostatin, and GDF11), and bone morphogenetic proteins (BMPs) (e.g., BMP9).

There exist two types of activin type II receptors: ActRIIA and ActRIIB. Studies have shown that BMP9 binds ActRIIB with about 300-fold higher binding affinity than ActRIIA (see, e.g., Townson et al., J. Biol. Chem. 287:27313, 2012). ActRIIA-Fc is known to have a longer half-life compared to ActRIIB-Fc. Described herein are three types of ActRII variants: 1) extracellular ActRIIA variants that are constructed by introducing amino acid residues of ActRIIB into ActRIIA, with the goal of imparting physiological properties conferred by ActRIIB, while also maintaining beneficial physiological and pharmacokinetic properties of ActRIIA; 2) extracellular ActRIIB variants that are constructed by introducing amino acid residues of ActRIIA into ActRIIB, or by introducing novel amino acid substitutions, with the goal of reducing BMP9 binding to prevent or reduce disruption of endogenous BMP9 signaling; and 3) extracellular ActRII chimeras that are constructed by combining portions of extracellular ActRIIA and ActRIIB with the goal of generating proteins that bind to ActRII ligands (e.g., activin A, activin B, myostatin, and GDF11) and retain the function of wild-type extracellular ActRII proteins.

The present invention is based, in part, on the discovery that administration of a polypeptide including an ActRIIA variant described herein (an ActRIIA variant-Fc polypeptide) increased serum EPO levels in mice in the absence of a compensatory downregulation of bone marrow EPO receptors. Administration of the ActRIIA variant-Fc polypeptide increased both EPO levels in serum and EPO receptor levels in bone marrow precursors. Increases in serum EPO were observed at four days after injection and persisted to at least day 14 after injection, and increases in bone marrow EPO receptor expression were observed seven and 14 days after injection. These data suggest that not only could polypeptides including an ActRIIA variant be used to increase EPO and EPO receptor levels and, therefore, be used as a replacement for EPO therapy, but also that polypeptides including an ActRIIA variant could be administered less frequently than current EPO therapies based on the sustained increases in EPO and EPO receptor levels observed in these studies. Less frequent dosing would greatly improve convenience for patients and could potentially reduce adverse effects. Accordingly, polypeptides including ActRIIA variants described herein could be used to treat diseases or conditions that can be treated with EPO or an ESA.

### ActRIIA variants

In some embodiments, the ActRII variant for use according to the methods described herein is an extracellular ActRIIA variant constructed by introducing amino acid residues of ActRIIB to ActRIIA, with the goal of imparting physiological properties conferred by ActRIIB, while also maintaining beneficial physiological and pharmacokinetic properties of ActRIIA. The preferred ActRIIA variants also exhibit similar or improved binding to activins and/or myostatin compared to wild-type ActRIIA, which allows them to compete with endogenous activin receptors for ligand binding and reduce or inhibit endogenous activin receptor signaling. In some embodiments, amino acid substitutions may be introduced to an extracellular ActRIIA variant to reduce or remove the binding affinity of the variant to BMP9. The wild-type amino acid sequences of the extracellular portions of human ActRIIA and ActRIIB are shown below.

Human ActRIIA, extracellular portion (SEQ ID NO: 73):

GAILGRSETQECLFFNANWEKDRTNQTGVEPCYGDKDKRRHCFATWKNISGSIEIVKQGC
WLDDINCYDRTDCVEKKDSPEVYFCCCEGNMCNEKFSYFPEMEVTQPTS

Human ActRIIB, extracellular portion (SEQ ID NO: 74):

GRGEAETRECIYYNANWELERTNQSGLERCEGEQDKRLHCYASWRNSSGTIELVKKGCWL
DDFNCYDRQECVATEENPQVYFCCCEGNFCNERFTHLPEAGGPEVTYEPPPTAPT

Polypeptides described herein include an extracellular ActRIIA variant having at least one amino acid substitution relative to the wild-type extracellular ActRIIA having the sequence of SEQ ID NO: 73 or the extracellular ActRIIA having any one of the sequences of SEQ ID NOs: 76-96. Possible amino acid substitutions at 27 different positions may be introduced to an extracellular ActRIIA variant (Table 1). In some embodiments, an extracellular ActRIIA variant may have at least 85% (e.g., at least 85%, 87%, 90%, 92%, 95%, 97%, or greater) amino acid sequence identity to the sequence of a wild-type extracellular ActRIIA (SEQ ID NO: 73). An extracellular ActRIIA variant may have one or more (e.g., 1-27, 1-25, 1-23, 1-21, 1-19, 1-17, 1-15, 1-13, 1-11, 1-9, 1-7, 1-5, 1-3, or 1-2; e.g., 1, 2, 3, 4, 5, 6, 7, 8, 9, 10, 11, 12, 13, 14, 15, 16, 17, 18, 19, 20, 21, 22, 23, 24, 25, 26, or 27) amino acid substitutions relative the sequence of a wild-type extracellular ActRIIA (SEQ ID NO: 73). In some embodiments, an extracellular ActRIIA variant (e.g., an extracellular ActRIIA variant having a sequence of SEQ ID NO: 1) may include amino acid substitutions at all of the 27 positions as listed in Table 1. In some embodiments, an extracellular ActRIIA variant may include amino acid substitutions at a number of positions, e.g., at 2, 4, 6, 8, 10, 12, 14, 16, 18, 20, 22, 24, or 26 out of the 27 positions, as listed in Table 1.

Amino acid substitutions can worsen or improve the activity and/or binding affinity of the ActRIIA variants of the invention. To maintain polypeptide function, it is important that the lysine (K) at position $X_{17}$ in the sequences shown in Tables 1 and 2 (SEQ ID NOs: 1-72 (e.g., SEQ ID NOs: 6-72)) be retained. Substitutions at that position can lead to a loss of activity. For example, an ActRIIA variant having the sequence GAILGRSETQECLFYNANWELERTNQTGVERCEGEK DKRLHCYATWRNISGSIEIVAKGCWLDDFNCYD RTDCVETEENPQVYFCCCEGNMCNEKFSYFPEMEVTQPTS (SEQ ID NO: 149) has reduced activity in vivo, indicating that the substitution of alanine (A) for lysine (K) at $X_{17}$ is not tolerated. ActRIIA variants of the invention, including variants in Tables 1 and 2 (e.g., SEQ ID NOs: 1-72 (e.g., SEQ ID NOs: 6-72), therefore, retain amino acid K at position $X_{17}$.

The ActRIIA variants of the invention preferably have reduced, weak, or no substantial binding to BMP9. BMP9 binding is reduced in ActRIIA variants (e.g., reduced compared to wild-type ActRIIA) containing the amino acid sequence TEEN (SEQ ID NO: 374) at positions $X_{23}$, $X_{24}$, $X_{25}$, and $X_{26}$, as well as in variants that maintain the amino acid K at position $X_{24}$ and have the amino acid sequence TKEN (SEQ ID NO: 375) at positions $X_{23}$, $X_{24}$, $X_{25}$, and $X_{26}$. The sequences TEEN (SEQ ID NO: 374) and TKEN (SEQ ID NO: 375) can be employed interchangeably in the ActRIIA variants (e.g., the variants in Tables 1 and 2, e.g., SEQ ID NOs: 1-72 (e.g., SEQ ID NOs: 6-72)) of the invention to provide reduced BMP9 binding.

The ActRIIA variants of the invention may further include a C-terminal extension (e.g., additional amino acids at the C-terminus). The C-terminal extension can add one or more additional amino acids at the C-terminus (e.g., 1, 2, 3, 4, 5, 6 or more additional amino acids) to any of the variants shown in Tables 1 and 2 (e.g., SEQ ID NOs: 1-70 (e.g., SEQ ID NOs: 6-70)). The C-terminal extension may correspond to sequence from the same position in wild-type ActRIIA. One potential C-terminal extension that can be included in the ActRIIA variants of the invention is amino acid sequence NP. For example, a sequence including the C-terminal extension NP is SEQ ID NO: 71 (e.g., SEQ ID NO: 69 with a C-terminal extension of NP). Another exemplary C-terminal extension that can be included in the ActRIIA variants of the invention is amino acid sequence NPVTPK (SEQ ID NO: 154). For example, a sequence including the C-terminal extension NPVTPK is SEQ ID NO: 72 (e.g., SEQ ID NO: 69 with a C-terminal extension of NPVTPK).

Table 1. Amino acid substitutions in an extracellular ActRIIA variant having a sequence of any one of SEQ ID NOs: 1-5

GAILGRSETQECLX$_1$X$_2$NANWX$_3$X$_4$X$_5$X$_6$TNQTGVEX$_7$CX$_8$GX$_9$X$_{10}$X$_{11}$X$_{12}$X$_{13}$X$_{14}$HCX$_{15}$ATWX$_{16}$NISGSIEIV
X$_{17}$X$_{18}$GCX$_{19}$X$_{20}$X$_{21}$DX$_{22}$NCYDRTDCVEX$_{23}$X$_{24}$X$_{25}$X$_{26}$PX$_{27}$VYFCCCEGNMCNEKFSYFPEMEVTQPTS
(SEQ ID NO: 1)

GAILGRSETQECLFX$_2$NANWX$_3$X$_4$X$_5$X$_6$TNQTGVEX$_7$CX$_8$GX$_9$KX$_{11}$X$_{12}$X$_{13}$X$_{14}$HCX$_{15}$ATWX$_{16}$NISGSIEIVX$_{17}$X$_{18}$GCX$_{19}$X$_{20}$X$_{21}$DX$_{22}$NCYDRTDCVEX$_{23}$X$_{24}$X$_{25}$X$_{26}$PX$_{27}$VYFCCCEGNMCNEKFSYFPEMEVTQPTS
(SEQ ID NO: 2)

GAILGRSETQECLFX$_2$NANWEX$_4$X$_5$RTNQTGVEX$_7$CX$_8$GX$_9$KDKRX$_{14}$HCX$_{15}$ATWX$_{16}$NISGSIEIVKX$_{18}$GC
WLDDX$_{22}$NCYDRTDCVEX$_{23}$X$_{24}$X$_{25}$X$_{26}$PX$_{27}$VYFCCCEGNMCNEKFSYFPEMEVTQPTS (SEQ ID NO: 3)

GAILGRSETQECLFX$_2$NANWEX$_4$DRTNQTGVEX$_7$CX$_8$GX$_9$KDKRX$_{14}$HCX$_{15}$ATWX$_{16}$NISGSIEIVKX$_{18}$GC
WLDDX$_{22}$NCYDRTDCVEX$_{23}$KX$_{25}$X$_{26}$PX$_{27}$VYFCCCEGNMCNEKFSYFPEMEVTQPTS (SEQ ID NO: 4)

GAILGRSETQECLFX$_2$NANWEX$_4$DRTNQTGVEPCX$_8$GX$_9$KDKRX$_{14}$HCFATWKNISGSIEIVKX$_{18}$GCWLD
DINCYDRTDCVEX$_{23}$KX$_{25}$X$_{26}$PX$_{27}$VYFCCCEGNMCNEKFSYFPEMEVTQPTS (SEQ ID NO: 5)

| X$_1$ | F or Y | X$_{15}$ | F or Y |
|---|---|---|---|
| X$_2$ | F or Y | X$_{16}$ | K, R, or A |
| X$_3$ | E or A | X$_{17}$ | K, A, Y, F, or I |
| X$_4$ | K or L | X$_{18}$ | Q or K |
| X$_5$ | D or E | X$_{19}$ | W or A |
| X$_6$ | R or A | X$_{20}$ | L or A |
| X$_7$ | P or R | X$_{21}$ | D, K, R, A, F, G, M, N, or I |
| X$_8$ | Y or E | X$_{22}$ | I, F, or A |
| X$_9$ | D or E | X$_{23}$ | K or T |
| X$_{10}$ | K or Q | X$_{24}$ | K or E |
| X$_{11}$ | D or A | X$_{25}$ | D or E |
| X$_{12}$ | K or A | X$_{26}$ | S or N |
| X$_{13}$ | R or A | X$_{27}$ | E or Q |
| X$_{14}$ | R or L | | |

In some embodiments of the extracellular ActRIIA variant having the sequence of SEQ ID NO: 1 or 2, $X_3$ is E, $X_6$ is R, $X_{11}$ is D, $X_{12}$ is K, $X_{13}$ is R, $X_{16}$ is K or R, $X_{17}$ is K, $X_{19}$ is W, $X_{20}$ is L, $X_{21}$ is D, and $X_{22}$ is I or F. In some embodiments of the extracellular ActRIIA variant having the sequence of SEQ ID NO: 1, $X_2$ is Y; $X_4$ is L; $X_8$ is E; $X_9$ is E; $X_{14}$ is L; $X_{18}$ is K; $X_{23}$ is T; $X_{25}$ is E; $X_{26}$ is N; and $X_{27}$ is Q. These substitutions in SEQ ID NO: 1 can also be made in SEQ ID NOs: 2-5. In some embodiments of the extracellular ActRIIA variant having the sequence of SEQ ID NO: 1, $X_1$ is F or Y; $X_2$ is Y; $X_4$ is L; $X_5$ is D or E; $X_7$ is P or R; $X_8$ is E; $X_9$ is E; $X_{10}$ is K or Q; $X_{14}$ is L; $X_{15}$ is F or Y; $X_{16}$ is K or R; $X_{18}$ is K; $X_{22}$ is I or F; $X_{23}$ is T; $X_{24}$ is K or E; $X_{25}$ is E; $X_{26}$ is N; and $X_{27}$ is Q. In some embodiments of the extracellular ActRIIA variant having the sequence of SEQ ID NO: 1, $X_1$ is F or Y; $X_2$ is Y; $X_3$ is E; $X_4$ is L; $X_5$ is D or E; $X_6$ is R; $X_7$ is P or R; $X_8$ is E; $X_9$ is E; $X_{10}$ is K or Q; $X_{11}$ is D; $X_{12}$ is K; $X_{13}$ is R; $X_{14}$ is L; $X_{15}$ is F or Y; $X_{16}$ is K or R; $X_{17}$ is K; $X_{18}$ is K; $X_{19}$ is W; $X_{20}$ is L; $X_{21}$ is D; $X_{22}$ is I or F; $X_{23}$ is T; $X_{24}$ is K or E; $X_{25}$ is E; $X_{26}$ is N; and $X_{27}$ is Q. In some embodiments of the extracellular ActRIIA variant having the sequence of SEQ ID NO: 1 or 2, $X_{17}$ is K. In some embodiments of the extracellular ActRIIA variant having the sequence of SEQ ID NOs: 1-3, $X_{17}$ is K, $X_{23}$ is T, $X_{24}$ is E, $X_{25}$ is E, and $X_{26}$ is N. In some embodiments of the extracellular ActRIIA variant having the sequence of any one of SEQ ID NOs: 1-5, $X_{17}$ is K, $X_{23}$ is T, $X_{24}$ is K, $X_{25}$ is E, and $X_{26}$ is N.

In some embodiments, a polypeptide described herein includes an extracellular ActRIIA variant having a sequence of any one of SEQ ID NOs: 6-72 (Table 2).

Table 2. Extracellular ActRIIA variants having the sequences of SEQ ID NOs: 6-72

| SEQ ID NO | Amino Acid Sequence |
|---|---|
| 6 | GAILGRSETQECLFYNANWELDRTNQTGVEPCEGEKDKRLHCFATWKNISGSIEIV KKGCWLDDINCYDRTDCVETKENPQVYFCCCEGNMCNEKFSYFPEMEVTQPTS |
| 7 | GAILGRSETQECLFYNANWELERTNQTGVEPCEGEKDKRLHCFATWKNISGSIEIV KKGCWLDDINCYDRTDCVETKENPQVYFCCCEGNMCNEKFSYFPEMEVTQPTS |
| 8 | GAILGRSETQECLFYNANWELDRTNQTGVERCEGEKDKRLHCFATWKNISGSIEIV KKGCWLDDINCYDRTDCVETKENPQVYFCCCEGNMCNEKFSYFPEMEVTQPTS |
| 9 | GAILGRSETQECLFYNANWELDRTNQTGVEPCEGEKDKRLHCYATWKNISGSIEIV KKGCWLDDINCYDRTDCVETKENPQVYFCCCEGNMCNEKFSYFPEMEVTQPTS |
| 10 | GAILGRSETQECLFYNANWELDRTNQTGVEPCEGEKDKRLHCFATWRNISGSIEIV KKGCWLDDINCYDRTDCVETKENPQVYFCCCEGNMCNEKFSYFPEMEVTQPTS |
| 11 | GAILGRSETQECLFYNANWELDRTNQTGVEPCEGEKDKRLHCFATWKNISGSIEIV KKGCWLDDFNCYDRTDCVETKENPQVYFCCCEGNMCNEKFSYFPEMEVTQPTS |
| 12 | GAILGRSETQECLFYNANWELDRTNQTGVEPCEGEKDKRLHCFATWKNISGSIEIV KKGCWLDDINCYDRTDCVETEENPQVYFCCCEGNMCNEKFSYFPEMEVTQPTS |
| 13 | GAILGRSETQECLFYNANWELERTNQTGVERCEGEKDKRLHCFATWKNISGSIEIV KKGCWLDDINCYDRTDCVETKENPQVYFCCCEGNMCNEKFSYFPEMEVTQPTS |
| 14 | GAILGRSETQECLFYNANWELERTNQTGVEPCEGEKDKRLHCYATWKNISGSIEIV KKGCWLDDINCYDRTDCVETKENPQVYFCCCEGNMCNEKFSYFPEMEVTQPTS |
| 15 | GAILGRSETQECLFYNANWELERTNQTGVEPCEGEKDKRLHCFATWRNISGSIEIV KKGCWLDDINCYDRTDCVETKENPQVYFCCCEGNMCNEKFSYFPEMEVTQPTS |
| 16 | GAILGRSETQECLFYNANWELERTNQTGVEPCEGEKDKRLHCFATWKNISGSIEIV KKGCWLDDFNCYDRTDCVETKENPQVYFCCCEGNMCNEKFSYFPEMEVTQPTS |
| 17 | GAILGRSETQECLFYNANWELERTNQTGVEPCEGEKDKRLHCFATWKNISGSIEIV KKGCWLDDINCYDRTDCVETEENPQVYFCCCEGNMCNEKFSYFPEMEVTQPTS |

(continued)

| SEQ ID NO | Amino Acid Sequence |
|---|---|
| 18 | GAILGRSETQECLFYNANWELDRTNQTGVERCEGEKDKRLHCYATWKNISGSIEIV KKGCWLDDINCYDRTDCVETKENPQVYFCCCEGNMCNEKFSYFPEMEVTQPTS |
| 19 | GAILGRSETQECLFYNANWELDRTNQTGVERCEGEKDKRLHCFATWRNISGSIEIV KKGCWLDDINCYDRTDCVETKENPQVYFCCCEGNMCNEKFSYFPEMEVTQPTS |
| 20 | GAILGRSETQECLFYNANWELDRTNQTGVERCEGEKDKRLHCFATWKNISGSIEIV KKGCWLDDFNCYDRTDCVETKENPQVYFCCCEGNMCNEKFSYFPEMEVTQPTS |
| 21 | GAILGRSETQECLFYNANWELDRTNQTGVERCEGEKDKRLHCFATWKNISGSIEIV KKGCWLDDINCYDRTDCVETEENPQVYFCCCEGNMCNEKFSYFPEMEVTQPTS |
| 22 | GAILGRSETQECLFYNANWELDRTNQTGVEPCEGEKDKRLHCYATWRNISGSIEIV KKGCWLDDINCYDRTDCVETKENPQVYFCCCEGNMCNEKFSYFPEMEVTQPTS |
| 23 | GAILGRSETQECLFYNANWELDRTNQTGVEPCEGEKDKRLHCYATWKNISGSIEIV KKGCWLDDFNCYDRTDCVETKENPQVYFCCCEGNMCNEKFSYFPEMEVTQPTS |
| 24 | GAILGRSETQECLFYNANWELDRTNQTGVEPCEGEKDKRLHCYATWKNISGSIEIV KKGCWLDDINCYDRTDCVETEENPQVYFCCCEGNMCNEKFSYFPEMEVTQPTS |
| 25 | GAILGRSETQECLFYNANWELDRTNQTGVEPCEGEKDKRLHCFATWRNISGSIEIV KKGCWLDDFNCYDRTDCVETKENPQVYFCCCEGNMCNEKFSYFPEMEVTQPTS |
| 26 | GAILGRSETQECLFYNANWELDRTNQTGVEPCEGEKDKRLHCFATWRNISGSIEIV KKGCWLDDINCYDRTDCVETEENPQVYFCCCEGNMCNEKFSYFPEMEVTQPTS |
| 27 | GAILGRSETQECLFYNANWELDRTNQTGVEPCEGEKDKRLHCFATWKNISGSIEIV KKGCWLDDFNCYDRTDCVETEENPQVYFCCCEGNMCNEKFSYFPEMEVTQPTS |
| 28 | GAILGRSETQECLFYNANWELERTNQTGVERCEGEKDKRLHCYATWKNISGSIEIV KKGCWLDDINCYDRTDCVETKENPQVYFCCCEGNMCNEKFSYFPEMEVTQPTS |
| 29 | GAILGRSETQECLFYNANWELERTNQTGVERCEGEKDKRLHCFATWRNISGSIEIV KKGCWLDDINCYDRTDCVETKENPQVYFCCCEGNMCNEKFSYFPEMEVTQPTS |
| 30 | GAILGRSETQECLFYNANWELERTNQTGVERCEGEKDKRLHCFATWKNISGSIEIV KKGCWLDDFNCYDRTDCVETKENPQVYFCCCEGNMCNEKFSYFPEMEVTQPTS |
| 31 | GAILGRSETQECLFYNANWELERTNQTGVERCEGEKDKRLHCFATWKNISGSIEIV KKGCWLDDINCYDRTDCVETEENPQVYFCCCEGNMCNEKFSYFPEMEVTQPTS |
| 32 | GAILGRSETQECLFYNANWELERTNQTGVEPCEGEKDKRLHCYATWRNISGSIEIV KKGCWLDDINCYDRTDCVETKENPQVYFCCCEGNMCNEKFSYFPEMEVTQPTS |
| 33 | GAILGRSETQECLFYNANWELERTNQTGVEPCEGEKDKRLHCYATWKNISGSIEIV KKGCWLDDFNCYDRTDCVETKENPQVYFCCCEGNMCNEKFSYFPEMEVTQPTS |

(continued)

| SEQ ID NO | Amino Acid Sequence |
|---|---|
| 34 | GAILGRSETQECLFYNANWELERTNQTGVEPCEGEKDKRLHCYATWKNISGSIEIV KKGCWLDDINCYDRTDCVETEENPQVYFCCCEGNMCNEKFSYFPEMEVTQPTS |
| 35 | GAILGRSETQECLFYNANWELERTNQTGVEPCEGEKDKRLHCFATWRNISGSIEIV KKGCWLDDFNCYDRTDCVETKENPQVYFCCCEGNMCNEKFSYFPEMEVTQPTS |
| 36 | GAILGRSETQECLFYNANWELERTNQTGVEPCEGEKDKRLHCFATWRNISGSIEIV KKGCWLDDINCYDRTDCVETEENPQVYFCCCEGNMCNEKFSYFPEMEVTQPTS |
| 37 | GAILGRSETQECLFYNANWELERTNQTGVEPCEGEKDKRLHCFATWKNISGSIEIV KKGCWLDDFNCYDRTDCVETEENPQVYFCCCEGNMCNEKFSYFPEMEVTQPTS |
| 38 | GAILGRSETQECLFYNANWELDRTNQTGVERCEGEKDKRLHCYATWRNISGSIEIV KKGCWLDDINCYDRTDCVETKENPQVYFCCCEGNMCNEKFSYFPEMEVTQPTS |
| 39 | GAILGRSETQECLFYNANWELDRTNQTGVERCEGEKDKRLHCYATWKNISGSIEIV KKGCWLDDFNCYDRTDCVETKENPQVYFCCCEGNMCNEKFSYFPEMEVTQPTS |
| 40 | GAILGRSETQECLFYNANWELDRTNQTGVERCEGEKDKRLHCYATWKNISGSIEIV KKGCWLDDINCYDRTDCVETEENPQVYFCCCEGNMCNEKFSYFPEMEVTQPTS |
| 41 | GAILGRSETQECLFYNANWELDRTNQTGVERCEGEKDKRLHCFATWRNISGSIEIV KKGCWLDDFNCYDRTDCVETKENPQVYFCCCEGNMCNEKFSYFPEMEVTQPTS |
| 42 | GAILGRSETQECLFYNANWELDRTNQTGVERCEGEKDKRLHCFATWRNISGSIEIV KKGCWLDDINCYDRTDCVETEENPQVYFCCCEGNMCNEKFSYFPEMEVTQPTS |
| 43 | GAILGRSETQECLFYNANWELDRTNQTGVERCEGEKDKRLHCFATWKNISGSIEIV KKGCWLDDFNCYDRTDCVETEENPQVYFCCCEGNMCNEKFSYFPEMEVTQPTS |
| 44 | GAILGRSETQECLFYNANWELDRTNQTGVEPCEGEKDKRLHCYATWRNISGSIEIV KKGCWLDDFNCYDRTDCVETKENPQVYFCCCEGNMCNEKFSYFPEMEVTQPTS |
| 45 | GAILGRSETQECLFYNANWELDRTNQTGVEPCEGEKDKRLHCYATWRNISGSIEIV KKGCWLDDINCYDRTDCVETEENPQVYFCCCEGNMCNEKFSYFPEMEVTQPTS |
| 46 | GAILGRSETQECLFYNANWELDRTNQTGVEPCEGEKDKRLHCYATWKNISGSIEIV KKGCWLDDINCYDRTDCVETEENPQVYFCCCEGNMCNEKFSYFPEMEVTQPTS |
| 47 | GAILGRSETQECLFYNANWELDRTNQTGVEPCEGEKDKRLHCFATWRNISGSIEIV KKGCWLDDFNCYDRTDCVETEENPQVYFCCCEGNMCNEKFSYFPEMEVTQPTS |
| 48 | GAILGRSETQECLFYNANWELERTNQTGVERCEGEKDKRLHCYATWRNISGSIEIV KKGCWLDDINCYDRTDCVETKENPQVYFCCCEGNMCNEKFSYFPEMEVTQPTS |
| 49 | GAILGRSETQECLFYNANWELERTNQTGVERCEGEKDKRLHCYATWKNISGSIEIV KKGCWLDDFNCYDRTDCVETKENPQVYFCCCEGNMCNEKFSYFPEMEVTQPTS |

(continued)

| SEQ ID NO | Amino Acid Sequence |
|---|---|
| 50 | GAILGRSETQECLFYNANWELERTNQTGVERCEGEKDKRLHCYATWKNISGSIEIV KKGCWLDDINCYDRTDCVETEENPQVYFCCCEGNMCNEKFSYFPEMEVTQPTS |
| 51 | GAILGRSETQECLFYNANWELERTNQTGVERCEGEKDKRLHCFATWRNISGSIEIV KKGCWLDDFNCYDRTDCVETKENPQVYFCCCEGNMCNEKFSYFPEMEVTQPTS |
| 52 | GAILGRSETQECLFYNANWELERTNQTGVERCEGEKDKRLHCFATWRNISGSIEIV KKGCWLDDINCYDRTDCVETEENPQVYFCCCEGNMCNEKFSYFPEMEVTQPTS |
| 53 | GAILGRSETQECLFYNANWELERTNQTGVERCEGEKDKRLHCFATWKNISGSIEIV KKGCWLDDFNCYDRTDCVETEENPQVYFCCCEGNMCNEKFSYFPEMEVTQPTS |
| 54 | GAILGRSETQECLFYNANWELERTNQTGVEPCEGEKDKRLHCYATWRNISGSIEIV KKGCWLDDFNCYDRTDCVETKENPQVYFCCCEGNMCNEKFSYFPEMEVTQPTS |
| 55 | GAILGRSETQECLFYNANWELERTNQTGVEPCEGEKDKRLHCYATWRNISGSIEIV KKGCWLDDINCYDRTDCVETEENPQVYFCCCEGNMCNEKFSYFPEMEVTQPTS |
| 56 | GAILGRSETQECLFYNANWELERTNQTGVEPCEGEKDKRLHCYATWKNISGSIEIV KKGCWLDDFNCYDRTDCVETEENPQVYFCCCEGNMCNEKFSYFPEMEVTQPTS |
| 57 | GAILGRSETQECLFYNANWELERTNQTGVEPCEGEKDKRLHCFATWRNISGSIEIV KKGCWLDDFNCYDRTDCVETEENPQVYFCCCEGNMCNEKFSYFPEMEVTQPTS |
| 58 | GAILGRSETQECLFYNANWELDRTNQTGVERCEGEKDKRLHCYATWRNISGSIEIV KKGCWLDDFNCYDRTDCVETKENPQVYFCCCEGNMCNEKFSYFPEMEVTQPTS |
| 59 | GAILGRSETQECLFYNANWELDRTNQTGVERCEGEKDKRLHCYATWRNISGSIEIV KKGCWLDDINCYDRTDCVETEENPQVYFCCCEGNMCNEKFSYFPEMEVTQPTS |
| 60 | GAILGRSETQECLFYNANWELDRTNQTGVERCEGEKDKRLHCYATWKNISGSIEIV KKGCWLDDFNCYDRTDCVETEENPQVYFCCCEGNMCNEKFSYFPEMEVTQPTS |
| 61 | GAILGRSETQECLFYNANWELDRTNQTGVERCEGEKDKRLHCFATWRNISGSIEIV KKGCWLDDFNCYDRTDCVETEENPQVYFCCCEGNMCNEKFSYFPEMEVTQPTS |
| 62 | GAILGRSETQECLFYNANWELDRTNQTGVEPCEGEKDKRLHCYATWRNISGSIEIV KKGCWLDDFNCYDRTDCVETEENPQVYFCCCEGNMCNEKFSYFPEMEVTQPTS |
| 63 | GAILGRSETQECLFYNANWELERTNQTGVERCEGEKDKRLHCYATWRNISGSIEIV KKGCWLDDFNCYDRTDCVETKENPQVYFCCCEGNMCNEKFSYFPEMEVTQPTS |
| 64 | GAILGRSETQECLFYNANWELERTNQTGVERCEGEKDKRLHCYATWRNISGSIEIV KKGCWLDDINCYDRTDCVETEENPQVYFCCCEGNMCNEKFSYFPEMEVTQPTS |
| 65 | GAILGRSETQECLFYNANWELERTNQTGVERCEGEKDKRLHCYATWKNISGSIEIV KKGCWLDDFNCYDRTDCVETEENPQVYFCCCEGNMCNEKFSYFPEMEVTQPTS |

(continued)

| SEQ ID NO | Amino Acid Sequence |
|---|---|
| 66 | GAILGRSETQECLFYNANWELERTNQTGVERCEGEKDKRLHCFATWRNISGSIEIV KKGCWLDDFNCYDRTDCVETEENPQVYFCCCEGNMCNEKFSYFPEMEVTQPTS |
| 67 | GAILGRSETQECLFYNANWELDRTNQTGVERCEGEKDKRLHCYATWRNISGSIEIV KKGCWLDDFNCYDRTDCVETEENPQVYFCCCEGNMCNEKFSYFPEMEVTQPTS |
| 68 | GAILGRSETQECLFYNANWELERTNQTGVEPCEGEKDKRLHCYATWRNISGSIEIV KKGCWLDDFNCYDRTDCVETEENPQVYFCCCEGNMCNEKFSYFPEMEVTQPTS |
| 69 | GAILGRSETQECLFYNANWELERTNQTGVERCEGEKDKRLHCYATWRNISGSIEIV KKGCWLDDFNCYDRTDCVETEENPQVYFCCCEGNMCNEKFSYFPEMEVTQPTS |
| 70 | GAILGRSETQECLYYNANWELERTNQTGVERCEGEQDKRLHCYATWRNISGSIEIV KKGCWLDDFNCYDRTDCVETEENPQVYFCCCEGNMCNEKFSYFPEMEVTQPTS |
| 71 | GAILGRSETQECLFYNANWELERTNQTGVERCEGEKDKRLHCYATWRNISGSIEIV KKGCWLDDFNCYDRTDCVETEENPQVYFCCCEGNMCNEKFSYFPEMEVTQPTSN P |
| 72 | GAILGRSETQECLFYNANWELERTNQTGVERCEGEKDKRLHCYATWRNISGSIEIV KKGCWLDDFNCYDRTDCVETEENPQVYFCCCEGNMCNEKFSYFPEMEVTQPTSN PVTPK |

In some embodiments, a polypeptide of the invention including an extracellular ActRIIA variant (e.g., any one of SEQ ID NOs: 1-72 (e.g., SEQ ID NOs: 6-72)) has amino acid K at position $X_{17}$. Altering the amino acid at position $X_{17}$ can result in reduced activity. For example, an ActRIIA variant having the sequence GAILGRSETQECLFYNANWELERTN QTGVERCEGEKDKRLHCYATWRNISGSIEIVAKGCWLDDFNCYD RTDCVETEENPQVYFCCCEGNMCNEKFSYFPE-MEVTQPTS (SEQ ID NO: 149) has reduced activity in vivo, indicating that the substitution of A for K at $X_{17}$ is not tolerated.

In some embodiments, a polypeptide of the invention including an extracellular ActRIIA variant (e.g., any one of SEQ ID NOs: 1-72 (e.g., SEQ ID NOs: 6-72)) with the sequence TEEN (SEQ ID NO: 374) at positions $X_{23}$, $X_{24}$, $X_{25}$, and $X_{26}$ can have a substitution of the amino acid K for the amino acid E at position $X_{24}$. In some embodiments, a polypeptide of the invention including an extracellular ActRIIA variant (e.g., any one of SEQ ID NOs: 1-72 (e.g., SEQ ID NOs: 6-72)) with the sequence TKEN (SEQ ID NO: 375) at positions $X_{23}$, $X_{24}$, $X_{25}$, and $X_{26}$ can have a substitution of the amino acid E for the amino acid K at position $X_{24}$. Polypeptides having the sequence TEEN (SEQ ID NO: 374) or TKEN (SEQ ID NO: 375) at positions $X_{23}$, $X_{24}$, $X_{25}$, and $X_{26}$ have reduced or weak binding to BMP9 (e.g., reduced binding to BMP9 compared to BMP9 binding of wild-type ActRIIA).

In some embodiments, a polypeptide of the invention including an extracellular ActRIIA variant (e.g., any one of SEQ ID NOs: 1-70 (e.g., SEQ ID NOs: 6-70)) may further include a C-terminal extension (e.g., one more additional amino acids at the C-terminus). The C-terminal extension may correspond to sequence from the same position in wild-type ActRIIA. In some embodiments, the C-terminal extension is amino acid sequence NP. For example, a sequence including the C-terminal extension NP is SEQ ID NO: 71 (e.g., SEQ ID NO: 69 with a C-terminal extension of NP). In some embodiments, the C-terminal extension is amino acid sequence NPVTPK (SEQ ID NO: 154). For example, a sequence including the C-terminal extension NPVTPK is SEQ ID NO: 72 (e.g., SEQ ID NO: 69 with a C-terminal extension of NPVTPK). The C-terminal extension can add one or more amino acids at the C-terminus (e.g., 1, 2, 3, 4, 5, 6 or more additional amino acids).

In some embodiments, a polypeptide of the invention including an extracellular ActRIIA variant may further include a moiety (e.g., Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin), which may be fused to the N or C-terminus (e.g., C-terminus) of the extracellular ActRIIA variant by way of a linker or other covalent bonds. A polypeptide including an extracellular ActRIIA variant fused to an Fc domain monomer may form a dimer (e.g., homodimer or heterodimer) through the interaction between two Fc domain monomers, which combine to form an Fc domain in the dimer.

In some embodiments, an extracellular ActRIIA variant described herein does not have the sequence of any one of SEQ ID NOs: 76-96 shown in Table 3 below.

Table 3. Excluded Extracellular ActRIIA Variants

| SEQ ID NO | Amino Acid Sequence |
|---|---|
| 76 | GAILGRSETQECLFFNANWEKDRTNQTGVEPCYGDKDKRRHCFATWANISGSIEIV KQGCWLDDINCYDRTDCVEKKDSPEVYFCCCEGNMCNEKFSYFPEMEVTQPTS |
| 77 | GAILGRSETQECLFFNANWAKDRTNQTGVEPCYGDKDKRRHCFATWKNISGSIEIV KQGCWLDDINCYDRTDCVEKKDSPEVYFCCCEGNMCNEKFSYFPEMEVTQPTS |
| 78 | GAILGRSETQECLFFNANWEKDATNQTGVEPCYGDKDKRRHCFATWKNISGSIEIV KQGCWLDDINCYDRTDCVEKKDSPEVYFCCCEGNMCNEKFSYFPEMEVTQPTS |
| 79 | GAILGRSETQECLFFNANWEKDRTNQTGVEPCYGDKAKRRHCFATWKNISGSIEIV KQGCWLDDINCYDRTDCVEKKDSPEVYFCCCEGNMCNEKFSYFPEMEVTQPTS |
| 80 | GAILGRSETQECLFFNANWEKDRTNQTGVEPCYGDKDARRHCFATWKNISGSIEIV KQGCWLDDINCYDRTDCVEKKDSPEVYFCCCEGNMCNEKFSYFPEMEVTQPTS |
| 81 | GAILGRSETQECLFFNANWEKDRTNQTGVEPCYGDKDKARHCFATWKNISGSIEIV KQGCWLDDINCYDRTDCVEKKDSPEVYFCCCEGNMCNEKFSYFPEMEVTQPTS |
| 82 | GAILGRSETQECLFFNANWEKDRTNQTGVEPCYGDKDKRRHCFATWKNISGSIEIV AQGCWLDDINCYDRTDCVEKKDSPEVYFCCCEGNMCNEKFSYFPEMEVTQPTS |
| 83 | GAILGRSETQECLFFNANWEKDRTNQTGVEPCYGDKDKRRHCFATWKNISGSIEIV YQGCWLDDINCYDRTDCVEKKDSPEVYFCCCEGNMCNEKFSYFPEMEVTQPTS |
| 84 | GAILGRSETQECLFFNANWEKDRTNQTGVEPCYGDKDKRRHCFATWKNISGSIEIV FQGCWLDDINCYDRTDCVEKKDSPEVYFCCCEGNMCNEKFSYFPEMEVTQPTS |
| 85 | GAILGRSETQECLFFNANWEKDRTNQTGVEPCYGDKDKRRHCFATWKNISGSIEIVI QGCWLDDINCYDRTDCVEKKDSPEVYFCCCEGNMCNEKFSYFPEMEVTQPTS |
| 86 | GAILGRSETQECLFFNANWEKDRTNQTGVEPCYGDKDKRRHCFATWKNISGSIEIV KQGCALDDINCYDRTDCVEKKDSPEVYFCCCEGNMCNEKFSYFPEMEVTQPTS |
| 87 | GAILGRSETQECLFFNANWEKDRTNQTGVEPCYGDKDKRRHCFATWKNISGSIEIV KQGCWADDINCYDRTDCVEKKDSPEVYFCCCEGNMCNEKFSYFPEMEVTQPTS |
| 88 | GAILGRSETQECLFFNANWEKDRTNQTGVEPCYGDKDKRRHCFATWKNISGSIEIV KQGCWLKDINCYDRTDCVEKKDSPEVYFCCCEGNMCNEKFSYFPEMEVTQPTS |
| 89 | GAILGRSETQECLFFNANWEKDRTNQTGVEPCYGDKDKRRHCFATWKNISGSIEIV KQGCWLRDINCYDRTDCVEKKDSPEVYFCCCEGNMCNEKFSYFPEMEVTQPTS |
| 90 | GAILGRSETQECLFFNANWEKDRTNQTGVEPCYGDKDKRRHCFATWKNISGSIEIV KQGCWLADINCYDRTDCVEKKDSPEVYFCCCEGNMCNEKFSYFPEMEVTQPTS |

(continued)

| SEQ ID NO | Amino Acid Sequence |
|---|---|
| 91 | GAILGRSETQECLFFNANWEKDRTNQTGVEPCYGDKDKRRHCFATWKNISGSIEIV KQGCWLFDINCYDRTDCVEKKDSPEVYFCCCEGNMCNEKFSYFPEMEVTQPTS |
| 92 | GAILGRSETQECLFFNANWEKDRTNQTGVEPCYGDKDKRRHCFATWKNISGSIEIV KQGCWLGDINCYDRTDCVEKKDSPEVYFCCCEGNMCNEKFSYFPEMEVTQPTS |
| 93 | GAILGRSETQECLFFNANWEKDRTNQTGVEPCYGDKDKRRHCFATWKNISGSIEIV KQGCWLMDINCYDRTDCVEKKDSPEVYFCCCEGNMCNEKFSYFPEMEVTQPTS |
| 94 | GAILGRSETQECLFFNANWEKDRTNQTGVEPCYGDKDKRRHCFATWKNISGSIEIV KQGCWLNDINCYDRTDCVEKKDSPEVYFCCCEGNMCNEKFSYFPEMEVTQPTS |
| 95 | GAILGRSETQECLFFNANWEKDRTNQTGVEPCYGDKDKRRHCFATWKNISGSIEIV KQGCWLIDINCYDRTDCVEKKDSPEVYFCCCEGNMCNEKFSYFPEMEVTQPTS |
| 96 | GAILGRSETQECLFFNANWEKDRTNQTGVEPCYGDKDKRRHCFATWKNISGSIEIV KQGCWLDDANCYDRTDCVEKKDSPEVYFCCCEGNMCNEKFSYFPEMEVTQPTS |

Furthermore, in some embodiments, a polypeptide described herein (e.g., an ActRIIA variant-Fc fusion protein) has a serum half-life of at least 7 days in humans. The polypeptide including an extracellular ActRIIA variant may bind to activin A with a $K_D$ of 10 pM or higher. In some embodiments, the polypeptide including an extracellular ActRIIA variant does not bind to BMP9 or activin A. In some embodiments, the polypeptide including an extracellular ActRIIA variant binds to activin A, activin B, and/or myostatin and exhibits reduced (e.g., weak) binding to BMP9 (e.g., reduced BMP9 binding compared to BMP9 binding of wild-type ActRIIA). In some embodiments, the polypeptide including an extracellular ActRIIA variant that has reduced or weak binding to BMP9 has the sequence TEEN (SEQ ID NO: 374) or TKEN (SEQ ID NO: 375) at positions $X_{23}$, $X_{24}$, $X_{25}$, and $X_{26}$. In some embodiments, the polypeptide including an extracellular ActRIIA variant does not substantially bind to human BMP9.

In some embodiments, the polypeptide including an extracellular ActRIIA variant may bind to human activin A with a $K_D$ of about 800 pM or less (e.g., a $K_D$ of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a $K_D$ of between about 800 pM and about 200 pM). In some embodiments, the polypeptide including an extracellular ActRIIA variant may bind to human activin B with a $K_D$ of 800 pM or less (e.g., a $K_D$ of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a $K_D$ of between about 800 pM and about 200 pM) The polypeptide including an extracellular ActRIIA variant may also bind to growth and differentiation factor 11 (GDF-11) with a $K_D$ of approximately 5 pM or higher (e.g., a $K_D$ of about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 pM or higher).

*ActRIIB variants*

In some embodiments, the ActRII variant for use according to the methods described herein is an extracellular ActRIIB variant constructed by introducing amino acid residues of ActRIIA into ActRIIB, or by introducing novel amino acid substitutions into ActRIIB, with the goal of reducing BMP9 binding to prevent or reduce disruption of endogenous BMP9 signaling. The amino acid substitutions may also impart beneficial physiological and pharmacokinetic properties of ActRIIA, such as longer half-life as an Fc fusion protein. The preferred ActRIIB variants also exhibit similar or improved binding to activins and/or myostatin compared to wild-type ActRIIB, which allows them to compete with endogenous activin receptors for ligand binding and reduce or inhibit endogenous activin receptor signaling. In some embodiments, amino acid substitutions may be introduced to an extracellular ActRIIB variant to reduce or remove the binding affinity of the variant to BMP9. Polypeptides including an ActRIIB variant described herein can be used to increase EPO and EPO receptor levels and, therefore, can be used as a replacement for EPO therapy. These polypeptides can also be administered less frequently than current EPO therapies, which would greatly improve convenience for patients and could potentially reduce adverse effects. Accordingly, polypeptides including ActRIIB variants described herein can be used to treat diseases or conditions that can be treated with EPO or an ESA.

Polypeptides described herein include an extracellular ActRIIB variant having at least one amino acid substitution relative to the wild-type extracellular ActRIIB having the sequence of SEQ ID NO: 74. Possible amino acid substitutions at 28 different positions may be introduced to an extracellular ActRIIB variant (Table 4). An extracellular ActRIIB variant may have one or more (e.g., 1-28, 1-25, 1-23, 1-21, 1-19, 1-17, 1-15, 1-13, 1-11, 1-9, 1-7, 1-5, 1-3, or 1-2; e.g., 1, 2, 3, 4, 5, 6, 7, 8, 9, 10, 11, 12, 13, 14, 15, 16, 17, 18, 19, 20, 21, 22, 23, 24, 25, 26, 27, or 28) amino acid substitutions relative the sequence of a wild-type extracellular ActRIIB (SEQ ID NO: 74). In some embodiments, an extracellular ActRIIB variant (e.g., an extracellular ActRIIB variant having a sequence of SEQ ID NO: 1) may include amino acid substitutions at all of the 28 positions as listed in Table 4. In some embodiments, an extracellular ActRIIB variant may include amino acid substitutions at a number of positions, e.g., at 3, 4, 5, 6, 7, 8, 9, 10, 12, 15, 16, 18, 20, 22, 24, 26, or 27 out of the 28 positions, as listed in Table 4. In some embodiments, the substitutions are substitutions of an amino acid from ActRIIA into the same position in ActRIIB. In some embodiments, the substitutions are novel changes (e.g., substitutions of amino acids that are not in the corresponding position of ActRIIA, e.g., S48T, I51L, Q69D, or E70T).

Amino acid substitutions can worsen or improve the activity and/or binding affinity of the ActRIIB variants of the invention (e.g., an extracellular ActRIIB variant having the sequence of any one of SEQ ID NOs: 157-171 (e.g., SEQ ID NOs: 158-171)). In some embodiments, the amino acid substitutions worsen the binding affinity of the ActRIIB variants to BMP9 (e.g., the variants have reduced binding to BMP9 relative to wild-type extracellular ActRIIB, or have lower binding to BMP9 than to other ActRIIB ligands (e.g., activin A or B, myostatin, or GDF-11)). In some embodiments, the ActRIIB variants have reduced, weak, or no substantial binding to BMP9. In some embodiments, the amino acid substitutions improve the binding affinity of ActRIIB to myostatin, activin A or B, and/or GDF-11 (e.g., the variants have improved binding affinity relative to wild-type extracellular ActRIIB, or bind more strongly to myostatin, activin A or B, or GDF-11 than to BMP9). In some embodiments, the amino acid substitutions reduce the binding affinity of ActRIIB to myostatin, activin A or B, and/or GDF-11 (e.g., the variants have decreased binding affinity relative to wild-type extracellular ActRIIB, or bind more weakly to myostatin, activin A or B, or GDF-11 than to BMP9). In some embodiments, the amino acid substitutions do not substantially change extracellular ActRIIB function (e.g., the ActRIIB variants are functionally equivalent to the wild-type extracellular ActRIIB). In some embodiments, the amino acid substitutions confer an ActRIIA property or activity on the ActRIIB variant (e.g., the ActRIIB variant has a longer half-life as an Fc fusion protein than WT extracellular ActRIIB-Fc). Preferably, the ActRIIB variants have one or more, two or more, or three or more of the above properties (e.g., reduced BMP9 binding and improved binding to activin A or B, myostatin, and/or GDF-11, or reduced BMP9 binding and functional equivalence to wild-type ActRIIB).

The ActRIIB variants of the invention (e.g., an extracellular ActRIIB variant having the sequence of any one of SEQ ID NOs: 157-171 (e.g., SEQ ID NOs: 158-171)) preferably have one or more amino acid substitutions that reduce BMP9 binding. In some embodiments, the amino acid substitution that reduces BMP9 binding is E75K (e.g., $X_{24}$ is K in SEQ ID NO: 157). In some embodiments, the amino acid substitutions that reduce BMP9 binding are Q69T and E70D (e.g., $X_{21}$ is T and $X_{22}$ is D in SEQ ID NO: 157). In some embodiments, the amino acid substitutions that reduce BMP9 binding are Q69D and E70T (e.g., $X_{21}$ is D and $X_{22}$ is T in SEQ ID NO: 157). In some embodiments, the amino acid substitutions that reduce BMP9 binding are T74K, E75K, E76D, N77S, and Q79E (e.g., $X_{23}$, $X_{24}$, $X_{25}$, $X_{26}$, and $X_{28}$ are K, K, D, S, and E, respectively, in SEQ ID NO: 157). In some embodiments, the ActRIIB variants have more than one of the aforementioned amino acid substitutions that reduce BMP9 binding (e.g., substitution E75K and substitutions Q69D and E70T, or substitution E75K and substitutions Q69T and E70D). In some embodiments, the ActRIIB variants of the invention have one or more amino acid substitutions that reduce BMP9 binding, and one or more additional amino acid substitutions. The additional amino acid substitutions may confer other beneficial properties, such as altered binding to activins or myostatin or improved activity. For example, amino acid substitutions T74K, E75K, E76D, N77S, and Q79E lead to a reduction in ActRIIB variant activity (e.g., compared to wild-type extracellular ActRIIB), but including additional substitutions S25T and S47I; E31Y, E33D, and Q34K; or Y41F, R45K, and K56Q improves the effect of the ActRIIB variant. The additional amino acid substitutions may include one or more of substitutions I11L, Y12F, L19K, E20D, S25T, L27V, R29P, E31Y, E33D, Q34K, L38R, Y41F, R45K, S47I, S48T, T50S, I51L, L53I, K56Q and F63I, T74K, E76D, N77S, Q79E, or F89M.

In some embodiments, a polypeptide described herein includes an extracellular ActRIIB variant having the sequence of SEQ ID NO: 157.

Table 4. Amino acid substitutions in an extracellular ActRIIB variant having a sequence of SEQ ID NO: 157

GRGEAETRECX$_1$X$_2$YNANWEX$_3$X$_4$RTNQX$_5$GX$_6$EX$_7$CX$_8$GX$_9$X$_{10}$DKRX$_{11}$HCX$_{12}$ASWX$_{13}$NX$_{14}$X$_{15}$GX$_{16}$X$_{17}$E

X$_{18}$VKX$_{19}$GCWLDDX$_{20}$NCYDRX$_{21}$X$_{22}$CVAX$_{23}$X$_{24}$X$_{25}$X$_{26}$PX$_{27}$VYFCCCEGNX$_{28}$CNERFTHLPEAGGPEVT

YEPPPTAPT

(SEQ ID NO: 157)

| | | | |
|---|---|---|---|
| **X$_1$** | I or L | **X$_{15}$** | S or T |
| **X$_2$** | F or Y | **X$_{16}$** | S or T |
| **X$_3$** | L or K | **X$_{17}$** | I or L |
| **X$_4$** | D or E | **X$_{18}$** | I or L |
| **X$_5$** | T or S | **X$_{19}$** | K or Q |
| **X$_6$** | L or V | **X$_{20}$** | F or I |
| **X$_7$** | P or R | **X$_{21}$** | Q, T, or D |
| **X$_8$** | Y or E | **X$_{22}$** | E, D, or T |
| **X$_9$** | D or E | **X$_{23}$** | K or T |
| **X$_{10}$** | K or Q | **X$_{24}$** | K or E |
| **X$_{11}$** | R or L | **X$_{25}$** | D or E |
| **X$_{12}$** | Y or F | **X$_{26}$** | S or N |
| **X$_{13}$** | R or K | **X$_{27}$** | E or Q |
| **X$_{14}$** | S or I | **X$_{28}$** | F or M |

In some embodiments, a polypeptide described herein includes an extracellular ActRIIB variant having a sequence of any one of SEQ ID NOs: 158-171 (Table 5).

Table 5. Extracellular ActRIIB variants having the sequences of SEQ ID NOs: 158-171

| SEQ ID NO | Amino Acid Sequence |
|---|---|
| 158 | GRGEAETRECIFYNANWEKDRTNQSGLEPCYGDQDKRRHCFASWKNSSGTIELVKQGCWLDDINCYDRQECVAKKDSPEVYFCCCEGNFCNERFTHLPEAGGPEVTYEPPPTAPT |
| 159 | GRGEAETRECIYYNANWELDRTNQSGLERCEGEQDKRLHCYASWRNSSGTIELVKKGCWLDDINCYDRQECVATKENPQVYFCCCEGNFCNERFTHLPEAGGPEVTYEPPPTAPT |
| 160 | GRGEAETRECIYYNANWELERTNQSGLERCEGEQDKRLHCYASWRNSSGTIELVKKGCWLDDFNCYDRQECVAKKDSPEVYFCCCEGNFCNERFTHLPEAGGPEVTYEPPPTAPT |
| 161 | GRGEAETRECIYYNANWELERTNQTGLERCEGEQDKRLHCYASWRNISGTIELVKKGCWLDDFNCYDRQECVAKKDSPEVYFCCCEGNFCNERFTHLPEAGGPEVTYEPPPTAPT |
| 162 | GRGEAETRECIYYNANWELERTNQTGLERCEGEQDKRLHCYASWRNITGTIELVKKGCWLDDFNCYDRQECVAKKDSPEVYFCCCEGNFCNERFTHLPEAGGPEVTYEPPPTAPT |
| 163 | GRGEAETRECIYYNANWELERTNQSGLEPCEGEQDKRLHCYASWRNSSGTIELVKKGCWLDDFNCYDRQECVAKKDSPEVYFCCCEGNFCNERFTHLPEAGGPEVTYEPPPTAPT |
| 164 | GRGEAETRECIYYNANWELERTNQSGLERCYGDKDKRLHCYASWRNSSGTIELVKKGCWLDDFNCYDRQECVAKKDSPEVYFCCCEGNFCNERFTHLPEAGGPEVTYEPPPTAPT |
| 165 | GRGEAETRECIYYNANWELERTNQSGLERCEGEQDKRLHCFASWKNSSGTIELVKQGCWLDDFNCYDRQECVAKKDSPEVYFCCCEGNFCNERFTHLPEAGGPEVTYEPPPTAPT |
| 166 | GRGEAETRECIFYNANWEKDRTNQSGLERCEGEQDKRLHCYASWRNSSGTIELVKKGCWLDDFNCYDRQECVAKKDSPEVYFCCCEGNFCNERFTHLPEAGGPEVTYEPPPTAPT |
| 167 | GRGEAETRECIYYNANWELERTNQSGLERCYGDQDKRRHCYASWRNSSGTIELVKKGCWLDDFNCYDRQECVAKKDSPEVYFCCCEGNFCNERFTHLPEAGGPEVTYEPPPTAPT |
| 168 | GRGEAETRECLYYNANWELERTNQSGVERCEGEKDKRLHCYASWRNSSGSLEIVKKGCWLDDFNCYDRTDCVATEENPQVYFCCCEGNMCNERFTHLPEAGGPEVTYEPPPTAPT |

(continued)

| SEQ ID NO | Amino Acid Sequence |
|-----------|---------------------|
| 169 | GRGEAETRECLYYNANWELERTNQSGVERCEGEKDKRLHCYASWRNSSGSLEIV KKGCWLDDFNCYDRDTCVATEENPQVYFCCCEGNMCNERFTHLPEAGGPEVTYE PPPTAPT |
| 170 | GRGEAETRECLYYNANWELERTNQSGVERCEGEKDKRLHCYASWRNSSGSLEIV KKGCWLDDFNCYDRTDCVATKENPQVYFCCCEGNMCNERFTHLPEAGGPEVTYE PPPTAPT |
| 171 | GRGEAETRECLYYNANWELERTNQSGVERCEGEKDKRLHCYASWRNSSGSLEIV KKGCWLDDFNCYDRDTCVATKENPQVYFCCCEGNMCNERFTHLPEAGGPEVTYE PPPTAPT |

In some embodiments, the extracellular ActRIIB variants described herein have an N-terminal truncation of 1-7 amino acids (e.g., 1, 2, 3, 4, 5, 6, or 7 amino acids). The N-terminal truncation can involve the removal of 1-7 amino acids from the N-terminus of any of the ActRIIB variants shown in Tables 4 and 5. The N-terminal truncation can remove amino acids up two to amino acids before the first cysteine (e.g., the two amino acids before the first cysteine (RE) are retained in the N-terminally truncated ActRIIB variants).

In some embodiments, a polypeptide of the invention including an extracellular ActRIIB variant may further include a moiety (e.g., Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin), which may be fused to the Nor C-terminus (e.g., C-terminus) of the extracellular ActRIIB variant by way of a linker or other covalent bonds. A polypeptide including an extracellular ActRIIB variant fused to an Fc domain monomer may form a dimer (e.g., homodimer or heterodimer) through the interaction between two Fc domain monomers, which combine to form an Fc domain in the dimer.

Furthermore, in some embodiments, a polypeptide described herein (e.g., an ActRIIB variant-Fc fusion protein) has a serum half-life of at least 7 days in humans. The polypeptide including an extracellular ActRIIB variant may bind to activin A with a $K_D$ of 10 pM or higher. In some embodiments, the polypeptide including an extracellular ActRIIB variant does not bind to BMP9 or activin A. In some embodiments, the polypeptide including an extracellular ActRIIB variant binds to activin A, activin B, and/or myostatin and exhibits reduced (e.g., weak) binding to BMP9. In some embodiments, the polypeptide including an extracellular ActRIIB variant does not substantially bind to human BMP9.

In some embodiments, the polypeptide including an extracellular ActRIIB variant may bind to human activin A with a $K_D$ of about 800 pM or less (e.g., a $K_D$ of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a $K_D$ of between about 800 pM and about 200 pM). In some embodiments, the polypeptide including an extracellular ActRIIB variant may bind to human activin B with a $K_D$ of 800 pM or less (e.g., a $K_D$ of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a $K_D$ of between about 800 pM and about 200 pM) The polypeptide including an extracellular ActRIIB variant may also bind to growth and differentiation factor 11 (GDF-11) with a $K_D$ of approximately 5 pM or higher (e.g., a $K_D$ of about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 pM or higher).

*ActRII chimeras*

In some embodiments, the ActRII variant for use according to the methods described herein is an extracellular ActRII chimera constructed by combining portions of extracellular ActRIIA and ActRIIB with the goal of generating proteins that bind to ActRII ligands (e.g., activin A, activin B, myostatin, and GDF11) and retain the function of wild-type extracellular ActRII proteins. In some embodiments, the ActRII chimeras exhibit reduced BMP9 binding relative to wild-type extracellular ActRIIB, which can prevent or reduce disruption of endogenous BMP9 signaling. In some embodiments, the chimeras have properties of both ActRIIA (e.g., low binding affinity to BMP9 and/or longer serum half-life as an Fc fusion protein) and ActRIIB (e.g., strong binding affinity to activins A and B). The ActRII chimeras may exhibit similar or improved binding to activins (e.g., activin A and/or activin B) and/or myostatin compared to wild-type extracellular ActRIIA and/or ActRIIB, allowing them to compete with endogenous activin receptors for ligand binding and reduce or inhibit endogenous activin receptor signaling. Polypeptides including an ActRII chimera described herein can be used to increase EPO and EPO receptor levels and, therefore, can be used as a replacement for EPO therapy. These polypeptides can also be administered less frequently than current EPO therapies, which would greatly improve convenience for patients and could potentially reduce adverse effects. Accordingly, polypeptides including ActRII chimeras described herein can be used to treat diseases or conditions that can be treated with EPO or an ESA.

Polypeptides described herein include an extracellular ActRII chimera containing sequence from both the extracellular portion of ActRIIB and the extracellular portion of ActRIIA. The ActRII chimeras described herein result from joining an N-terminal portion of extracellular ActRIIB (SEQ ID NO: 74 shown above) to a C-terminal portion of extracellular ActRIIA (SEQ ID NO: 73 shown above) such that the sequences are contiguous (e.g., the ActRIIA sequence continues where the ActRIIB sequence left off, starting with the next the amino acid located in the corresponding position of ActRIIA). In some embodiments, the N-terminus of the ActRII chimera includes the six amino acids found at the N-terminus of extracellular ActRIIA joined to the fifth amino acid of extracellular ActRIIB. In some embodiments, the N-terminus of the ActRII chimera begins with the first amino acid located at the N-terminus of extracellular ActRIIB. Accordingly, in some embodiments, the N-terminal portion of ActRIIB begins with the amino acid in the fifth position of SEQ ID NO: 74 (A), while in other embodiments (e.g., in embodiments in which the six amino acids found at the N-terminus of extracellular ActRIIA are not included in the chimera), the N-terminal portion of ActRIIB begins with the amino acid in the first position of SEQ ID NO: 74 (G). In some embodiments, the N-terminus of the ActRII chimera includes the first ten amino acids found at the N-terminus of extracellular ActRIIA joined to the ninth amino acid of extracellular ActRIIB, in which case the N-terminal portion of ActRIIB begins with the amino acid in the ninth position of SEQ ID NO: 74 (E). The extracellular ActRII chimera may also include one or more amino acid substitutions in the portion of the chimera that corresponds to the sequence of ActRIIB compared to wild-type extracellular ActRIIB (e.g., SEQ ID NO: 74 shown above), and one or more amino acid substitutions in the portion of the chimera that corresponds to the sequence of ActRIIA compared to wild-type extracellular ActRIIA (e.g., SEQ ID NO: 73 shown above). Amino acid substitutions at 9 different positions may be introduced into an extracellular ActRII chimera (Table 6). An extracellular ActRII chimera may have one or more (e.g., 1, 2, 3, 4, 5, 6, 7, 8, or 9) amino acid substitutions relative the sequence of a wild-type sequence (e.g., relative to the sequence of wild-type extracellular ActRIIB (SEQ ID NO: 74) if the portion of the chimera corresponds to a region of wild-type extracellular ActRIIB, or relative to the sequence of wild-type extracellular ActRIIA (SEQ ID NO: 73) if the portion of the chimera corresponds to a region of wild-type extracellular ActRIIA). The positions at which amino acid substitutions may be made, as well as the amino acids that may be substituted at these positions, are listed in Table 6.

Amino acid substitutions can alter the activity and/or binding affinity of the extracellular ActRII chimeras of the invention. In some embodiments, the extracellular ActRII chimeras bind to activin A, activin B, myostatin, and/or GDF11 with sufficient affinity to compete with endogenous activin receptors for binding to one or more of these ligands. In some embodiments, the extracellular ActRII chimeras of the invention have reduced, weak, or no substantial binding to BMP9 (e.g., compared to wild-type ActRIIB). BMP9 binding may be reduced in extracellular ActRII chimeras containing the amino acid sequence TEEN (SEQ ID NO: 374) or TKEN (SEQ ID NO: 375) at positions $X_3$, $X_4$, $X_5$, and $X_6$. In some embodiments, a polypeptide of the invention including an extracellular ActRII chimera (e.g., any one of SEQ ID NOs: 174-216 (e.g., SEQ ID NOs: 195-216)) with the sequence TEEN (SEQ ID NO: 374) at positions $X_3$, $X_4$, $X_5$, and $X_6$ can have a substitution of the amino acid K for the amino acid E at position $X_4$. In some embodiments, a polypeptide of the invention including an extracellular ActRII chimera (e.g., any one of SEQ ID NOs: 174-216 (e.g., SEQ ID NOs: 195-216)) with the sequence TKEN (SEQ ID NO: 375) at positions $X_3$, $X_4$, $X_5$, and $X_6$ can have a substitution of the amino acid E for the amino acid K at position $X_4$. The sequences TEEN (SEQ ID NO: 374) and TKEN (SEQ ID NO: 375) can be used interchangeably in the extracellular ActRII chimeras (e.g., the chimeras in Tables 6 and 7, e.g., SEQ ID NOs: 174-216 (e.g., SEQ ID NOs: 195-216)) of the invention.

The extracellular ActRII chimeras of the invention may further include a C-terminal extension (e.g., additional amino acids at the C-terminus). The C-terminal extension can add one or more additional amino acids at the C-terminus (e.g., 1, 2, 3, 4, 5, 6 or more additional amino acids) to any of the chimeras shown in Tables 6 and 7 (e.g., SEQ ID NOs: 174-216 (e.g., SEQ ID NOs: 195-216)). The C-terminal extension may correspond to sequence from the same position in wild-type ActRIIA or ActRIIB. For example, C-terminal extensions that can be included in the extracellular ActRII chimeras of the invention are the amino acid sequence NP and the amino acid sequence NPVTPK (SEQ ID NO: 154), which correspond to sequence found in the same position in wild-type ActRIIA.

Table 6. Amino acid substitutions in an extracellular ActRII chimera having a sequence of any one of SEQ ID NOs: 174-194

| |
|---|
| GAILGRAETRECIYYNANWELERTNQSGLERCEGEQX$_1$KRRHCFATWKNISGSIEIVKQGCWLDDX$_2$X$_3$<br><br>CYDRTDCVX$_4$X$_5$X$_6$X$_7$X$_8$PX$_9$VYFCCCEGNMCNEKFSYFPEMEVTQPTS (SEQ ID NO: 174)<br><br>GAILGRAETRECIYYNANWELERTNQSGLERCEGEQX$_1$KRLHCFATWKNISGSIEIVKQGCWLDDX$_2$X$_3$<br><br>CYDRTDCVX$_4$X$_5$X$_6$X$_7$X$_8$PX$_9$VYFCCCEGNMCNEKFSYFPEMEVTQPTS (SEQ ID NO: 175) |
| GAILGRAETRECIYYNANWELERTNQSGLERCEGEQX$_1$KRLHCYASWKNISGSIEIVKQGCWLDDX$_2$X$_3$<br><br>CYDRTDCVX$_4$X$_5$X$_6$X$_7$X$_8$PX$_9$VYFCCCEGNMCNEKFSYFPEMEVTQPTS (SEQ ID NO: 176)<br><br>GAILGRAETRECIYYNANWELERTNQSGLERCEGEQX$_1$KRLHCYASWRNSSGSIEIVKQGCWLDDX$_2$<br><br>X$_3$CYDRTDCVX$_4$X$_5$X$_6$X$_7$X$_8$PX$_9$VYFCCCEGNMCNEKFSYFPEMEVTQPTS (SEQ ID NO: 177)<br><br>GAILGRAETRECIYYNANWELERTNQSGLERCEGEQX$_1$KRLHCYASWRNSSGTIEIVKQGCWLDDX$_2$X<br><br>$_3$CYDRTDCVX$_4$X$_5$X$_6$X$_7$X$_8$PX$_9$VYFCCCEGNMCNEKFSYFPEMEVTQPTS (SEQ ID NO: 178)<br><br>GAILGRAETRECIYYNANWELERTNQSGLERCEGEQX$_1$KRLHCYASWRNSSGTIELVKKGCWLDDX$_2$<br><br>X$_3$CYDRTDCVX$_4$X$_5$X$_6$X$_7$X$_8$PX$_9$VYFCCCEGNMCNEKFSYFPEMEVTQPTS (SEQ ID NO: 179)<br><br>GAILGRAETRECIYYNANWELERTNQSGLERCEGEQX$_1$KRLHCYASWRNSSGTIELVKKGCWLDDX$_2$<br><br>X$_3$CYDRQECVX$_4$X$_5$X$_6$X$_7$X$_8$PX$_9$VYFCCCEGNMCNEKFSYFPEMEVTQPTS (SEQ ID NO: 180)<br><br>GRGEAETRECIYYNANWELERTNQSGLERCEGEQX$_1$KRRHCFATWKNISGSIEIVKQGCWLDDX$_2$X$_3$C<br><br>YDRTDCVX$_4$X$_5$X$_6$X$_7$X$_8$PX$_9$VYFCCCEGNMCNEKFSYFPEMEVTQPTS (SEQ ID NO: 181)<br><br>GRGEAETRECIYYNANWELERTNQSGLERCEGEQX$_1$KRLHCFATWKNISGSIEIVKQGCWLDDX$_2$X$_3$C<br><br>YDRTDCVX$_4$X$_5$X$_6$X$_7$X$_8$PX$_9$VYFCCCEGNMCNEKFSYFPEMEVTQPTS (SEQ ID NO: 182)<br><br>GRGEAETRECIYYNANWELERTNQSGLERCEGEQX$_1$KRLHCYASWKNISGSIEIVKQGCWLDDX$_2$X$_3$C<br><br>YDRTDCVX$_4$X$_5$X$_6$X$_7$X$_8$PX$_9$VYFCCCEGNMCNEKFSYFPEMEVTQPTS (SEQ ID NO: 183)<br><br>GRGEAETRECIYYNANWELERTNQSGLERCEGEQX$_1$KRLHCYASWRNSSGSIEIVKQGCWLDDX$_2$X$_3$C<br><br>YDRTDCVX$_4$X$_5$X$_6$X$_7$X$_8$PX$_9$VYFCCCEGNMCNEKFSYFPEMEVTQPTS (SEQ ID NO: 184)<br><br>GRGEAETRECIYYNANWELERTNQSGLERCEGEQX$_1$KRLHCYASWRNSSGTIEIVKQGCWLDDX$_2$X$_3$C<br><br>YDRTDCVX$_4$X$_5$X$_6$X$_7$X$_8$PX$_9$VYFCCCEGNMCNEKFSYFPEMEVTQPTS (SEQ ID NO: 185) |

(continued)

GRGEAETRECIYYNANWELERTNQSGLERCEGEQX$_1$KRLHCYASWRNSSGTIELVKKGCWLDDX$_2$X$_3$
CYDRTDCVX$_4$X$_5$X$_6$X$_7$X$_8$PX$_9$VYFCCCEGNMCNEKFSYFPEMEVTQPTS (SEQ ID NO: 186)

GRGEAETRECIYYNANWELERTNQSGLERCEGEQX$_1$KRLHCYASWRNSSGTIELVKKGCWLDDX$_2$X$_3$
CYDRQECVX$_4$X$_5$X$_6$X$_7$X$_8$PX$_9$VYFCCCEGNMCNEKFSYFPEMEVTQPTS (SEQ ID NO: 187)

GAILGRSETQECIYYNANWELERTNQSGLERCEGEQX$_1$KRRHCFATWKNISGSIEIVKQGCWLDDX$_2$X$_3$
CYDRTDCVX$_4$X$_5$X$_6$X$_7$X$_8$PX$_9$VYFCCCEGNMCNEKFSYFPEMEVTQPTS (SEQ ID NO: 188)

GAILGRSETQECIYYNANWELERTNQSGLERCEGEQX$_1$KRLHCFATWKNISGSIEIVKQGCWLDDX$_2$X$_3$
CYDRTDCVX$_4$X$_5$X$_6$X$_7$X$_8$PX$_9$VYFCCCEGNMCNEKFSYFPEMEVTQPTS (SEQ ID NO: 189)

GAILGRSETQECIYYNANWELERTNQSGLERCEGEQX$_1$KRLHCYASWKNISGSIEIVKQGCWLDDX$_2$X$_3$
CYDRTDCVX$_4$X$_5$X$_6$X$_7$X$_8$PX$_9$VYFCCCEGNMCNEKFSYFPEMEVTQPTS (SEQ ID NO: 190)

GAILGRSETQECIYYNANWELERTNQSGLERCEGEQX$_1$KRLHCYASWRNSSGSIEIVKQGCWLDDX$_2$
X$_3$CYDRTDCVX$_4$X$_5$X$_6$X$_7$X$_8$PX$_9$VYFCCCEGNMCNEKFSYFPEMEVTQPTS (SEQ ID NO: 191)

GAILGRSETQECIYYNANWELERTNQSGLERCEGEQX$_1$KRLHCYASWRNSSGTIEIVKQGCWLDDX$_2$
X$_3$CYDRTDCVX$_4$X$_5$X$_6$X$_7$X$_8$PX$_9$VYFCCCEGNMCNEKFSYFPEMEVTQPTS (SEQ ID NO: 192)

GAILGRSETQECIYYNANWELERTNQSGLERCEGEQX$_1$KRLHCYASWRNSSGTIELVKKGCWLDDX$_2$
X$_3$CYDRTDCVX$_4$X$_5$X$_6$X$_7$X$_8$PX$_9$VYFCCCEGNMCNEKFSYFPEMEVTQPTS (SEQ ID NO: 193)

GAILGRSETQECIYYNANWELERTNQSGLERCEGEQX$_1$KRLHCYASWRNSSGTIELVKKGCWLDDX$_2$
X$_3$CYDRQECVX$_4$X$_5$X$_6$X$_7$X$_8$PX$_9$VYFCCCEGNMCNEKFSYFPEMEVTQPTS (SEQ ID NO: 194)

| X$_1$ | | D or R | X$_6$ | E or K |
|---|---|---|---|---|
| X$_2$ | | I, F, E, D, Y, S, N, Q, or T | X$_7$ | E or D |
| X$_3$ | | N or T | X$_8$ | N or S |
| X$_4$ | | A or E | X$_9$ | Q, E, K, R, D, or N |
| X$_5$ | | T or K | | |

29

In some embodiments, in the extracellular ActRII chimeras of SEQ ID NOs: 174-216 (shown in Table 6), $X_1$ is D, $X_2$ is I, F, or E, $X_3$ is N or T, $X_4$ is A or E, $X_5$ is T or K, $X_6$ is E or K, $X_7$ is E or D, $X_8$ is N or S, and $X_9$ is E or Q. In some embodiments, in the extracellular ActRII chimeras of SEQ ID NOs: 174-216, $X_1$ is D, $X_2$ is I or F, $X_3$ is N, $X_4$ is A or E, $X_5$ is T or K, $X_6$ is E or K, $X_7$ is E or D, $X_8$ is N or S, and $X_9$ is E or Q.

In some embodiments, a polypeptide described herein includes an extracellular ActRII chimera having the sequence of any one of SEQ ID NOs: 195-216 (Table 7).

Table 7. Extracellular ActRII chimeras having the sequences of SEQ ID NOs: 195-216

| SEQ ID NO: | Amino Acid Sequence |
| --- | --- |
| 195 | GAILGRAETRECIYYNANWELERTNQSGLERCEGEQDKRLHCYASWRNSSGTIEIV KQGCWLDDINCYDRTDCVATEENPQVYFCCCEGNMCNEKFSYFPEMEVTQPTS |
| 196 | GRGEAETRECIYYNANWELERTNQSGLERCEGEQDKRLHCYASWRNSSGTIEIVK QGCWLDDINCYDRTDCVEKKDSPEVYFCCCEGNMCNEKFSYFPEMEVTQPTS |
| 197 | GRGEAETRECIYYNANWELERTNQSGLERCEGEQDKRLHCYASWRNSSGTIEIVK QGCWLDDINCYDRTDCVEKKDSPQVYFCCCEGNMCNEKFSYFPEMEVTQPTS |
| 198 | GRGEAETRECIYYNANWELERTNQSGLERCEGEQDKRLHCYASWRNSSGTIEIVK QGCWLDDFNCYDRTDCVEKKDSPQVYFCCCEGNMCNEKFSYFPEMEVTQPTS |
| 199 | GAILGRAETRECIYYNANWELERTNQSGLERCEGEQDKRLHCYASWRNSSGTIEIV KQGCWLDDFNCYDRTDCVATEENPQVYFCCCEGNMCNEKFSYFPEMEVTQPTS |
| 200 | GRGEAETRECIYYNANWELERTNQSGLERCEGEQDKRLHCYASWRNSSGTIEIVK QGCWLDDFNCYDRTDCVEKKDSPEVYFCCCEGNMCNEKFSYFPEMEVTQPTS |
| 201 | GRGEAETRECIYYNANWELERTNQSGLERCEGEQDKRRHCFATWKNISGSIEIVKQ GCWLDDFNCYDRTDCVEKKDSPQVYFCCCEGNMCNEKFSYFPEMEVTQPTS |
| 202 | GRGEAETRECIYYNANWELERTNQSGLERCEGEQDKRLHCFATWKNISGSIEIVKQ GCWLDDFNCYDRTDCVEKKDSPQVYFCCCEGNMCNEKFSYFPEMEVTQPTS |
| 203 | GRGEAETRECIYYNANWELERTNQSGLERCEGEQDKRLHCYASWKNISGSIEIVKQ GCWLDDFNCYDRTDCVEKKDSPQVYFCCCEGNMCNEKFSYFPEMEVTQPTS |
| 204 | GRGEAETRECIYYNANWELERTNQSGLERCEGEQDKRLHCYASWRNSSGSIEIVK QGCWLDDFNCYDRTDCVEKKDSPQVYFCCCEGNMCNEKFSYFPEMEVTQPTS |
| 205 | GRGEAETRECIYYNANWELERTNQSGLERCEGEQDKRLHCYASWRNSSGTIELVK KGCWLDDFNCYDRTDCVEKKDSPQVYFCCCEGNMCNEKFSYFPEMEVTQPTS |
| 206 | GRGEAETRECIYYNANWELERTNQSGLERCEGEQDKRLHCYASWRNSSGTIELVK KGCWLDDFNCYDRQECVATKDSPQVYFCCCEGNMCNEKFSYFPEMEVTQPTS |
| 207 | GRGEAETRECIYYNANWELERTNQSGLERCEGEQDKRLHCYASWRNSSGTIELVK KGCWLDDFNCYDRQECVATEENPQVYFCCCEGNMCNEKFSYFPEMEVTQPTS |
| 208 | GRGEAETRECIYYNANWELERTNQSGLERCEGEQDKRLHCYASWRNSSGTIEIVK QGCWLDDNNCYDRTDCVEKKDSPQVYFCCCEGNMCNEKFSYFPEMEVTQPTS |

(continued)

| SEQ ID NO: | Amino Acid Sequence |
|---|---|
| 209 | GRGEAETRECIYYNANWELERTNQSGLERCEGEQDKRLHCYASWRNSSGTIEIVK QGCWLDDTNCYDRTDCVEKKDSPQVYFCCCEGNMCNEKFSYFPEMEVTQPTS |
| 210 | GRGEAETRECIYYNANWELERTNQSGLERCEGEQDKRLHCYASWRNSSGTIEIVK QGCWLDDETCYDRTDCVEKKDSPQVYFCCCEGNMCNEKFSYFPEMEVTQPTS |
| 211 | GRGEAETRECIYYNANWELERTNQSGLERCEGEQRKRLHCYASWRNSSGTIEIVK QGCWLDDFNCYDRTDCVEKKDSPQVYFCCCEGNMCNEKFSYFPEMEVTQPTS |
| 212 | GRGEAETRECIYYNANWELERTNQSGLERCEGEQDKRLHCYASWRNSSGTIEIVK QGCWLDDFNCYDRTDCVETKDSPQVYFCCCEGNMCNEKFSYFPEMEVTQPTS |
| 213 | GAILGRAETRECIYYNANWELERTNQSGLERCEGEQDKRLHCYASWRNSSGTIEIV KQGCWLDDFNCYDRTDCVEKKDSPQVYFCCCEGNMCNEKFSYFPEMEVTQPTS |
| 214 | GAILGRSETQECIYYNANWELERTNQSGLERCEGEQDKRLHCYASWRNSSGTIEIV KQGCWLDDFNCYDRTDCVEKKDSPQVYFCCCEGNMCNEKFSYFPEMEVTQPTS |
| 215 | GRGEAETRECIYYNANWELERTNQSGLERCEGEQDKRLHCYASWRNSSGTIELVK KGCWLDDFNCYDRQECVATKDSPEVYFCCCEGNMCNEKFSYFPEMEVTQPTS |
| 216 | GRGEAETRECIYYNANWELERTNQSGLERCEGEQDKRLHCYASWRNSSGTIELVK KGCWLDDFNCYDRQECVATKENPQVYFCCCEGNMCNEKFSYFPEMEVTQPTS |

In some embodiments, the extracellular ActRII chimeras described herein have an N-terminal truncation of 1-9 amino acids (e.g., 1, 2, 3, 4, 5, 6, 7, 8, or 9 amino acids). The N-terminal truncation can involve the removal of 1-9 amino acids from the N-terminus of any of the chimeras shown in Tables 6 and 7 (e.g., SEQ ID NOs: 174-216 (e.g., SEQ ID NOs: 195-216)). The N-terminal truncation can remove amino acids up two to amino acids before the first cysteine (e.g., the two amino acids before the first cysteine (RE or QE) are retained in the N-terminally truncated ActRII chimeras). Exemplary ActRII chimeras having N-terminal truncations are provided in Table 8, below.

Table 8. Extracellular ActRII chimeras having N-terminal truncations

| SEQ ID NO: | Amino Acid Sequence |
|---|---|
| 221 | ILGRAETRECIYYNANWELERTNQSGLERCEGEQDKRLHCYASWRNSSGTIEIVKQ GCWLDDINCYDRTDCVATEENPQVYFCCCEGNMCNEKFSYFPEMEVTQPTS |
| 222 | RGEAETRECIYYNANWELERTNQSGLERCEGEQDKRLHCYASWRNSSGTIEIVKQ GCWLDDINCYDRTDCVEKKDSPEVYFCCCEGNMCNEKFSYFPEMEVTQPTS |
| 223 | RGEAETRECIYYNANWELERTNQSGLERCEGEQDKRLHCYASWRNSSGTIEIVKQ GCWLDDINCYDRTDCVEKKDSPQVYFCCCEGNMCNEKFSYFPEMEVTQPTS |
| 224 | RGEAETRECIYYNANWELERTNQSGLERCEGEQDKRLHCYASWRNSSGTIEIVKQ GCWLDDFNCYDRTDCVEKKDSPQVYFCCCEGNMCNEKFSYFPEMEVTQPTS |
| 225 | LGRAETRECIYYNANWELERTNQSGLERCEGEQDKRLHCYASWRNSSGTIEIVKQ GCWLDDINCYDRTDCVATEENPQVYFCCCEGNMCNEKFSYFPEMEVTQPTS |

(continued)

| SEQ ID NO: | Amino Acid Sequence |
|---|---|
| 226 | GEAETRECIYYNANWELERTNQSGLERCEGEQDKRLHCYASWRNSSGTIEIVKQG CWLDDINCYDRTDCVEKKDSPEVYFCCCEGNMCNEKFSYFPEMEVTQPTS |
| 227 | GEAETRECIYYNANWELERTNQSGLERCEGEQDKRLHCYASWRNSSGTIEIVKQG CWLDDINCYDRTDCVEKKDSPQVYFCCCEGNMCNEKFSYFPEMEVTQPTS |
| 228 | GEAETRECIYYNANWELERTNQSGLERCEGEQDKRLHCYASWRNSSGTIEIVKQG CWLDDFNCYDRTDCVEKKDSPQVYFCCCEGNMCNEKFSYFPEMEVTQPTS |
| 229 | GRAETRECIYYNANWELERTNQSGLERCEGEQDKRLHCYASWRNSSGTIEIVKQG CWLDDINCYDRTDCVATEENPQVYFCCCEGNMCNEKFSYFPEMEVTQPTS |
| 230 | EAETRECIYYNANWELERTNQSGLERCEGEQDKRLHCYASWRNSSGTIEIVKQGC WLDDINCYDRTDCVEKKDSPEVYFCCCEGNMCNEKFSYFPEMEVTQPTS |
| 231 | EAETRECIYYNANWELERTNQSGLERCEGEQDKRLHCYASWRNSSGTIEIVKQGC WLDDINCYDRTDCVEKKDSPQVYFCCCEGNMCNEKFSYFPEMEVTQPTS |
| 232 | EAETRECIYYNANWELERTNQSGLERCEGEQDKRLHCYASWRNSSGTIEIVKQGC WLDDFNCYDRTDCVEKKDSPQVYFCCCEGNMCNEKFSYFPEMEVTQPTS |
| 233 | RAETRECIYYNANWELERTNQSGLERCEGEQDKRLHCYASWRNSSGTIEIVKQGC WLDDINCYDRTDCVATEENPQVYFCCCEGNMCNEKFSYFPEMEVTQPTS |
| 234 | AETRECIYYNANWELERTNQSGLERCEGEQDKRLHCYASWRNSSGTIEIVKQGCW LDDINCYDRTDCVEKKDSPEVYFCCCEGNMCNEKFSYFPEMEVTQPTS |
| 235 | AETRECIYYNANWELERTNQSGLERCEGEQDKRLHCYASWRNSSGTIEIVKQGCW LDDINCYDRTDCVEKKDSPQVYFCCCEGNMCNEKFSYFPEMEVTQPTS |
| 236 | AETRECIYYNANWELERTNQSGLERCEGEQDKRLHCYASWRNSSGTIEIVKQGCW LDDFNCYDRTDCVEKKDSPQVYFCCCEGNMCNEKFSYFPEMEVTQPTS |
| 237 | AETRECIYYNANWELERTNQSGLERCEGEQDKRLHCYASWRNSSGTIEIVKQGCW LDDINCYDRTDCVATEENPQVYFCCCEGNMCNEKFSYFPEMEVTQPTS |
| 238 | ETRECIYYNANWELERTNQSGLERCEGEQDKRLHCYASWRNSSGTIEIVKQGCWL DDINCYDRTDCVEKKDSPEVYFCCCEGNMCNEKFSYFPEMEVTQPTS |
| 239 | ETRECIYYNANWELERTNQSGLERCEGEQDKRLHCYASWRNSSGTIEIVKQGCWL DDINCYDRTDCVEKKDSPQVYFCCCEGNMCNEKFSYFPEMEVTQPTS |
| 240 | ETRECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTS |
| 241 | ETRECIYYNANWELERTNQSGLERCEGEQDKRLHCYASWRNSSGTIEIVKQGCWL DDINCYDRTDCVATEENPQVYFCCCEGNMCNEKFSYFPEMEVTQPTS |

(continued)

| SEQ ID NO: | Amino Acid Sequence |
|---|---|
| 242 | TRECIYYNANWELERTNQSGLERCEGEQDKRLHCYASWRNSSGTIEIVKQGCWLDDINCYDRTDCVEKKDSPEVYFCCCEGNMCNEKFSYFPEMEVTQPTS |
| 243 | TRECIYYNANWELERTNQSGLERCEGEQDKRLHCYASWRNSSGTIEIVKQGCWLDDINCYDRTDCVEKKDSPQVYFCCCEGNMCNEKFSYFPEMEVTQPTS |
| 244 | TRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 245 | TRECIYYNANWELERTNQSGLERCEGEQDKRLHCYASWRNSSGTIEIVKQGCWLDDINCYDRTDCVATEENPQVYFCCCEGNMCNEKFSYFPEMEVTQPTS |
| 246 | RECIYYNANWELERTNQSGLERCEGEQDKRLHCYASWRNSSGTIEIVKQGCWLDDINCYDRTDCVEKKDSPEVYFCCCEGNMCNEKFSYFPEMEVTQPTS |
| 247 | RECIYYNANWELERTNQSGLERCEGEQDKRLHCYASWRNSSGTIEIVKQGCWLDDINCYDRTDCVEKKDSPQVYFCCCEGNMCNEKFSYFPEMEVTQPTS |
| 248 | RECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 249 | RECIYYNANWELERTNQSGLERCEGEQDKRLHCYASWRNSSGTIEIVKQGCWLDDINCYDRTDCVATEENPQVYFCCCEGNMCNEKFSYFPEMEVTQPTS |
| 250 | RGEAETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTS |
| 251 | RGEAETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTS |
| 252 | ILGRAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 253 | ILGRSETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 254 | RGEAETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTS |
| 255 | RGEAETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTS |
| 256 | GEAETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTS |
| 257 | GEAETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTS |

(continued)

| SEQ ID NO: | Amino Acid Sequence |
|---|---|
| 258 | LGRAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 259 | LGRSETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 260 | GEAETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTS |
| 261 | GEAETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTS |
| 262 | EAETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTS |
| 263 | EAETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTS |
| 264 | GRAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 265 | GRSETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 266 | EAETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTS |
| 267 | EAETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTS |
| 268 | AETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTS |
| 269 | AETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTS |
| 270 | RAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 271 | RSETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS |
| 272 | AETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTS |
| 273 | AETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTS |

(continued)

| SEQ ID NO: | Amino Acid Sequence |
|---|---|
| 274 | ETRECIYYNANWELERTNQSGLERCEGEQDKRRHCFATWKNISGSIEIVKQGCWL DDFNCYDRTDCVEKKDSPQVYFCCCEGNMCNEKFSYFPEMEVTQPTS |
| 275 | ETRECIYYNANWELERTNQSGLERCEGEQDKRLHCYASWRNSSGTIEIVKQGCWL DDETCYDRTDCVEKKDSPQVYFCCCEGNMCNEKFSYFPEMEVTQPTS |
| 276 | AETRECIYYNANWELERTNQSGLERCEGEQDKRLHCYASWRNSSGTIEIVKQGCW LDDFNCYDRTDCVEKKDSPQVYFCCCEGNMCNEKFSYFPEMEVTQPTS |
| 277 | SETQECIYYNANWELERTNQSGLERCEGEQDKRLHCYASWRNSSGTIEIVKQGCW LDDFNCYDRTDCVEKKDSPQVYFCCCEGNMCNEKFSYFPEMEVTQPTS |
| 278 | ETRECIYYNANWELERTNQSGLERCEGEQDKRLHCYASWRNSSGTIELVKKGCWL DDFNCYDRQECVATKDSPEVYFCCCEGNMCNEKFSYFPEMEVTQPTS |
| 279 | ETRECIYYNANWELERTNQSGLERCEGEQDKRLHCYASWRNSSGTIELVKKGCWL DDFNCYDRQECVATKENPQVYFCCCEGNMCNEKFSYFPEMEVTQPTS |
| 280 | TRECIYYNANWELERTNQSGLERCEGEQDKRRHCFATWKNISGSIEIVKQGCWLD DFNCYDRTDCVEKKDSPQVYFCCCEGNMCNEKFSYFPEMEVTQPTS |
| 281 | TRECIYYNANWELERTNQSGLERCEGEQDKRLHCYASWRNSSGTIEIVKQGCWLD DETCYDRTDCVEKKDSPQVYFCCCEGNMCNEKFSYFPEMEVTQPTS |
| 282 | ETRECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTS |
| 283 | ETQECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTS |
| 284 | TRECIYYNANWELERTNQSGLERCEGEQDKRLHCYASWRNSSGTIELVKKGCWLD DFNCYDRQECVATKDSPEVYFCCCEGNMCNEKFSYFPEMEVTQPTS |
| 285 | TRECIYYNANWELERTNQSGLERCEGEQDKRLHCYASWRNSSGTIELVKKGCWLD DFNCYDRQECVATKENPQVYFCCCEGNMCNEKFSYFPEMEVTQPTS |
| 286 | RECIYYNANWELERTNQSGLERCEGEQDKRRHCFATWKNISGSIEIVKQGCWLDD FNCYDRTDCVEKKDSPQVYFCCCEGNMCNEKFSYFPEMEVTQPTS |
| 287 | RECIYYNANWELERTNQSGLERCEGEQDKRLHCYASWRNSSGTIEIVKQGCWLDD ETCYDRTDCVEKKDSPQVYFCCCEGNMCNEKFSYFPEMEVTQPTS |
| 288 | TRECIYYNANWELERTNQSGLERCEGEQDKRLHCYASWRNSSGTIEIVKQGCWLD DFNCYDRTDCVEKKDSPQVYFCCCEGNMCNEKFSYFPEMEVTQPTS |
| 289 | TQECIYYNANWELERTNQSGLERCEGEQDKRLHCYASWRNSSGTIEIVKQGCWLD DFNCYDRTDCVEKKDSPQVYFCCCEGNMCNEKFSYFPEMEVTQPTS |

(continued)

| SEQ ID NO: | Amino Acid Sequence |
|---|---|
| 290 | RECIYYNANWELERTNQSGLERCEGEQDKRLHCYASWRNSSGTIELVKKGCWLD DFNCYDRQECVATKDSPEVYFCCCEGNMCNEKFSYFPEMEVTQPTS |
| 291 | RECIYYNANWELERTNQSGLERCEGEQDKRLHCYASWRNSSGTIELVKKGCWLD DFNCYDRQECVATKENPQVYFCCCEGNMCNEKFSYFPEMEVTQPTS |
| 292 | RECIYYNANWELERTNQSGLERCEGEQDKRLHCYASWRNSSGTIEIVKQGCWLDD FNCYDRTDCVEKKDSPQVYFCCCEGNMCNEKFSYFPEMEVTQPTS |
| 293 | QECIYYNANWELERTNQSGLERCEGEQDKRLHCYASWRNSSGTIEIVKQGCWLDD FNCYDRTDCVEKKDSPQVYFCCCEGNMCNEKFSYFPEMEVTQPTS |

In some embodiments, a polypeptide of the invention including an extracellular ActRII chimera may further include a moiety (e.g., Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin), which may be fused to the N or C-terminus (e.g., C-terminus) of the extracellular ActRII chimera by way of a linker or other covalent bonds. A polypeptide including an extracellular ActRII chimera fused to an Fc domain monomer may form a dimer (e.g., homodimer or heterodimer) through the interaction between two Fc domain monomers, which combine to form an Fc domain in the dimer. Exemplary polypeptides containing an ActRII chimera, an Fc domain, and a linker are provided in Table 9, below. In some embodiments, the terminal lysine is absent from the Fc domain amino acid sequence.

Table 9. Polypeptides containing an extracellular ActRII chimera fused to an Fc domain by way of a linker

| SEQ ID NO: | Amino Acid Sequence |
|---|---|
| 217 | GAILGRAETRECIYYNANWELERTNQSGLERCEGEQDKRLHCYASWRNSSGTIEIVKQGC WLDDINCYDRTDCVATEENPQVYFCCCEGNMCNEKFSYFPEMEVTQPTSGGGDKTHTC PPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK |
| 218 | GRGEAETRECIYYNANWELERTNQSGLERCEGEQDKRLHCYASWRNSSGTIEIVKQGCW LDDINCYDRTDCVEKKDSPEVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 219 | GRGEAETRECIYYNANWELERTNQSGLERCEGEQDKRLHCYASWRNSSGTIEIVKQGCW LDDINCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |

(continued)

| SEQ ID NO: | Amino Acid Sequence |
|---|---|
| 220 | GRGEAETRECIYYNANWELERTNQSGLERCEGEQDKRLHCYASWRNSSGTIEIVKQGCW LDDFNCYDRTDCVEKKDSPQVYFCCCEGNMCNEKFSYFPEMEVTQPTSGGGDKTHTCP PCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK |
| 294 | ILGRAETRECIYYNANWELERTNQSGLERCEGEQDKRLHCYASWRNSSGTIEIVKQGCWL DDINCYDRTDCVATEENPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 295 | RGEAETRECIYYNANWELERTNQSGLERCEGEQDKRLHCYASWRNSSGTIEIVKQGCWL DDINCYDRTDCVEKKDSPEVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 296 | RGEAETRECIYYNANWELERTNQSGLERCEGEQDKRLHCYASWRNSSGTIEIVKQGCWL DDINCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 297 | RGEAETRECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 298 | LGRAETRECIYYNANWELERTNQSGLERCEGEQDKRLHCYASWRNSSGTIEIVKQGCWL DDINCYDRTDCVATEENPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |

(continued)

| SEQ ID NO: | Amino Acid Sequence |
|---|---|
| 299 | GEAETRECIYYNANWELERTNQSGLERCEGEQDKRLHCYASWRNSSGTIEIVKQGCWLD DINCYDRTDCVEKKDSPEVYFCCCEGNMCNEKFSYFPEMEVTQPTSGGGDKTHTCPPCP APELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK |
| 300 | GEAETRECIYYNANWELERTNQSGLERCEGEQDKRLHCYASWRNSSGTIEIVKQGCWLD DINCYDRTDCVEKKDSPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 301 | GEAETRECIYYNANWELERTNQSGLERCEGEQDKRLHCYASWRNSSGTIEIVKQGCWLD DFNCYDRTDCVEKKDSPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 302 | GRAETRECIYYNANWELERTNQSGLERCEGEQDKRLHCYASWRNSSGTIEIVKQGCWLD DINCYDRTDCVATEENPQVYFCCCEGNMCNEKFSYFPEMEVTQPTSGGGDKTHTCPPCP APELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK |
| 303 | EAETRECIYYNANWELERTNQSGLERCEGEQDKRLHCYASWRNSSGTIEIVKQGCWLDDI NCYDRTDCVEKKDSPEVYFCCCEGNMCNEKFSYFPEMEVTQPTSGGGDKTHTCPPCPA PELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK |
| 304 | EAETRECIYYNANWELERTNQSGLERCEGEQDKRLHCYASWRNSSGTIEIVKQGCWLDDI NCYDRTDCVEKKDSPQVYFCCCEGNMCNEKFSYFPEMEVTQPTSGGGDKTHTCPPCPA PELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK |

(continued)

| SEQ ID NO: | Amino Acid Sequence |
|---|---|
| 305 | EAETRECIYYNANWELERTNQSGLERCEGEQDKRLHCYASWRNSSGTIEIVKQGCWLDD FNCYDRTDCVEKKDSPQVYFCCCEGNMCNEKFSYFPEMEVTQPTSGGGDKTHTCPPCP APELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK |
| 306 | RAETRECIYYNANWELERTNQSGLERCEGEQDKRLHCYASWRNSSGTIEIVKQGCWLDDI NCYDRTDCVATEENPQVYFCCCEGNMCNEKFSYFPEMEVTQPTSGGGDKTHTCPPCPA PELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK |
| 307 | AETRECIYYNANWELERTNQSGLERCEGEQDKRLHCYASWRNSSGTIEIVKQGCWLDDIN CYDRTDCVEKKDSPEVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |
| 308 | AETRECIYYNANWELERTNQSGLERCEGEQDKRLHCYASWRNSSGTIEIVKQGCWLDDIN CYDRTDCVEKKDSPQVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |
| 309 | AETRECIYYNANWELERTNQSGLERCEGEQDKRLHCYASWRNSSGTIEIVKQGCWLDDF NCYDRTDCVEKKDSPQVYFCCCEGNMCNEKFSYFPEMEVTQPTSGGGDKTHTCPPCPA PELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK |
| 310 | AETRECIYYNANWELERTNQSGLERCEGEQDKRLHCYASWRNSSGTIEIVKQGCWLDDIN CYDRTDCVATEENPQVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |

(continued)

| SEQ ID NO: | Amino Acid Sequence |
|---|---|
| 311 | ETRECIYYNANWELERTNQSGLERCEGEQDKRLHCYASWRNSSGTIEIVKQGCWLDDIN CYDRTDCVEKKDSPEVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |
| 312 | ETRECIYYNANWELERTNQSGLERCEGEQDKRLHCYASWRNSSGTIEIVKQGCWLDDIN CYDRTDCVEKKDSPQVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |
| 313 | ETRECIYYNANWELERTNQSGLERCEGEQDKRLHCYASWRNSSGTIEIVKQGCWLDDFN CYDRTDCVEKKDSPQVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |
| 314 | ETRECIYYNANWELERTNQSGLERCEGEQDKRLHCYASWRNSSGTIEIVKQGCWLDDIN CYDRTDCVATEENPQVYFCCCEGNMCNEKFSYFPEMEVTQPTSGGGDKTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK |
| 315 | TRECIYYNANWELERTNQSGLERCEGEQDKRLHCYASWRNSSGTIEIVKQGCWLDDINC YDRTDCVEKKDSPEVYFCCCEGNMCNEKFSYFPEMEVTQPTSGGGDKTHTCPPCPAPE LLGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVD KSRWQQGNVFSCSVMHEALHNHYTQKSLSLSPGK |
| 316 | TRECIYYNANWELERTNQSGLERCEGEQDKRLHCYASWRNSSGTIEIVKQGCWLDDINC YDRTDCVEKKDSPQVYFCCCEGNMCNEKFSYFPEMEVTQPTSGGGDKTHTCPPCPAPE LLGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVD KSRWQQGNVFSCSVMHEALHNHYTQKSLSLSPGK |

(continued)

| SEQ ID NO: | Amino Acid Sequence |
|---|---|
| 317 | TRECIYYNANWELERTNQSGLERCEGEQDKRLHCYASWRNSSGTIEIVKQGCWLDDFNC YDRTDCVEKKDSPQVYFCCCEGNMCNEKFSYFPEMEVTQPTSGGGDKTHTCPPCPAPE LLGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVD KSRWQQGNVFSCSVMHEALHNHYTQKSLSLSPGK |
| 318 | TRECIYYNANWELERTNQSGLERCEGEQDKRLHCYASWRNSSGTIEIVKQGCWLDDINC YDRTDCVATEENPQVYFCCCEGNMCNEKFSYFPEMEVTQPTSGGGDKTHTCPPCPAPE LLGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVD KSRWQQGNVFSCSVMHEALHNHYTQKSLSLSPGK |
| 319 | RECIYYNANWELERTNQSGLERCEGEQDKRLHCYASWRNSSGTIEIVKQGCWLDDINCY DRTDCVEKKDSPEVYFCCCEGNMCNEKFSYFPEMEVTQPTSGGGDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 320 | RECIYYNANWELERTNQSGLERCEGEQDKRLHCYASWRNSSGTIEIVKQGCWLDDINCY DRTDCVEKKDSPQVYFCCCEGNMCNEKFSYFPEMEVTQPTSGGGDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 321 | RECIYYNANWELERTNQSGLERCEGEQDKRLHCYASWRNSSGTIEIVKQGCWLDDFNCY DRTDCVEKKDSPQVYFCCCEGNMCNEKFSYFPEMEVTQPTSGGGDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 322 | RECIYYNANWELERTNQSGLERCEGEQDKRLHCYASWRNSSGTIEIVKQGCWLDDINCY DRTDCVATEENPQVYFCCCEGNMCNEKFSYFPEMEVTQPTSGGGDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |

| SEQ ID NO: | Amino Acid Sequence |
|---|---|
| 323 | GRGEAETRECIYYNANWELERTNQSGLERCEGEQDKRRHCFATWKNISGSIEIVKQGCW LDDFNCYDRTDCVEKKDSPQVYFCCCEGNMCNEKFSYFPEMEVTQPTSGGGDKTHTCP PCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK |
| 324 | GRGEAETRECIYYNANWELERTNQSGLERCEGEQDKRLHCYASWRNSSGTIEIVKQGCW LDDETCYDRTDCVEKKDSPQVYFCCCEGNMCNEKFSYFPEMEVTQPTSGGGDKTHTCP PCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK |
| 325 | GAILGRAETRECIYYNANWELERTNQSGLERCEGEQDKRLHCYASWRNSSGTIEIVKQGC WLDDFNCYDRTDCVEKKDSPQVYFCCCEGNMCNEKFSYFPEMEVTQPTSGGGDKTHTC PPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK |
| 326 | GAILGRSETQECIYYNANWELERTNQSGLERCEGEQDKRLHCYASWRNSSGTIEIVKQGC WLDDFNCYDRTDCVEKKDSPQVYFCCCEGNMCNEKFSYFPEMEVTQPTSGGGDKTHTC PPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK |
| 327 | GRGEAETRECIYYNANWELERTNQSGLERCEGEQDKRLHCYASWRNSSGTIELVKKGC WLDDFNCYDRQECVATKDSPEVYFCCCEGNMCNEKFSYFPEMEVTQPTSGGGDKTHTC PPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK |
| 328 | GRGEAETRECIYYNANWELERTNQSGLERCEGEQDKRLHCYASWRNSSGTIELVKKGC WLDDFNCYDRQECVATKENPQVYFCCCEGNMCNEKFSYFPEMEVTQPTSGGGDKTHTC PPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK |

(continued)

| SEQ ID NO: | Amino Acid Sequence |
|---|---|
| 329 | RGEAETRECIYYNANWELERTNQSGLERCEGEQDKRRHCFATWKNISGSIEIVKQGCWL DDFNCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 330 | RGEAETRECIYYNANWELERTNQSGLERCEGEQDKRLHCYASWRNSSGTIEIVKQGCWL DDETCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 331 | ILGRAETRECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 332 | ILGRSETQECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 333 | RGEAETRECIYYNANWELERTNQSGLERCEGEQDKRLHCYASWRNSSGTIELVKKGCWL DDFNCYDRQECVATKDSPEVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 334 | RGEAETRECIYYNANWELERTNQSGLERCEGEQDKRLHCYASWRNSSGTIELVKKGCWL DDFNCYDRQECVATKENPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |

| SEQ ID NO: | Amino Acid Sequence |
|---|---|
| 335 | GEAETRECIYYNANWELERTNQSGLERCEGEQDKRRHCFATWKNISGSIEIVKQGCWLD DFNCYDRTDCVEKKDSPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 336 | GEAETRECIYYNANWELERTNQSGLERCEGEQDKRLHCYASWRNSSGTIEIVKQGCWLD DETCYDRTDCVEKKDSPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 337 | LGRAETRECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 338 | LGRSETQECIYYNANWELERTNQSGLERCEGEQDKRLHCYASWRNSSGTIEIVKQGCWL DDFNCYDRTDCVEKKDSPQVYFCCCEGNMCNEKFSYFPEMEVTQPTSGGGDKTHTCPP CPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK |
| 339 | GEAETRECIYYNANWELERTNQSGLERCEGEQDKRLHCYASWRNSSGTIELVKKGCWLD DFNCYDRQECVATKDSPEVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 340 | GEAETRECIYYNANWELERTNQSGLERCEGEQDKRLHCYASWRNSSGTIELVKKGCWLD DFNCYDRQECVATKENPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |

(continued)

| SEQ ID NO: | Amino Acid Sequence |
|---|---|
| 341 | EAETRECIYYNANWELERTNQSGLERCEGEQDKRRHCFATWKNISGSIEIVKQGCWLDDF NCYDRTDCVEKKDSPQVYFCCCEGNMCNEKFSYFPEMEVTQPTSGGGDKTHTCPPCPA PELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK |
| 342 | EAETRECIYYNANWELERTNQSGLERCEGEQDKRLHCYASWRNSSGTIEIVKQGCWLDD ETCYDRTDCVEKKDSPQVYFCCCEGNMCNEKFSYFPEMEVTQPTSGGGDKTHTCPPCP APELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK |
| 343 | GRAETRECIYYNANWELERTNQSGLERCEGEQDKRLHCYASWRNSSGTIEIVKQGCWLD DFNCYDRTDCVEKKDSPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 344 | GRSETQECIYYNANWELERTNQSGLERCEGEQDKRLHCYASWRNSSGTIEIVKQGCWLD DFNCYDRTDCVEKKDSPQVYFCCCEGNMCNEKFSYFPEMEVTQPTSGGGDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 345 | EAETRECIYYNANWELERTNQSGLERCEGEQDKRLHCYASWRNSSGTIELVKKGCWLDD FNCYDRQECVATKDSPEVYFCCCEGNMCNEKFSYFPEMEVTQPTSGGGDKTHTCPPCP APELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK |
| 346 | EAETRECIYYNANWELERTNQSGLERCEGEQDKRLHCYASWRNSSGTIELVKKGCWLDD FNCYDRQECVATKENPQVYFCCCEGNMCNEKFSYFPEMEVTQPTSGGGDKTHTCPPCP APELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK |

(continued)

| SEQ ID NO: | Amino Acid Sequence |
|---|---|
| 347 | AETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 348 | AETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 349 | RAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 350 | RSETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 351 | AETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 352 | AETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Amino Acid Sequence |
|---|---|
| 353 | ETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 354 | ETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 355 | AETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 356 | SETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 357 | ETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 358 | ETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Amino Acid Sequence |
|---|---|
| 359 | TRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 360 | TRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 361 | QECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 362 | ETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 363 | ETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 364 | TRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Amino Acid Sequence |
|---|---|
| 365 | TRECIYYNANWELERTNQSGLERCEGEQDKRLHCYASWRNSSGTIELVKKGCWLDDFNCYDRQECVATKENPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 366 | RECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 367 | RECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 368 | TRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 369 | TQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 370 | RECIYYNANWELERTNQSGLERCEGEQDKRLHCYASWRNSSGTIELVKKGCWLDDFNCYDRQECVATKDSPEVYFCCCEGNMCNEKFSYFPEMEVTQPTSGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Amino Acid Sequence |
|---|---|
| 371 | RECIYYNANWELERTNQSGLERCEGEQDKRLHCYASWRNSSGTIELVKKGCWLDDFNCY DRQECVATKENPQVYFCCCEGNMCNEKFSYFPEMEVTQPTSGGGDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 372 | RECIYYNANWELERTNQSGLERCEGEQDKRLHCYASWRNSSGTIEIVKQGCWLDDFNCY DRTDCVEKKDSPQVYFCCCEGNMCNEKFSYFPEMEVTQPTSGGGDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 373 | GRGEAETRECIYYNANWELERTNQSGLERCEGEQRKRLHCYASWRNSSGTIEIVKQGCW LDDFNCYDRTDCVEKKDSPQVYFCCCEGNMCNEKFSYFPEMEVTQPTSGGGDKTHTCP PCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK |

Furthermore, in some embodiments, a polypeptide described herein (e.g., an ActRII chimera-Fc fusion protein) has a serum half-life of at least 7 days in humans. The polypeptide including an extracellular ActRII chimera may bind to activin A with a $K_D$ of 10 pM or higher. In some embodiments, the polypeptide including an extracellular ActRII chimera binds to activin A, activin B, and/or myostatin and exhibits reduced (e.g., weak) binding to BMP9 (e.g., compared to wild-type extracellular ActRIIB). In some embodiments, the polypeptide including an extracellular ActRII chimera that has reduced or weak binding to BMP9 has the sequence TEEN (SEQ ID NO: 374) or TKEN (SEQ ID NO: 375) at positions $X_3$, $X_4$, $X_5$, and $X_6$. In some embodiments, the polypeptide including an extracellular ActRII chimera that has reduced or weak binding to BMP9 has the sequence KKDS or TKDS at positions $X_3$, $X_4$, $X_5$, and $X_6$. In some embodiments, the polypeptide including an extracellular ActRII chimera does not substantially bind to human BMP9.

In some embodiments, the polypeptide including an extracellular ActRII chimera may bind to human activin A with a $K_D$ of about 800 pM or less (e.g., a $K_D$ of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a $K_D$ of between about 800 pM and about 30 pM). In some embodiments, the polypeptide including an extracellular ActRII chimera may bind to human activin B with a $K_D$ of 800 pM or less (e.g., a $K_D$ of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a $K_D$ of between about 800 pM and about 5 pM) The polypeptide including an extracellular ActRII chimera may also bind to growth and differentiation factor 11 (GDF-11) with a $K_D$ of approximately 5 pM or higher (e.g., a $K_D$ of about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 pM or higher).

## II. Fc domains

In some embodiments, a polypeptide described herein may include an extracellular ActRII variant (e.g., an extracellular ActRIIA variant, an extracellular ActRIIB variant, or an extracellular ActRII chimera) fused to an Fc domain monomer of an immunoglobulin or a fragment of an Fc domain to increase the serum half-life of the polypeptide. A polypeptide including an extracellular ActRII variant fused to an Fc domain monomer may form a dimer (e.g., homodimer or heterodimer) through the interaction between two Fc domain monomers, which form an Fc domain in the dimer. As conventionally known in the art, an Fc domain is the protein structure that is found at the C-terminus of an immunoglobulin. An Fc domain includes two Fc domain monomers that are dimerized by the interaction between the $C_H3$ antibody constant domains. A wild-type Fc domain forms the minimum structure that binds to an Fc receptor, e.g., FcγRI, FcγRIIa, FcγRIIb, FcγRIIIa, FcγRIIIb, FcγRIV. In some embodiments, an Fc domain may be mutated to lack effector functions, typical of a "dead" Fc domain. For example, an Fc domain may include specific amino acid substitutions that are known to minimize the interaction between the Fc domain and an Fcγ receptor. In some embodiments, an Fc domain is from an IgG1 antibody and includes amino acid substitutions L234A, L235A, and G237A. In some embodiments, an Fc domain is from an IgG1 antibody and includes amino acid substitutions D265A, K322A, and N434A. The aforementioned amino acid positions are defined according to Kabat (Sequences of Proteins of Immunological Interest, 5th Ed. Public Health Service, National Institutes of Health, Bethesda, MD. (1991)). The Kabat numbering of amino acid residues may be determined for a given antibody by alignment at regions of homology of the sequence of the antibody with a "standard" Kabat numbered sequence. Furthermore, in some embodiments, an Fc domain does not induce any immune system-related response. For example, the Fc domain in a dimer of a polypeptide including an extracellular ActRII variant fused to an Fc domain monomer may be modified to reduce the interaction or binding between the Fc domain and an Fcγ receptor. The sequence of an Fc domain monomer that may be fused to an extracellular ActRII variant (e.g., an extracellular ActRIIA variant, an extracellular ActRIIB variant, or an extracellular ActRII chimera) is shown below (SEQ ID NO: 97):

THTCPPCPAPELLGGPSVFLFPPKPKDTLMISRTPEVTCVVVDVSHEDPEVKFNWYVDGVEVHN

AKTKPREEQYNSTYRVVSVLTVLHQDWLNGKEYKCKVSNKALPVPIEKTISKAKGQPREPQVYTL

PPSREEMTKNQVSLTCLVKGFYPSDIAVEWESNGQPENNYKTTPPVLDSDGPFFLYSKLTVDKS

RWQQGNVFSCSVMHEALHNHYTQKSLSLSPGK

In some embodiments, an Fc domain is from an IgG1 antibody and includes amino acid substitutions L12A, L13A, and G15A, relative to the sequence of SEQ ID NO: 97. In some embodiments, an Fc domain is from an IgG1 antibody and includes amino acid substitutions D43A, K100A, and N212A, relative to the sequence of SEQ ID NO: 97. In some embodiments, the terminal lysine is absent from the Fc domain monomer having the sequence of SEQ ID NO: 97. In some embodiments, an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) may be fused to the N- or C-terminus of an Fc domain monomer (e.g., SEQ ID NO: 97) through conventional genetic or chemical means, e.g., chemical conjugation. If desired, a linker (e.g., a spacer) can be inserted between the extracellular ActRII variant and the Fc domain monomer. The Fc domain monomer can be fused to the N- or C-terminus (e.g., C-terminus) of the extracellular ActRII variant.

In some embodiments, a polypeptide described herein may include an extracellular ActRII variant (e.g., an extracellular ActRIIA variant, an extracellular ActRIIB variant, or an extracellular ActRII chimera) fused to an Fc domain. In some embodiments, the Fc domain contains one or more amino acid substitutions that reduce or inhibit Fc domain dimerization. In some embodiments, the Fc domain contains a hinge domain. The Fc domain can be of immunoglobulin antibody isotype IgG, IgE, IgM, IgA, or IgD. Additionally, the Fc domain can be an IgG subtype (e.g., IgG1, IgG2a, IgG2b, IgG3, or IgG4). The Fc domain can also be a non-naturally occurring Fc domain, e.g., a recombinant Fc domain.

Methods of engineering Fc domains that have reduced dimerization are known in the art. In some embodiments, one or more amino acids with large side chains (e.g., tyrosine or tryptophan) may be introduced to the $C_H3$-$C_H3$ dimer interface to hinder dimer formation due to steric clash. In other embodiments, one or more amino acids with small side chains (e.g., alanine, valine, or threonine) may be introduced to the $C_H3$-$C_H3$ dimer interface to remove favorable interactions. Methods of introducing amino acids with large or small side chains in the $C_H3$ domain are described in, e.g., Ying et al. (J Biol Chem. 287:19399-19408, 2012), U.S. Patent Publication No. 2006/0074225, U.S. Patent Nos. 8,216,805 and 5,731,168, Ridgway et al. (Protein Eng. 9:617-612, 1996), Atwell et al. (J Mol Biol. 270:26-35, 1997), and Merchant et al. (Nat Biotechnol. 16:677-681, 1998), all of which are incorporated herein by reference in their entireties.

In yet other embodiments, one or more amino acid residues in the $C_H3$ domain that make up the $C_H3$-$C_H3$ interface between two Fc domains are replaced with positively-charged amino acid residues (e.g., lysine, arginine, or histidine) or negatively-charged amino acid residues (e.g., aspartic acid or glutamic acid) such that the interaction becomes electrostatically unfavorable depending on the specific charged amino acids introduced. Methods of introducing charged amino acids in the $C_H3$ domain to disfavor or prevent dimer formation are described in, e.g., Ying et al. (J Biol Chem. 287:19399-19408, 2012), U.S. Patent Publication Nos. 2006/0074225, 2012/0244578, and 2014/0024111, all of which are incorporated herein by reference in their entireties.

In some embodiments of the invention, an Fc domain includes one or more of the following amino acid substitutions: T366W, T366Y, T394W, F405W, Y349T, Y349E, Y349V, L351T, L351H, L351N, L352K, P353S, S354D, D356K, D356R, D356S, E357K, E357R, E357Q, S364A, T366E, L368T, L368Y, L368E, K370E, K370D, K370Q, K392E, K392D, T394N, P395N, P396T, V397T, V397Q, L398T, D399K, D399R, D399N, F405T, F405H, F405R, Y407T, Y407H, Y407I, K409E, K409D, K409T, and K409I, relative to the sequence of human IgG1. In some embodiments, the terminal lysine is absent from the Fc domain amino acid sequence. In one particular embodiment, an Fc domain includes the amino acid substitution T366W, relative to the sequence of human IgG1. The sequence of a wild-type Fc domain is shown below in SEQ ID NO: 150:

DKTHTCPPCPAPELLGGPSVFLFPPKPKDTLMISRTPEVTCVVVDVSHEDPEVKFNWYVDGVEV

HNAKTKPREEQYNSTYRVVSVLTVLHQDWLNGKEYKCKVSNKALPAPIEKTISKAKGQPREPQV

YTLPPSRDELTKNQVSLTCLVKGFYPSDIAVEWESNGQPENNYKTTPPVLDSDGSFFLYSKLTVD

KSRWQQGNVFSCSVMHEALHNHYTQKSLSLSPGK.

An exemplary sequence for a wild-type Fc domain lacking the terminal lysine is provided below (SEQ ID NO: 155):

DKTHTCPPCPAPELLGGPSVFLFPPKPKDTLMISRTPEVTCVVVDVSHEDPEVKFNWYV

DGVEVHNAKTKPREEQYNSTYRVVSVLTVLHQDWLNGKEYKCKVSNKALPAPIEKTISK

AKGQPREPQVYTLPPSRDELTKNQVSLTCLVKGFYPSDIAVEWESNGQPENNYKTTPP

VLDSDGSFFLYSKLTVDKSRWQQGNVFSCSVMHEALHNHYTQKSLSLSPG.

### III. Albumin-binding peptide

In some embodiments, a polypeptide described herein may include an extracellular ActRII variant (e.g., an extracellular ActRIIA variant, an extracellular ActRIIB variant, or an extracellular ActRII chimera) fused to a serum protein-binding peptide. Binding to serum protein peptides can improve the pharmacokinetics of protein pharmaceuticals.

As one example, albumin-binding peptides that can be used in the methods and compositions described here are generally known in the art. In one embodiment, the albumin binding peptide includes the sequence DICLPRWGCLW (SEQ ID NO: 151).

In the present invention, albumin-binding peptides may be joined to the N- or C-terminus (e.g., C-terminus) of an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) to increase the serum half-life of the extracellular ActRII variant. In some embodiments, an albumin-binding peptide is joined, either directly or through a linker, to the N- or C-terminus of an extracellular ActRII variant.

In some embodiments, an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) may be fused to the N- or C-terminus of albumin-binding peptide (e.g., SEQ ID NO: 151) through conventional genetic or chemical means, e.g., chemical conjugation. If desired, a linker (e.g., a spacer) can be inserted between the extracellular ActRII variant and the albumin-binding peptide. Without being bound to a theory, it is expected that inclusion of an albumin-binding peptide in an extracellular ActRII variant described herein may lead to prolonged retention of the therapeutic protein through its binding to serum albumin.

### IV. Fibronectin domain

In some embodiments, a polypeptide described herein may include an extracellular ActRII variant (e.g., an extracellular ActRIIA variant, an extracellular ActRIIB variant, or an extracellular ActRII chimera) fused to fibronectin domains. Binding to fibronectin domains can improve the pharmacokinetics of protein pharmaceuticals.

Fibronectin domain is a high molecular weight glycoprotein of the extracellular matrix, or a fragment thereof, that binds to, e.g., membrane-spanning receptor proteins such as integrins and extracellular matrix components such as collagens and fibrins. In some embodiments of the present invention, a fibronectin domain is joined to the N- or C-terminus (e.g., C-terminus) of an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) to increase the serum half-life of the extracellular ActRII variant. A fibronectin domain can be joined, either directly or through a linker, to the N- or C-terminus of an extracellular ActRII variant.

As one example, fibronectin domains that can be used in the methods and compositions described here are generally known in the art. In one embodiment, the fibronectin domain is a fibronectin type III domain (SEQ ID NO: 152, below) having amino acids 610-702 of the sequence of UniProt ID NO: P02751.

GPVEVFITETPSQPNSHPIQWNAPQPSHISKYILRWRPKNSVGRWKEATIPGHLNSYTIK

GLKPGVVYEGQLISIQQYGHQEVTRFDFTTTST

In another embodiment, the fibronectin domain is an adnectin protein.

In some embodiments, an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) may be fused to the N- or C-terminus of a fibronectin domain (e.g., SEQ ID NO: 152) through conventional genetic or chemical means, e.g., chemical conjugation. If desired, a linker (e.g., a spacer) can be inserted between the extracellular ActRII variant and the fibronectin domain. Without being bound to a theory, it is expected that inclusion of a fibronectin domain in an extracellular ActRII variant described herein may lead to prolonged retention of the therapeutic protein through its binding to integrins and extracellular matrix components such as collagens and fibrins.

**V. Serum albumin**

In some embodiments, a polypeptide described herein may include an extracellular ActRII variant (e.g., an extracellular ActRIIA variant, an extracellular ActRIIB variant, or an extracellular ActRII chimera) fused to serum albumin. Binding to serum albumins can improve the pharmacokinetics of protein pharmaceuticals.

Serum albumin is a globular protein that is the most abundant blood protein in mammals. Serum albumin is produced in the liver and constitutes about half of the blood serum proteins. It is monomeric and soluble in the blood. Some of the most crucial functions of serum albumin include transporting hormones, fatty acids, and other proteins in the body, buffering pH, and maintaining osmotic pressure needed for proper distribution of bodily fluids between blood vessels and body tissues. In preferred embodiments, serum albumin is human serum albumin. In some embodiments of the present invention, a human serum albumin is joined to the N- or C-terminus (e.g., C-terminus) of an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) to increase the serum half-life of the extracellular ActRII variant. A human serum albumin can be joined, either directly or through a linker, to the N- or C-terminus of an extracellular ActRII variant.

As one example, serum albumins that can be used in the methods and compositions described herein are generally known in the art. In one embodiment, the serum albumin includes the sequence of UniProt ID NO: P02768 (SEQ ID NO: 153, below).

MKWVTFISLLFLFSSAYSRGVFRRDAHKSEVAHRFKDLGEENFKALVLIAFAQYLQQCPF

EDHVKLVNEVTEFAKTCVADESAENCDKSLHTLFGDKLCTVATLRETYGEMADCCAKQEP

ERNECFLQHKDDNPNLPRLVRPEVDVMCTAFHDNEETFLKKYLYEIARRHPYFYAPELLF

FAKRYKAAFTECCQAADKAACLLPKLDELRDEGKASSAKQRLKCASLQKFGERAFKAWAV

ARLSQRFPKAEFAEVSKLVTDLTKVHTECCHGDLLECADDRADLAKYICENQDSISSKLK

ECCEKPLLEKSHCIAEVENDEMPADLPSLAADFVESKDVCKNYAEAKDVFLGMFLYEYAR

RHPDYSVVLLLRLAKTYETTLEKCCAAADPHECYAKVFDEFKPLVEEPQNLIKQNCELFE

QLGEYKFQNALLVRYTKKVPQVSTPTLVEVSRNLGKVGSKCCKHPEAKRMPCAEDYLSVV

LNQLCVLHEKTPVSDRVTKCCTESLVNRRPCFSALEVDETYVPKEFNAETFTFHADICTL

SEKERQIKKQTALVELVKHKPKATKEQLKAVMDDFAAFVEKCCKADDKETCFAEEGKKLV

AASQAALGL

In some embodiments, an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) may be fused to the N- or C-terminus of a human serum albumin (e.g., SEQ ID NO: 153) through conventional genetic or chemical means, e.g., chemical conjugation. If desired, a linker (e.g., a spacer) can be inserted between the extracellular ActRII variant and the human serum albumin. Without being bound to a theory, it is expected that inclusion of a human serum albumin in an extracellular ActRII variant described herein may lead to prolonged retention of the therapeutic protein.

## VI. Linkers

A polypeptide described herein may include an extracellular ActRII variant (e.g., an extracellular ActRIIA variant having a sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) fused to a moiety by way of a linker. In some embodiments, the moiety increases stability of the polypeptide. Exemplary moieties include an Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin. In the present invention, a linker between a moiety (e.g., an Fc domain monomer (e.g., the sequence of SEQ ID NO: 97), an Fc domain (e.g., a wild-type Fc domain (e.g., SEQ ID NO: 150 or SEQ ID NO: 155)), an Fc domain with amino acid substitutions (e.g., one or more substitutions that reduce dimerization), an albumin-binding peptide (e.g., SEQ ID NO: 151), a fibronectin domain (e.g., SEQ ID NO: 152), or a human serum albumin (e.g., SEQ ID NO: 153)) and an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)), can be an amino acid spacer including 1-200 amino acids. Suitable peptide spacers are known in the art, and include, for example, peptide linkers containing flexible amino acid residues such as glycine, alanine, and serine. In some embodiments, a spacer can contain motifs, e.g., multiple or repeating motifs, of GA, GS, GG, GGA, GGS, GGG, GGGA (SEQ ID NO: 98), GGGS (SEQ ID NO: 99), GGGG (SEQ ID NO: 100), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), or SGGG (SEQ ID NO: 107). In some embodiments, a spacer can contain 2 to 12 amino acids including motifs of GA or GS, e.g., GA, GS, GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), and GSGSGSGSGSGS (SEQ ID NO: 117). In some embodiments, a spacer can contain 3 to 12 amino acids including motifs of GGA or GGS, e.g., GGA, GGS, GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), and GGSGGSGGSGGS (SEQ ID NO: 123). In yet some embodiments, a spacer can contain 4 to 12 amino acids including motifs of GGAG (SEQ ID NO: 104), GGSG (SEQ ID NO: 105), e.g., GGAG (SEQ ID NO: 104), GGSG (SEQ ID NO: 105), GGAGGGAG (SEQ ID NO: 124), GGSGGGSG (SEQ ID NO: 125), GGAGGGAGGGAG (SEQ ID NO: 126), and GGSGGGSGGGSG (SEQ ID NO: 127). In some embodiments, a spacer can contain motifs of GGGGA (SEQ ID NO: 101) or GGGGS (SEQ ID NO: 102), e.g., GGGGAGGGGAGGGGA (SEQ ID NO: 128) and GGGGSGGGGSGGGGS (SEQ ID NO: 129). In some embodiments of the invention, an amino acid spacer between a moiety (e.g., an Fc domain monomer, an Fc domain (e.g., a wild-type Fc domain), an Fc domain with amino acid substitutions (e.g., one or more substitutions that reduce dimerization), an albumin-binding peptide, a fibronectin domain, or a human serum albumin) and an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) may be GGG, GGGA (SEQ ID NO: 98), GGGG (SEQ ID NO: 100), GGGAG (SEQ ID NO: 130), GGGAGG (SEQ ID NO: 131), or GGGAGGG (SEQ ID NO: 132).

In some embodiments, a spacer can also contain amino acids other than glycine, alanine, and serine, e.g., AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACKIPNDLKQKVMNH (SEQ ID NO: 143), GGSAGGSGSGSSGGSSGASGTGTAGGTGSGSGTGSG (SEQ ID NO: 144), AAANSSIDLISVPVDSR (SEQ ID NO: 145), or GGSGGGSEGGGSEGGGSEGGGSEGGGSEGGGSGGGS (SEQ ID NO: 146). In some embodiments, a spacer can contain motifs, e.g., multiple or repeating motifs, of EAAAK (SEQ ID NO: 147). In some embodiments, a spacer can contain motifs, e.g., multiple or repeating motifs, of proline-rich sequences such as $(XP)_n$, in which X may be any amino acid (e.g., A, K, or E) and n is from 1-5, and PAPAP (SEQ ID NO: 148).

The length of the peptide spacer and the amino acids used can be adjusted depending on the two proteins involved and the degree of flexibility desired in the final protein fusion polypeptide. The length of the spacer can be adjusted to ensure proper protein folding and avoid aggregate formation.

In some embodiments, the linker between a moiety (e.g., an Fc domain monomer (e.g., the sequence of SEQ ID NO: 97), an Fc domain (e.g., a wild-type Fc domain (e.g., SEQ ID NO: 150 or SEQ ID NO: 155)), an Fc domain with amino acid substitutions (e.g., one or more substitutions that reduce dimerization), an albumin-binding peptide (e.g., SEQ ID NO: 151), a fibronectin domain (e.g., SEQ ID NO: 152), or a human serum albumin (e.g., SEQ ID NO: 153)) and an extracellular ActRII variant described herein (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)), is an amino acid spacer having the sequence GGG. For example, a polypeptide of the invention can contain an extracellular ActRIIA variant (e.g., any one of SEQ ID NOs: 6-72) fused to an Fc domain (e.g., SEQ ID NO: 155) by a GGG linker. An exemplary polypeptide containing an ActRIIA variant of SEQ ID NO: 69, a GGG linker, and an Fc domain lacking a terminal lysine (SEQ ID NO: 155) is provided below (SEQ ID NO: 156):

GAILGRSETQECLFYNANWELERTNQTGVERCEGEKDKRLHCYATWRNISGSIEIVKKG
CWLDDFNCYDRTDCVETEENPQVYFCCCEGNMCNEKFSYFPEMEVTQPTSGGGDKT
HTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPG

In another example, a polypeptide of the invention can contain an extracellular ActRIIB variant (e.g., any one of SEQ ID NOs: 158-171) fused to an Fc domain (e.g., SEQ ID NO: 150 or SEQ ID NO: 155) by a GGG linker. An exemplary polypeptide containing an ActRIIB variant of SEQ ID NO: 171, a GGG linker, and an Fc domain (SEQ ID NO: 150) is provided below (SEQ ID NO: 172):

GRGEAETRECLYYNANWELERTNQSGVERCEGEKDKRLHCYASWRNSSGSLEIVKKG
CWLDDFNCYDRDTCVATKENPQVYFCCCEGNMCNERFTHLPEAGGPEVTYEPPPTAP
TGGGDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK

## VII. Vectors, host cells, and protein production

The polypeptides of the invention can be produced from a host cell. A host cell refers to a vehicle that includes the necessary cellular components, e.g., organelles, needed to express the polypeptides and fusion polypeptides described herein from their corresponding nucleic acids. The nucleic acids may be included in nucleic acid vectors that can be introduced into the host cell by conventional techniques known in the art (e.g., transformation, transfection, electroporation, calcium phosphate precipitation, direct microinjection, infection, or the like). The choice of nucleic acid vectors depends in part on the host cells to be used. Generally, preferred host cells are of either eukaryotic (e.g., mammalian) or prokaryotic (e.g., bacterial) origin.

*Nucleic acid vector construction and host cells*

A nucleic acid sequence encoding the amino acid sequence of a polypeptide of the invention may be prepared by a variety of methods known in the art. These methods include, but are not limited to, oligonucleotide-mediated (or site-directed) mutagenesis and PCR mutagenesis. A nucleic acid molecule encoding a polypeptide of the invention may be obtained using standard techniques, e.g., gene synthesis. Alternatively, a nucleic acid molecule encoding a wild-type extracellular ActRIIA or ActRIIB may be mutated to include specific amino acid substitutions using standard techniques in the art, e.g., QuikChange™ mutagenesis. Nucleic acid molecules can be synthesized using a nucleotide synthesizer or PCR techniques.

A nucleic acid sequence encoding a polypeptide of the invention may be inserted into a vector capable of replicating and expressing the nucleic acid molecule in prokaryotic or eukaryotic host cells. Many vectors are available in the art and can be used for the purpose of the invention. Each vector may include various components that may be adjusted and optimized for compatibility with the particular host cell. For example, the vector components may include, but are not limited to, an origin of replication, a selection marker gene, a promoter, a ribosome binding site, a signal sequence, the nucleic acid sequence encoding protein of interest, and a transcription termination sequence.

In some embodiments, mammalian cells may be used as host cells for the invention. Examples of mammalian cell types include, but are not limited to, human embryonic kidney (HEK) (e.g., HEK293, HEK 293F), Chinese hamster ovary (CHO), HeLa, COS, PC3, Vero, MC3T3, NS0, Sp2/0, VERY, BHK, MDCK, W138, BT483, Hs578T, HTB2, BT20, T47D, NS0 (a murine myeloma cell line that does not endogenously produce any immunoglobulin chains), CRL7O3O, and HsS78Bst cells. In some embodiments, E. *coli* cells may also be used as host cells for the invention. Examples of E. *coli* strains include, but are not limited to, E. *coli* 294 (ATCC® 31,446), E. *coli* λ 1776 (ATCC® 31,537, E. *coli* BL21 (DE3) (ATCC® BAA-1025), and E. *coli* RV308 (ATCC® 31,608). Different host cells have characteristic and specific mechanisms for the posttranslational processing and modification of protein products (e.g., glycosylation). Appropriate cell lines or host systems may be chosen to ensure the correct modification and processing of the polypeptide expressed. The above-described expression vectors may be introduced into appropriate host cells using conventional techniques in the art, e.g., transformation, transfection, electroporation, calcium phosphate precipitation, and direct microinjection. Once the vectors are introduced into host cells for protein production, host cells are cultured in conventional nutrient media modified as appropriate for inducing promoters, selecting transformants, or amplifying the genes encoding the desired sequences. Methods for expression of therapeutic proteins are known in the art, see, for example, Paulina Balbas, Argelia Lorence (eds.) Recombinant Gene Expression: Reviews and Protocols (Methods in Molecular Biology), Humana Press; 2nd ed. 2004 and Vladimir Voynov and Justin A. Caravella (eds.) Therapeutic Proteins: Methods and Protocols (Methods in Molecular Biology) Humana Press; 2nd ed. 2012.

*Protein production, recovery, and purification*

Host cells used to produce the polypeptides of the invention may be grown in media known in the art and suitable for culturing of the selected host cells. Examples of suitable media for mammalian host cells include Minimal Essential Medium (MEM), Dulbecco's Modified Eagle's Medium (DMEM), Expi293™ Expression Medium, DMEM with supplemented fetal bovine serum (FBS), and RPMI-1640. Examples of suitable media for bacterial host cells include Luria broth (LB) plus necessary supplements, such as a selection agent, e.g., ampicillin. Host cells are cultured at suitable temperatures, such as from about 20 °C to about 39 °C, e.g., from 25 °C to about 37 °C, preferably 37 °C, and $CO_2$ levels, such as 5 to 10%. The pH of the medium is generally from about 6.8 to 7.4, e.g., 7.0, depending mainly on the host organism. If an inducible promoter is used in the expression vector of the invention, protein expression is induced under conditions suitable for the activation of the promoter.

In some embodiments, depending on the expression vector and the host cells used, the expressed protein may be secreted from the host cells (e.g., mammalian host cells) into the cell culture media. Protein recovery may involve filtering the cell culture media to remove cell debris. The proteins may be further purified. A polypeptide of the invention may be purified by any method known in the art of protein purification, for example, by chromatography (e.g., ion exchange, affinity, and size-exclusion column chromatography), centrifugation, differential solubility, or by any other standard technique for the purification of proteins. For example, the protein can be isolated and purified by appropriately selecting and combining affinity columns such as Protein A column (e.g., POROS Protein A chromatography) with chromatography columns (e.g., POROS HS-50 cation exchange chromatography), filtration, ultra filtration, salting-out and dialysis procedures.

In other embodiments, host cells may be disrupted, e.g., by osmotic shock, sonication, or lysis, to recover the expressed protein. Once the cells are disrupted, cell debris may be removed by centrifugation or filtration. In some instances, a polypeptide can be conjugated to marker sequences, such as a peptide to facilitate purification. An example of a marker amino acid sequence is a hexa-histidine peptide (His-tag), which binds to nickel-functionalized agarose affinity column with micromolar affinity. Other peptide tags useful for purification include, but are not limited to, the hemagglutinin "HA" tag, which corresponds to an epitope derived from influenza hemagglutinin protein (Wilson et al., Cell 37:767, 1984).

Alternatively, the polypeptides of the invention can be produced by the cells of a subject (e.g., a human), e.g., in the context of gene therapy, by administrating a vector (such as a viral vector (e.g., a retroviral vector, adenoviral vector, poxviral vector (e.g., vaccinia viral vector, such as Modified Vaccinia Ankara (MVA)), adeno-associated viral vector, and alphaviral vector)) containing a nucleic acid molecule encoding the polypeptide of the invention. The vector, once inside a cell of the subject (e.g., by transformation, transfection, electroporation, calcium phosphate precipitation, direct microinjection, infection, etc.) will promote expression of the polypeptide, which is then secreted from the cell. If treatment of a disease or disorder is the desired outcome, no further action may be required. If collection of the protein is desired, blood may be collected from the subject and the protein purified from the blood by methods known in the art.

## VIII. Pharmaceutical compositions and preparations

The invention features pharmaceutical compositions that include the polypeptides described herein (e.g., a polypeptide including an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)). In some embodiments, a pharmaceutical composition of the invention includes a polypeptide including an extracellular ActRII variant (e.g., an extracellular ActRIIA variant having the sequence of any one of SEQ ID NOs: 1-70 (e.g., SEQ ID NOs: 6-70) or an extracellular ActRII chimera having the sequence of any one of SEQ ID NOs: 174-216 (e.g., 195-216)) with a C-terminal extension (e.g., 1, 2, 3, 4, 5, 6 or more additional amino acids) as the therapeutic protein. In some embodiments, a pharmaceutical composition of the invention includes a polypeptide including an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) fused to a moiety (e.g., Fc domain monomer, or a dimer thereof, an Fc domain (e.g., a wild-type Fc domain), an Fc domain with amino acid substitutions (e.g., one or more substitutions that reduce dimerization), an albumin-binding peptide, a fibronectin domain, or a human serum albumin) as the therapeutic protein. In some embodiments, a pharmaceutical composition of the invention including a polypeptide of the invention may be used in combination with other agents (e.g., therapeutic biologics and/or small molecules) or compositions in a therapy. In addition to a therapeutically effective amount of the polypeptide, the pharmaceutical composition may include one or more pharmaceutically acceptable carriers or excipients, which can be formulated by methods known to those skilled in the art. In some embodiments, a pharmaceutical composition of the invention includes a nucleic acid molecule (DNA or RNA, e.g., mRNA) encoding a polypeptide of the invention, or a vector containing such a nucleic acid molecule.

Acceptable carriers and excipients in the pharmaceutical compositions are nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbenzyl ammonium chloride, resorcinol, and benzalkonium chloride, proteins such as human serum albumin, gelatin, dextran, and immunoglobulins, hydrophilic polymers such as polyvinylpyrrolidone, amino acids such as glycine, glutamine, histidine, arginine, and lysine, and carbohydrates such as glucose, mannose, sucrose, and sorbitol. Pharmaceutical compositions of the invention can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water, physiological saline, and cell culture media (e.g., Dulbecco's Modified Eagle Medium (DMEM), $\alpha$-Modified Eagles Medium ($\alpha$-MEM), F-12 medium). Formulation methods are known in the art, see e.g., Banga (ed.) Therapeutic Peptides and Proteins: Formulation, Processing and Delivery Systems (3rd ed.) Taylor & Francis Group, CRC Press (2015).

The pharmaceutical compositions of the invention may be prepared in microcapsules, such as hydroxylmethylcellulose or gelatin-microcapsule and poly-(methylmethacrylate) microcapsule. The pharmaceutical compositions of the invention may also be prepared in other drug delivery systems such as liposomes, albumin microspheres, microemulsions, nanoparticles, and nanocapsules. Such techniques are described in Remington: The Science and Practice of Pharmacy 22nd edition (2012). The pharmaceutical compositions to be used for in vivo administration must be sterile. This is readily accomplished by filtration through sterile filtration membranes.

The pharmaceutical compositions of the invention may also be prepared as a sustained-release formulation. Suitable examples of sustained-release preparations include semipermeable matrices of solid hydrophobic polymers containing the polypeptides of the invention. Examples of sustained release matrices include polyesters, hydrogels, polylactides, copolymers of L-glutamic acid and $\gamma$ ethyl-L-glutamate, non-degradable ethylene-vinyl acetate, degradable lactic acid-glycolic acid copolymers such as LUPRON DEPOT™, and poly-D-(-)-3-hydroxybutyric acid. Some sustained-release formulations enable release of molecules over a few months, e.g., one to six months, while other formulations release pharmaceutical compositions of the invention for shorter time periods, e.g., days to weeks.

The pharmaceutical composition may be formed in a unit dose form as needed. The amount of active component, e.g., a polypeptide of the invention, included in the pharmaceutical preparations is such that a suitable dose within the designated range is provided (e.g., a dose within the range of 0.01-100 mg/kg of body weight).

The pharmaceutical composition for gene therapy can be in an acceptable diluent, or can include a slow release matrix in which the gene delivery vehicle is imbedded. If hydrodynamic injection is used as the delivery method, the pharmaceutical composition containing a nucleic acid molecule encoding a polypeptide described herein or a vector (e.g., a viral vector) containing the nucleic acid molecule is delivered rapidly in a large fluid volume intravenously. Vectors that may be used as in vivo gene delivery vehicle include, but are not limited to, retroviral vectors, adenoviral vectors, poxviral vectors (e.g., vaccinia viral vectors, such as Modified Vaccinia Ankara), adeno-associated viral vectors, and alphaviral vectors.

**IX. Routes, dosage, and administration**

Pharmaceutical compositions that include the polypeptides of the invention as the therapeutic proteins may be formulated for, e.g., intravenous administration, parenteral administration, subcutaneous administration, intramuscular administration, intra-arterial administration, intrathecal administration, or intraperitoneal administration. The pharmaceutical composition may also be formulated for, or administered via, oral, nasal, spray, aerosol, rectal, or vaginal administration. For injectable formulations, various effective pharmaceutical carriers are known in the art. See, e.g., ASHP Handbook on Injectable Drugs, Toissel, 18th ed. (2014).

In some embodiments, a pharmaceutical composition that includes a nucleic acid molecule encoding a polypeptide of the invention or a vector containing such nucleic acid molecule may be administered by way of gene delivery. Methods of gene delivery are well-known to one of skill in the art. Vectors that may be used for in vivo gene delivery and expression include, but are not limited to, retroviral vectors, adenoviral vectors, poxviral vectors (e.g., vaccinia viral vectors, such as Modified Vaccinia Ankara (MVA)), adeno-associated viral vectors, and alphaviral vectors. In some embodiments, mRNA molecules encoding polypeptides of the invention may be administered directly to a subject.

In some embodiments of the present invention, nucleic acid molecules encoding a polypeptide described herein or vectors containing such nucleic acid molecules may be administered using a hydrodynamic injection platform. In the hydrodynamic injection method, a nucleic acid molecule encoding a polypeptide described herein is put under the control of a strong promoter in an engineered plasmid (e.g., a viral plasmid). The plasmid is often delivered rapidly in a large fluid volume intravenously. Hydrodynamic injection uses controlled hydrodynamic pressure in veins to enhance cell permeability such that the elevated pressure from the rapid injection of the large fluid volume results in fluid and plasmid extravasation from the vein. The expression of the nucleic acid molecule is driven primarily by the liver. In mice, hydrodynamic injection is often performed by injection of the plasmid into the tail vein. In certain embodiments, mRNA molecules encoding a polypeptide described herein may be administered using hydrodynamic injection.

The dosage of the pharmaceutical compositions of the invention depends on factors including the route of administration, the disease to be treated, and physical characteristics, e.g., age, weight, general health, of the subject. A pharmaceutical composition of the invention may include a dosage of a polypeptide of the invention ranging from 0.01 to 500 mg/kg (e.g., 0.01, 0.1, 0.2, 0.3, 0.325, 0.35, 0.375, 0.4, 0.5, 0.75, 1, 1.25, 1.5, 1.75, 2, 2.25, 2.5, 2.75, 3, 3.25, 3.5, 3.75, 4, 4.25, 4.5, 4.75, 5, 10, 15, 20, 25, 30, 35, 40, 45, 50, 100, 150, 200, 250, 300, 350, 400, 450, or 500 mg/kg) and, in a more specific embodiment, about 0.1 to about 30 mg/kg and, in a more specific embodiment, about 0.3 to about 30 mg/kg. The dosage may be adapted by the physician in accordance with conventional factors such as the extent of the disease and different parameters of the subject.

The pharmaceutical compositions are administered in a manner compatible with the dosage formulation and in such amount as is therapeutically effective to result in an improvement or remediation of the symptoms. The pharmaceutical compositions are administered in a variety of dosage forms, e.g., intravenous dosage forms, subcutaneous dosage forms, and oral dosage forms (e.g., ingestible solutions, drug release capsules). Generally, therapeutic proteins are dosed at 0.1-100 mg/kg, e.g., 0.5-50 mg/kg. Pharmaceutical compositions that include a polypeptide of the invention may be administered to a subject in need thereof, for example, one or more times (e.g., 1-10 times or more) daily, weekly, biweekly, every four weeks, monthly, every eight weeks, bimonthly, every 12 weeks, quarterly, every sixteen weeks biannually, annually, or as medically necessary. In some embodiments, pharmaceutical compositions that include a polypeptide of the invention may be administered to a subject in need thereof weekly, biweekly, every four weeks, monthly, every eight weeks, bimonthly, every twelve weeks, quarterly, or every sixteen weeks. Dosages may be provided in either a single or multiple dosage regimens. The timing between administrations may decrease as the medical condition improves or increase as the health of the patient declines.

**X. Methods of Treatment**

The ActRII variants described herein have improved properties compared to endogenous ActRIIA and ActRIIB. For example, the ActRIIA variants generated by introducing residues from ActRIIB into ActRIIA retain the beneficial properties of ActRIIA, such as low binding affinity to BMP9 and longer serum half-life as an Fc fusion protein, and gain some of the beneficial properties of ActRIIB, such as increased binding to activins A and B. The ActRIIB variants generated by introducing residues from ActRIIA into ActRIIB may retain the beneficial properties of ActRIIB, such high binding affinity to activins A and B, and gain some of the beneficial properties of ActRIIA, such as reduced binding affinity to BMP9 and longer serum half-life as an Fc fusion protein. In addition, the ActRII chimeras generated by combining extracellular portions of ActRIIA and ActRIIB may possess beneficial properties of both ActRIIB (e.g., strong binding affinity to activins A and B) and ActRIIA (e.g., reduced binding affinity to BMP9 and/or longer serum half-life as an Fc fusion protein (e.g., compared to ActRIIB-Fc)). These ActRIIA variant, ActRIIB variant, and ActRII chimera properties make for a useful therapeutic that can compete with endogenous activin receptors for ligand binding. As the ActRIIA variants, ActRIIB variants, and ActRII chimeras contain the extracellular portion of the receptor, they will be soluble and able to bind to and sequester ligands (e.g., activins A and B, myostatin, GDF11) without activating intracellular signaling pathways. Based on the discovery that administration of a polypeptide containing an ActRIIA variant described herein led to an increase in serum EPO levels and bone marrow EPO receptor levels at least seven to fourteen days post-injection, polypeptides containing the ActRII variants described herein (e.g., ActRIIA variants, ActRIIB variants, or ActRII chimeras) can be used therapeutically in place of recombinant EPO or an EPO mimetic and can be used to treat any disease or condition that would benefit from increasing EPO and/or EPO receptor levels.

In some embodiments, the polypeptides described herein (e.g., a polypeptide including an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)), e.g., an effective amount of an ActRIIA variant, ActRIIB variant, or ActRII chimera) can be administered to increase EPO levels (e.g., serum EPO levels) and/or EPO receptor levels (e.g., EPO receptor levels in bone marrow cells) in a subject in need thereof (e.g., a subject with low serum EPO). The invention also includes methods of treating a subject having or at risk of developing (e.g., treating, delaying the development of, and/or preventing) a disease or condition that can be treated with EPO or an ESA (e.g., a disease or condition that can be treated by increasing EPO or EPO receptor levels) by administering to the subject an effective amount of a polypeptide described herein (e.g., a polypeptide including an extracellular ActRIIA variant having the sequence of any one of SEQ ID NOs: 1-72 (e.g., SEQ ID NOs: 6-72), a polypeptide including an extracellular ActRIIB variant having the sequence of any one of SEQ ID NOs: 157-171 (e.g., SEQ ID NOs: 158-171), or a polypeptide including an extracellular ActRII chimera having the sequence of any one of SEQ ID NOs: 174-216 (e.g., 195-216)), e.g., an effective amount of an ActRIIA variant, ActRIIB variant, or ActRII chimera). Diseases and conditions that can be treated by increasing EPO or EPO receptor levels include end-stage renal disease, renal insufficiency, polycythemia, anemia due to dialysis, early anemia of prematurity, iron overload (e.g., hemochromatosis), disease states, disorders, and states of hematologic irregularity, such as pregnancy, a menstrual disorder, and space flight, ischemia (CNS ischemia, liver ischemia, renal ischemia, or cardiac ischemia), ulcers, burns, wounds (e.g., chronic wounds), ischemia-reperfusion injury (e.g., ischemia-reperfusion injury associated with surgery or organ transplantation), an ischemic disorder or condition (e.g., myocardial infarction, ischemic stroke, occlusive arterial disease, chronic venous insufficiency, pulmonary embolism, circulatory shock, such as hemorrhagic, septic, or cardiogenic shock, acute respiratory failure, chronic heart failure, atherosclerosis, cardiac cirrhosis, macular degeneration, sleep apnea, Raynaud's disease, systemic sclerosis, nonbacterial thrombotic endocarditis, angina pectoris, transient ischemic attacks, chronic alcoholic liver disease, or ischemia resulting from general anesthesia), hypoxia (e.g., perinatal hypoxia or a hypoxic condition or disorder such as a pulmonary disorder (e.g., hypoxic hypoxia, such as COPD), severe pneumonia, pulmonary edema, hyaline membrane disease, liver or renal disease, cancer or other chronic illness, and altitude sickness), and aging. A polypeptide described herein can also be used to treat a subject receiving kidney dialysis, to treat a subject who has recently received a stem cell transplant, to increase red blood cell count in a subject prior to surgery, or as a pretreatment or further treatment for a tissue or organ to be transplanted (such as for treatment of the tissue or organ before (e.g., directly before), during, or directly after transplantation).

Given that EPO has been found to stimulate the mobilization, proliferation, migration, and differentiation of endothelial progenitor cells, the polypeptides described herein can also be used to treat a disease associated with dysfunction of endothelial progenitor cells. Such diseases include heart failure, angina pectoris, endotheliosis (e.g., reticuloendotheliosis), age-related cardiovascular disorder, coronary heart disease, atherosclerosis, myocardial ischemia, hypercholesterolemia, ischemic disorders of the extremities, Raynaud's disease, preeclampsia, pregnancy-induced hypertension, endothelium-mediated chronic inflammatory disorders (e.g., inflammation of the vessels), wound healing, and chronic or acute renal failure (also referred to as chronic kidney disease and acute kidney failure, respectively). Since EPO has been shown to have a mitogenic and chemotactic effect on vascular endothelial cells, the ActRII variant polypeptides (e.g., an extracellular ActRIIA variant, ActRIIB variant, or ActRII chimera) can also be used to promote the growth of new blood vessels (vasculogenesis) and/or the replacement of damaged vascular regions through local formation of new blood vessels, such as collateral coronary blood vessels (e.g., those that may occur after myocardial infarction), for granulation tissue formation (e.g. in damaged tissue, wounds, and ulcers), for trauma treatment, for post-vascular graft treatment, and for production of vascular prostheses such as heart valves.

EPO has also been found to have anti-inflammatory and neuroprotective effects. Therefore, the polypeptides described herein can also be used to treat a neurological disorder and/or an inflammatory brain disease, such as a demyelinating disease (e.g., multiple sclerosis, neuromyelitis optica, acute disseminated encephalomyelitis, transverse myelitis), epilepsy, spinal cord injury (e.g., an acute spinal cord injury), a complication following traumatic brain injury (e.g., to treat a symptom of the traumatic brain injury, such as hypotension, hypoxemia, brain swelling, headache, neck pain, difficulty remembering, difficulty concentrating, difficulty making decisions, fatigue, a mood change, nausea, photophobia, blurred vision, ear ringing, a loss of sense of taste, and a loss of sense of smell, seizures, coma, muscle weakness, paralysis, or a progressive decline in neurologic function), a chronic inflammatory brain disease (e.g., a neurodegenerative disease, such as Alzheimer's disease (AD), Parkinson's disease (PD), Huntington's disease, amyotrophic lateral sclerosis (ALS), or age-related macular degeneration (AMD)), or a neurological disorder associated with surgery, such as thoracoabdominal aortic surgery, in addition to diseases or conditions that have an inflammatory or autoimmune component, such as acute cerebrovascular injury, acute brain injury, acute cardiovascular injury, arthritis, an autoimmune disease, a stroke, a neurological injury, and immune-mediated inflammation. The polypeptide may treat the neurological disorder or inflammatory brain disease by reducing infiltration of mononuclear cells into the brain of the subject, improving a neurological deficit, and/or reducing axonal damage and/or neuronal and/or glial cell death in at least one region of the brain of the subject affected, directly or indirectly, by the disease, disorder or condition.

Gastrointestinal dysmotility can also be treated using EPO. Accordingly, the polypeptides described herein may be used to treat gastrointestinal dysmotility due to intestinal injury, abdominal trauma, an intestinal inflammatory condition (e.g., inflammatory bowel diseases (IBD), such as Crohn's Disease and Ulcerative Colitis), an intestinal infection (e.g., a bacterial infection, such as infections that lead to sepsis and bacteremia and localized infections such as peritonitis and ascites), slow transit constipation (e.g., chronic constipation, idiopathic constipation, constipation due to post-operative ileus, or constipation caused by opiate use), post-operative ileus, a neurodegenerative injury, a neurotraumatic injury, a congenital problem (e.g., Gastroschisis, omphalocele, aganglionic megacolon, Hirschsprung's disease, chronic intestinal pseudo-obstruction, small left colon syndrome, anorectal anomalies, esophageal dysplasia and atresias, ectopic anus, congenital hernias, internal anal sphincter achalasia), or a malnutrition-malabsorption problem (e.g., due to an intestinal injury, an abdominal trauma, an intestinal inflammatory condition, an intestinal infection, constipation (e.g., constipation caused by opiate use), post-operative ileus, a neurodegenerative injury, a neurotraumatic injury, a congenital problem, Gaucher disease, refeeding syndrome, extremely low birth weight infants, cancer cachexia, infection, cancer, spinal cord dysfunction, spinal dysraphism, bifida, tumor, central nervous system dysfunction, peripheral neuropathy, removal of part of the gastrointestinal tract, hemorrhage, liver dysfunction, celiac disease, cystic fibrosis, a muscular dystrophy, or cerebral palsy).

The polypeptides described herein can also be used to treat chronic or recurrent disease such as asthma, a viral disease or infection (e.g., HIV infection or HCV infection), hypertension, a systemic microbial infection, cancer, a disease of the endocrine system, a disease of the reproductive system, psychosis, a genetic disease, allergy, a gastrointestinal disease, arterial sclerosis, a cardiovascular disease, graft-vs-host disease, or an inflammatory disease. Polypeptides containing an ActRII variant (e.g., an ActRIIA variant, an ActRIIB variant, or an ActRII chimera) can also be used to enhance athletic performance, improve exercise capacity, and facilitate or enhance aerobic conditioning. Such methods can be used, e.g., by athletes to facilitate training and by soldiers to improve stamina and endurance. In some embodiments, the methods described herein are directed to affecting myostatin, activin A, activin B, and/or BMP9 signaling (e.g., reducing or inhibiting the binding of activin A, activin B, myostatin, and/or BMP9 to their endogenous receptors, e.g., ActRIIA, ActRIIB, and/or BMPRII) in a subject having a disease or condition that can be treated with EPO or an ESA. In some embodiments, the methods described herein increase EPO levels (e.g., serum EPO levels) and/or EPO receptor levels (e.g., bone marrow EPO receptor levels) compared to measurements obtained prior to treatment or compared to measurements obtained from untreated subjects or control treated subjects having the same disease or condition.

In some embodiments, the methods described herein (e.g., the methods of treating any of the diseases or conditions described herein) do not cause any vascular complications in the subject, such as increased vascular permeability or leakage.

In any of the methods described herein, a polypeptide including an extracellular ActRII variant (e.g., an extracellular ActRIIA variant having the sequence of any one of SEQ ID NOs: 1-71 (e.g., SEQ ID NOs: 6-71) or an extracellular ActRII chimera having the sequence of any one of SEQ ID NOs: 174-216 (e.g., 195-216)) that further includes a C-terminal extension of one or more amino acids (e.g., 1, 2, 3, 4, 5, 6 or more amino acids) may be used as the therapeutic protein. In any of the methods described herein, a dimer (e.g., homodimer or heterodimer) formed by the interaction of two Fc domain monomers that are each fused to a polypeptide including an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) may be used as the therapeutic protein. In any of the methods described herein, a polypeptide including an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., 195-216)) fused to a moiety (e.g., an Fc domain monomer, an Fc domain (e.g., a wild-type Fc domain), an Fc domain with amino acid substitutions (e.g., one more substitutions that reduce dimerization), an albumin-binding peptide, a fibronectin domain, or a human serum albumin) may be used as the therapeutic protein. Nucleic acids encoding the polypeptides described herein, or vectors containing said nucleic acids can also be administered according to any of the methods described herein. In any of the methods described herein, the polypeptide, nucleic acid, or vector can be administered as part of a pharmaceutical composition. Compositions that can be administered to a subject according to the methods described herein are provided in Table 10, Table 11, and Table 12, below.

Table 10

| Row | Composition |
|---|---|
| 1 | A polypeptide comprising an extracellular activin receptor type IIa (ActRIIa) variant, the variant having a sequence of $GAILGRSETQECLX_1X_2NANWX_3X_4X_5X_6TNQTGVEX_7CX_8GX_9X_{10}X_{11}X_{12}X_{13}X_{14}HCX_{15}ATWX_{16}NI$ $SGSIEIVX_{17}X_{18}GCX_{19}X_{20}X_{21}DX_{22}NCYDRTDCVEX_{23}X_{24}X_{25}X_{26}PX_{27}VYFCCCEGNMCNEKFSYF$ $PEMEVTQPTS$ (SEQ ID NO: 1), wherein $X_1$ is F or Y; $X_2$ is F or Y; $X_3$ is E or A; $X_4$ is K or L; $X_5$ is D or E; $X_6$ is R or A; $X_7$ is P or R; $X_8$ is Y or E; $X_9$ is D or E; $X_{10}$ is K or Q; $X_{11}$ is D or A; $X_{12}$ is K or A; $X_{13}$ is R or A; $X_{14}$ is R or L; $X_{15}$ is F or Y; $X_{16}$ is K, R, or A; $X_{17}$ is K, A, Y, F, or I; $X_{18}$ is Q or K; $X_{19}$ is W or A; $X_{20}$ is L or A; $X_{21}$ is D, K, R, A, F, G, M, N, or I; $X_{22}$ is I, F, or A; $X_{23}$ is K or T; $X_{24}$ is K or E; $X_{25}$ is D or E; $X_{26}$ is S or N; and $X_{27}$ is E or Q, and wherein the variant has at least one amino acid substitution relative to a wild-type extracellular ActRIIa having the sequence of SEQ ID NO: 73 or an extracellular ActRIIa having any one of the sequences of SEQ ID NOs: 76-96. |
| 2 | The polypeptide of row 1, wherein the variant has a sequence of $GAILGRSETQECLFX_2NANWX_3X_4X_5X_6TNQTGVEX_7CX_8GX_9KX_{11}X_{12}X_{13}X_{14}HCX_{15}ATWX_{16}NIS$ $GSIEIVX_{17}$ $X_{18}GCX_{19}X_{20}X_{21}DX_{22}NCYDRTDCVEX_{23}X_{24}X_{25}X_{26}PX_{27}VYFCCCEGNMCNEKFSYFPEMEVTQP$ $TS$ (SEQ ID NO: 2). |
| 3 | The polypeptide of row 1 or 2, wherein the variant has a sequence of $GAILGRSETQECLFX_2NANWEX_4X_5RTNQTGVEX_7CX_8GX_9KDKRX_{14}HCX_{15}ATWX_{16}NISGSIEIV$ $KX_{18}GCWLDDX_{22}NCYDRTDCVEX_{23}X_{24}X_{25}X_{26}PX_{27}VYFCCCEGNMCNEKFSYFPEMEVTQPTS$ (SEQ ID NO: 3). |
| 4 | The polypeptide of any one of rows 1-3, wherein the variant has a sequence of $GAILGRSETQECLFX_2NANWEX_4DRTNQTGVEX_7CX_8GX_9KDKRX_{14}HCX_{15}ATWX_{16}NISGSIEIV$ $KX_{18}GCWLDDX_{22}NCYDRTDCVEX_{23}KX_{25}X_{26}PX_{27}VYFCCCEGNMCNEKFSYFPEMEVTQPTS$ (SEQ ID NO: 4). |
| 5 | The polypeptide of any one of rows 1-4, wherein the variant has a sequence of $GAILGRSETQECLFX_2NANWEX_4DRTNQTGVEPCX_8GX_9KDKRX_{14}HCFATWKNISGSIEIVKX_{18}$ $GCWLDDINCYDRTDCVEX_{23}KX_{25}X_{26}PX_{27}VYFCCCEGNMCNEKFSYFPEMEVTQPTS$ (SEQ ID NO: 5). |
| 6 | The polypeptide of row 1, wherein $X_1$ is F. |

(continued)

| Row | Composition |
| --- | --- |
| 7 | The polypeptide of row 1, wherein $X_1$ is Y. |
| 8 | The polypeptide of row 1, 6, or 7 wherein $X_{10}$ is K. |
| 9 | The polypeptide of row 1, 6, or 7 wherein $X_{10}$ is Q. |
| 10 | The polypeptide of any one of rows 1-9, wherein $X_2$ is F. |
| 11 | The polypeptide of any one of rows 1-9, wherein $X_2$ is or Y. |
| 12 | The polypeptide of any one of rows 1, 2, and 6-11, wherein $X_3$ is E. |
| 13 | The polypeptide of any one of rows 1, 2, and 6-11, wherein $X_3$ is A. |
| 14 | The polypeptide of any one of rows 1-13, wherein $X_4$ is K. |
| 15 | The polypeptide of any one of rows 1-13, wherein $X_4$ is L. |
| 16 | The polypeptide of any one of rows 1, 2, 3, and 6-15, wherein $X_5$ is D. |
| 17 | The polypeptide of any one of rows 1, 2, 3, and 6-15, wherein $X_5$ is E. |
| 18 | The polypeptide of any one of rows 1, 2, and 6-17, wherein $X_6$ is R. |
| 19 | The polypeptide of any one of rows 1, 2, and 6-17, wherein $X_6$ is A. |
| 20 | The polypeptide of any one of rows 1-4 and 6-19, wherein $X_7$ is P. |
| 21 | The polypeptide of any one of rows 1-4 and 6-19, wherein $X_7$ is R. |
| 22 | The polypeptide of any one of rows 1-21, wherein $X_8$ is Y. |
| 23 | The polypeptide of any one of rows 1-21, wherein $X_8$ is E. |
| 24 | The polypeptide of any one of rows 1-23, wherein $X_s$ is D. |
| 25 | The polypeptide of any one of rows 1-23, wherein $X_9$ is E. |
| 26 | The polypeptide of any one of rows 1, 2, and 6-25, wherein $X_{11}$ is D. |
| 27 | The polypeptide of any one of rows 1, 2, and 6-25, wherein $X_{11}$ is A. |
| 28 | The polypeptide of any one of rows 1, 2, and 6-27, wherein $X_{12}$ is K. |
| 29 | The polypeptide of any one of rows 1, 2, and 6-27, wherein $X_{12}$ is A. |
| 30 | The polypeptide of any one of rows 1, 2, and 6-29, wherein $X_{13}$ is R. |
| 31 | The polypeptide of any one of rows 1, 2, and 6-29, wherein $X_{13}$ is A. |
| 32 | The polypeptide of any one of rows 1-31, wherein $X_{14}$ is R. |

(continued)

| Row | Composition |
|---|---|
| 33 | The polypeptide of any one of rows 1-31, wherein $X_{14}$ is L. |
| 34 | The polypeptide of any one of rows 1-4 and 6-33, wherein $X_{15}$ is F. |
| 35 | The polypeptide of any one of rows 1-4 and 6-33, wherein $X_{15}$ is Y. |
| 36 | The polypeptide of any one of rows 1-4 and 6-35, wherein $X_{16}$ is K. |
| 37 | The polypeptide of any one of rows 1-4 and 6-35, wherein $X_{16}$ is R. |
| 38 | The polypeptide of any one of rows 1-4 and 6-35, wherein $X_{16}$ is A. |
| 39 | The polypeptide of any one of rows 1, 2, and 6-38, wherein $X_{17}$ is K. |
| 40 | The polypeptide of any one of rows 1, 2, and 6-38, wherein $X_{17}$ is A. |
| 41 | The polypeptide of any one of rows 1, 2, and 6-38, wherein $X_{17}$ is Y. |
| 42 | The polypeptide of any one of rows 1, 2, and 6-38, wherein $X_{17}$ is F. |
| 43 | The polypeptide of any one of rows 1, 2, and 6-38, wherein $X_{17}$ is I. |
| 44 | The polypeptide of any one of rows 1-43, wherein $X_{18}$ is Q. |
| 45 | The polypeptide of any one of rows 1-43, wherein $X_{18}$ is K. |
| 46 | The polypeptide of any one of rows 1, 2, and 6-45, wherein $X_{19}$ is W. |
| 47 | The polypeptide of any one of rows 1, 2, and 6-45, wherein $X_{19}$ is A. |
| 48 | The polypeptide of any one of rows 1, 2, and 6-47, wherein $X_{20}$ is L. |
| 49 | The polypeptide of any one of rows 1, 2, and 6-47, wherein $X_{20}$ is A. |
| 50 | The polypeptide of any one of rows 1, 2, and 6-49, wherein $X_{21}$ is D. |
| 51 | The polypeptide of any one of rows 1, 2, and 6-49, wherein $X_{21}$ is K. |
| 52 | The polypeptide of any one of rows 1, 2, and 6-49, wherein $X_{21}$ is R. |
| 53 | The polypeptide of any one of rows 1, 2, and 6-49, wherein $X_{21}$ is A. |
| 54 | The polypeptide of any one of rows 1, 2, and 6-49, wherein $X_{21}$ is F. |
| 55 | The polypeptide of any one of rows 1, 2, and 6-49, wherein $X_{21}$ is G. |
| 56 | The polypeptide of any one of rows 1, 2, and 6-49, wherein $X_{21}$ is M. |
| 57 | The polypeptide of any one of rows 1, 2, and 6-49, wherein $X_{21}$ is N. |
| 58 | The polypeptide of any one of rows 1, 2, and 6-49, wherein $X_{21}$ is I. |

| Row | Composition |
|---|---|
| 59 | The polypeptide of any one of rows 1-4 and 6-58, wherein $X_{22}$ is I. |
| 60 | The polypeptide of any one of rows 1-4 and 6-58, wherein $X_{22}$ is F. |
| 61 | The polypeptide of any one of rows 1-4 and 6-58, wherein $X_{22}$ is A. |
| 62 | The polypeptide of any one of rows 1-61, wherein $X_{23}$ is K. |
| 63 | The polypeptide of any one of rows 1-61, wherein $X_{23}$ is T. |
| 64 | The polypeptide of any one of rows 1, 2, 3, and 6-63, wherein $X_{24}$ is K. |
| 65 | The polypeptide of any one of rows 1, 2, 3, and 6-63, wherein $X_{24}$ is E. |
| 66 | The polypeptide of any one of rows 1-65, wherein $X_{25}$ is D. |
| 67 | The polypeptide of any one of rows 1-65, wherein $X_{25}$ is E. |
| 68 | The polypeptide of any one of rows 1-67, wherein $X_{26}$ is S. |
| 69 | The polypeptide of any one of rows 1-67, wherein $X_{26}$ is N. |
| 70 | The polypeptide of any one of rows 1-69, wherein $X_{27}$ is E. |
| 71 | The polypeptide of any one of rows 1-69, wherein $X_{27}$ is Q. |
| 72 | The polypeptide of any one of rows 1-71, wherein $X_{23}$ is T, $X_{24}$ is E, $X_{25}$ is E, and $X_{26}$ is N. |
| 73 | The polypeptide of any one of rows 1-71, wherein $X_{23}$ is T, $X_{24}$ is K, $X_{25}$ is E, and $X_{26}$ is N. |
| 74 | The polypeptide of any one of rows 1-73, wherein $X_{17}$ is K. |
| 75 | The polypeptide of row 1, wherein the variant has the sequence of any one of SEQ ID NOs: 6-72. |
| 76 | The polypeptide of row 75, wherein the variant has the sequence of SEQ ID NO: 69. |
| 77 | The polypeptide of row 75, wherein the variant has the sequence of SEQ ID NO: 58. |
| 78 | The polypeptide of row 75, wherein the variant has the sequence of SEQ ID NO: 6. |
| 79 | The polypeptide of row 75, wherein the variant has the sequence of SEQ ID NO: 38. |
| 80 | The polypeptide of row 75, wherein the variant has the sequence of SEQ ID NO: 41. |
| 81 | The polypeptide of row 75, wherein the variant has the sequence of SEQ ID NO: 44. |
| 82 | The polypeptide of row 75, wherein the variant has the sequence of SEQ ID NO: 70. |
| 83 | The polypeptide of row 75, wherein the variant has the sequence of SEQ ID NO: 71. |
| 84 | The polypeptide of row 75, wherein the variant has the sequence of SEQ ID NO: 72. |

| Row | Composition |
|-----|-------------|
| 85 | The polypeptide of any one of rows 1-84, wherein the amino acid at position $X_{24}$ is replaced with the amino acid K. |
| 86 | The polypeptide of any one of rows 1-85, wherein the amino acid at position $X_{24}$ is replaced with the amino acid E. |
| 87 | The polypeptide of any one of rows 1-86, further comprising a C-terminal extension of one or more amino acids. |
| 88 | The polypeptide of row 87, wherein the C-terminal extension is NP. |
| 89 | The polypeptide of row 87, wherein the C-terminal extension is NPVTPK (SEQ ID NO: 154). |
| 90 | The polypeptide of any one of rows 1-89, further comprising an Fc domain monomer fused to the C-terminus of the polypeptide by way of a linker. |
| 91 | The polypeptide of row 90, wherein the Fc domain monomer comprises the sequence of SEQ ID NO: 97. |
| 92 | The polypeptide of any one of rows 1-89, further comprising a wild-type Fc domain fused to the C-terminus of the polypeptide by way of a linker. |
| 93 | The polypeptide of row 92, wherein the wild-type Fc domain comprises the sequence of SEQ ID NO: 150 or SEQ ID NO: 155. |
| 94 | The polypeptide of row 93, wherein the wild-type Fc domain comprises the sequence of SEQ ID NO: 155. |
| 95 | The polypeptide of any one of rows 1-89, further comprising an Fc domain comprising amino acid substitutions fused to the C-terminus of the polypeptide by way of a linker. |
| 96 | The polypeptide of row 95, wherein the Fc domain does not form a dimer. |
| 97 | The polypeptide of any one of rows 1-89, further comprising an albumin-binding peptide fused to the C-terminus of the polypeptide by way of a linker. |
| 98 | The polypeptide of row 97, wherein the albumin-binding peptide comprises the sequence of SEQ ID NO: 151. |
| 99 | The polypeptide of any one of rows 1-89, further comprising a fibronectin domain fused to the C-terminus of the polypeptide by way of a linker. |
| 100 | The polypeptide of row 99, wherein the fibronectin domain comprises the sequence of SEQ ID NO: 152. |
| 101 | The polypeptide of any one of rows 1-89, further comprising a human serum albumin fused to the C-terminus of the polypeptide by way of a linker. |
| 102 | The polypeptide of row 101, wherein the human serum albumin comprises the sequence of SEQ ID NO: 153. |
| 103 | The polypeptide of row 90 or 91, wherein the polypeptide forms a dimer. |
| 104 | The polypeptide of any one of rows 90-103, wherein the linker is an amino acid spacer. |
| 105 | The polypeptide of row 104, wherein the amino acid spacer is GGG, GGGA (SEQ ID NO: 98), GGGG (SEQ ID NO: 100), GGGAG (SEQ ID NO: 130), GGGAGG (SEQ ID NO: 131), or GGGAGGG (SEQ ID NO: 132). |
| 106 | The polypeptide of row 105, wherein the amino acid spacer is GGG. |
| 107 | The polypeptide of row 94 or 106, wherein the polypeptide has the sequence of SEQ ID NO: 156. |

| Row | Composition |
|---|---|
| 108 | The polypeptide of row 104, wherein the amino acid spacer is GGGS (SEQ ID NO: 99), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), SGGG (SEQ ID NO: 107), GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), and GSGSGSGSGSGS (SEQ ID NO: 117), GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), GGSGGSGGSGGS (SEQ ID NO: 123), GGAGGGAG (SEQ ID NO: 124), GGSGGGSG (SEQ ID NO: 125), GGAGGGAGGGAG (SEQ ID NO: 126), and GGSGGGSGGGSG (SEQ ID NO: 127), GGGGAGGGGAGGGGA (SEQ ID NO: 128), GGGGSGGGGSGGGGS (SEQ ID NO: 129), AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACKIPNDLKQKVMNH (SEQ ID NO: 143), GGSAGGSGSGSSGGSSGASGTGTAGGTGSGSGTGSG (SEQ ID NO: 144), AAANSSIDLISVPVDSR (SEQ ID NO: 145), GGSGGGSEGGGSEGGGSEGGGSEGGGSEGGGSGGGS (SEQ ID NO: 146), EAAAK (SEQ ID NO: 147), or PAPAP(SEQ ID NO: 148). |
| 109 | The polypeptide of any one of rows 1-108, wherein the polypeptide has a serum half-life of at least 7 days. |
| 110 | The polypeptide of any one of rows 1-109, wherein the polypeptide binds to activin A, activin B, and/or myostatin and has reduced or weak binding to human BMP9. |
| 111 | The polypeptide of row 110, wherein the polypeptide does not substantially bind to human BMP9. |
| 112 | The polypeptide of any one of rows 1-111, wherein the polypeptide binds to human activin A with a $K_D$ of 800 pM or less. |
| 113 | The polypeptide of any one of rows 1-112, wherein the polypeptide binds to human activin B with a $K_D$ of 800 pM or less. |
| 114 | The polypeptide of any one of rows 1-113, wherein the polypeptide binds to human GDF-11 with a $K_D$ of 5 pM or higher. |
| 115 | A nucleic acid molecule encoding a polypeptide of any one of rows 1-114. |
| 116 | A vector comprising the nucleic acid molecule of row 115. |
| 117 | A host cell that expresses a polypeptide of any one of rows 1-114, wherein the host cell comprises a nucleic acid molecule of row 115 or a vector of row 116, wherein the nucleic acid molecule or vector is expressed in the host cell. |
| 118 | A pharmaceutical composition comprising a polypeptide of any one of rows 1-114, a nucleic acid molecule of row 115, or a vector of row 116, and one or more pharmaceutically acceptable carriers or excipients. |
| 119 | The pharmaceutical composition of row 118, wherein the polypeptide is in a therapeutically effective amount. |
| 120 | A construct comprising two identical polypeptides (e.g., a homodimer), each comprising an extracellular ActRIIA variant of any one of rows 1-89 (e.g., an ActRIIA variant having a sequence of any one of SEQ ID NOs: 1-72) fused (e.g., linked using an amino acid spacer) to the N- or C-terminus of an Fc domain monomer (e.g., the sequence of SEQ ID NO: 97). The two Fc domain monomers in the two polypeptides interact to form an Fc domain in the construct. |
| 121 | A construct comprising two different polypeptides (e.g., a heterodimer), each comprising an extracellular ActRIIA variant of any one of rows 1-89 (e.g., an ActRIIA variant having a sequence of any one of SEQ ID NOs: 1-72) fused (e.g., linked using an amino acid spacer) to the N- or C-terminus of an Fc domain monomer (e.g., the sequence of SEQ ID NO: 97). The two Fc domain monomers in the two polypeptides interact to form an Fc domain in the construct. |

Table 11

| Row | Composition |
|---|---|
| 1 | A polypeptide comprising an extracellular activin receptor type IIB (ActRIIB) variant, the variant having one or more amino acid substitutions relative to the sequence of GRGEAETRECIYYNANWELERTNQSGLERCEGEQDKRLHCYASWRNSSGTIELVKKGCWL DDFNCYDRQECVATEENPQVYFCCCEGNFCNERFTHL PEAGGPEVTYEPPPTAPT (SEQ ID NO: 74), wherein the variant comprises one or more amino acid substitutions that impart reduced BMP9 binding relative to wild type extracellular ActRIIB and one or more additional amino acid substitutions, wherein the substitutions that reduce BMP9 binding comprise one or more of:<br><br>a) amino acid substitution E75K;<br>b) amino acid substitutions Q69T and E70D; or<br>c) amino acid substitutions Q69D and E70T, optionally wherein the variant is truncated from the N-terminus by deletion of 1, 2, 3, 4, 5, 6, or 7 amino acids. |

| Row | Composition |
|---|---|
| 2 | The polypeptide of row 1, wherein the variant comprises one or more amino acid substitutions selected from the group consisting of I11L, Y12F, L19K, E20D, S25T, L27V, R29P, E31Y, E33D, Q34K, L38R, Y41F, R45K, S47I, S48T, T50S, I51L, L53I, K56Q, F63I, T74K, E76D, N77S, Q79E, and F89M. |
| 3 | The polypeptide of row 1 or 2, wherein the variant comprises amino acid substitutions E75K, E20D, and F63I. |
| 4 | The polypeptide of row 1 or 2, wherein the variant comprises amino acid substitution E75K. |
| 5 | The polypeptide of row 4, wherein the variant comprises amino acid substitutions T74K, E76D, N77S, and Q79E. |
| 6 | The polypeptide of row 5, wherein the variant further comprises one or more additional amino acid substitutions. |
| 7 | The polypeptide of row 6, wherein the variant comprises amino acid substitutions Y41F, R45K, and K56Q. |
| 8 | The polypeptide of row 7, wherein the variant further comprises amino acid substitutions Y12F, L19K, E20D, R29P, E31Y, E33D, L38R, and F63I. |
| 9 | The polypeptide of row 6, wherein the variant comprises amino acid substitutions S25T and S47I. |
| 10 | The polypeptide of row 9, wherein the variant comprises amino acid substitution S48T. |
| 11 | The polypeptide of row 6, wherein the variant comprises amino acid substitution R29P. |
| 12 | The polypeptide of row 6, wherein the variant comprises amino acid substitutions E31Y, E33D, and Q34K. |
| 13 | The polypeptide of row 6, wherein the variant comprises amino acid substitutions Y12F, L19K, and E20D. |
| 14 | The polypeptide of row 6, wherein the variant comprises amino acid substitutions E31Y, E33D, and L38R. |
| 15 | The polypeptide of row 1 or 2, wherein the variant comprises amino acid substitutions Q69T and E70D. |
| 16 | The polypeptide of any one of rows 1, 2, and 15, wherein the variant comprises amino acid substitutions Q69T and E70D and additional amino acid substitutions I11L, L27V, Q34K, T50S, I51L, L53I, and F89M. |

EP 4 674 480 A2

| Row | Composition |
|---|---|
| 17 | The polypeptide of row 1 or 2, wherein the variant comprises amino acid substitutions Q69D and E70T. |
| 18 | The polypeptide of any one of rows 1, 2, and 17, wherein the variant comprises amino acid substitutions Q69D and E70T and additional amino acid substitutions I11L, L27V, Q34K, T50S, I51L, L53I, and F89M. |
| 19 | The polypeptide of any one of rows 15-18, wherein the variant comprises amino acid substitution E75K. |
| 20 | A polypeptide comprising an ActRIIB variant, the variant having a sequence of GRGEAETRECX$_1$X$_2$ YNANWEX$_3$ X$_4$RTNQX$_5$GX$_6$EX$_7$CX$_8$GX$_9$X$_{10}$DKRX$_{11}$HCX$_{12}$ASWX$_{13}$NX$_{14}$X$_{15}$ GX$_{16}$X$_{17}$EX$_{18}$VKX$_{19}$GCWLDDX$_{20}$NCYDRX$_{21}$X$_{22}$CVAX$_{23}$X$_{24}$X$_{25}$X$_{26}$PX$_{27}$VYFCCCEGNX$_{28}$CNERF THLPEAGG-PEVTYEPPPTAPT (SEQ ID NO: 157), wherein X$_1$ is I or L; X$_2$ is F or Y; X$_3$ is L or K; X$_4$ is D or E; X$_5$ is T or S; X$_6$ is L or V; X$_7$ is P or R; X$_8$ is Y or E; X$_9$ is D or E; X$_{10}$ is K or Q; X$_{11}$ is R or L; X$_{12}$ is Y or F; X$_{13}$ is R or K; X$_{14}$ is S or I; X$_{15}$ is S or T; X$_{16}$ is S or T; X$_{17}$ is I or L; X$_{18}$ is I or L; X$_{19}$ is K or Q; X$_{20}$ is F or I; X$_{21}$ is Q, T, or D; X$_{22}$ is E, D, or T; X$_{23}$ is K or T; X$_{24}$ is K or E; X$_{25}$ is D or E; X$_{26}$ is S or N; X$_{27}$ is E or Q; and X$_{28}$ is F or M, and wherein X$_{24}$ is E and/or either X$_{21}$ is T and X$_{22}$ is D or X$_{21}$ is D and X$_{22}$ is T, and wherein the variant has at least one amino acid substitution relative to a wild-type extracellular ActRIIB having the sequence of SEQ ID NO: 17, optionally wherein the variant is truncated from the N-terminus by deletion of 1, 2, 3, 4, 5, 6, or 7 amino acids. |
| 21 | The polypeptide of row 20, wherein X$_1$ is I. |
| 22 | The polypeptide of row 20, wherein X$_1$ is L. |
| 23 | The polypeptide of any one of rows 20-22, wherein X$_2$ is F. |
| 24 | The polypeptide of any one of rows 20-22, wherein X$_2$ is Y. |
| 25 | The polypeptide of any one of rows 20-24, wherein X$_3$ is L. |
| 26 | The polypeptide of any one of rows 20-24, wherein X$_3$ is K. |
| 27 | The polypeptide of any one of rows 20-26, wherein X$_4$ is D. |
| 28 | The polypeptide of any one of rows 20-26, wherein X$_4$ is E. |
| 29 | The polypeptide of any one of rows 20-28, wherein X$_5$ is T. |
| 30 | The polypeptide of any one of rows 20-28, wherein X$_5$ is S. |
| 31 | The polypeptide of any one of rows 20-30, wherein X$_6$ is L. |
| 32 | The polypeptide of any one of rows 20-30, wherein X$_6$ is V. |
| 33 | The polypeptide of any one of rows 20-32, wherein X$_7$ is P. |
| 34 | The polypeptide of any one of rows 20-32, wherein X$_7$ is R. |
| 35 | The polypeptide of any one of rows 20-34, wherein X$_8$ is Y. |

| Row | Composition |
|---|---|
| 36 | The polypeptide of any one of rows 20-34, wherein $X_8$ is E. |
| 37 | The polypeptide of any one of rows 20-36, wherein $X_9$ is D. |
| 38 | The polypeptide of any one of rows 20-36, wherein $X_9$ is E. |
| 39 | The polypeptide of any one of rows 20-38, wherein $X_{10}$ is K. |
| 40 | The polypeptide of any one of rows 20-38, wherein $X_{10}$ is Q. |
| 41 | The polypeptide of any one of rows 20-40, wherein $X_{11}$ is R. |
| 42 | The polypeptide of any one of rows 20-40, wherein $X_{11}$ is L. |
| 43 | The polypeptide of any one of rows 20-42, wherein $X_{12}$ is Y. |
| 44 | The polypeptide of any one of rows 20-42, wherein $X_{12}$ is F. |
| 45 | The polypeptide of any one of rows 20-44, wherein $X_{13}$ is R. |
| 46 | The polypeptide of any one of rows 20-44, wherein $X_{13}$ is K. |
| 47 | The polypeptide of any one of rows 20-46, wherein $X_{14}$ is S. |
| 48 | The polypeptide of any one of rows 20-46, wherein $X_{14}$ is I. |
| 49 | The polypeptide of any one of rows 20-48, wherein $X_{15}$ is S. |
| 50 | The polypeptide of any one of rows 20-48, wherein $X_{15}$ is T. |
| 51 | The polypeptide of any one of rows 20-50, wherein $X_{16}$ is S. |
| 52 | The polypeptide of any one of rows 20-50, wherein $X_{16}$ is T. |
| 53 | The polypeptide of any one of rows 20-52, wherein $X_{17}$ is I. |
| 54 | The polypeptide of any one of rows 20-52, wherein $X_{17}$ is L. |
| 55 | The polypeptide of any one of rows 20-54, wherein $X_{18}$ is I. |
| 56 | The polypeptide of any one of rows 20-54, wherein $X_{18}$ is L. |
| 57 | The polypeptide of any one of rows 20-56, wherein $X_{19}$ is K. |
| 58 | The polypeptide of any one of rows 20-56, wherein $X_{19}$ is Q. |
| 59 | The polypeptide of any one of rows 20-58, wherein $X_{20}$ is F. |
| 60 | The polypeptide of any one of rows 20-58, wherein $X_{20}$ is I. |
| 61 | The polypeptide of any one of rows 20-60, wherein $X_{21}$ is Q. |

(continued)

| Row | Composition |
|---|---|
| 62 | The polypeptide of any one of rows 20-60, wherein $X_{21}$ is T. |
| 63 | The polypeptide of any one of rows 20-60, wherein $X_{21}$ is D. |
| 64 | The polypeptide of any one of rows 20-61, wherein $X_{22}$ is E. |
| 65 | The polypeptide of any one of rows 20-60 and 62, wherein $X_{22}$ is D. |
| 66 | The polypeptide of any one of rows 20-60 and 63, wherein $X_{22}$ is T. |
| 67 | The polypeptide of any one of rows 20-66, wherein $X_{23}$ is K. |
| 68 | The polypeptide of any one of rows 20-66, wherein $X_{23}$ is T. |
| 69 | The polypeptide of any one of rows 20-68, wherein $X_{24}$ is K. |
| 70 | The polypeptide of any one of rows 20-60, 62, 63, and 65-68, wherein $X_{24}$ is E. |
| 71 | The polypeptide of any one of rows 20-70, wherein $X_{25}$ is D. |
| 72 | The polypeptide of any one of rows 20-70, wherein $X_{25}$ is E. |
| 73 | The polypeptide of any one of rows 20-72, wherein $X_{26}$ is S. |
| 74 | The polypeptide of any one of rows 20-72, wherein $X_{26}$ is N. |
| 75 | The polypeptide of any one of rows 20-74, wherein $X_{27}$ is E. |
| 76 | The polypeptide of any one of rows 20-74, wherein $X_{27}$ is Q. |
| 77 | The polypeptide of any one of rows 20-76, wherein $X_{28}$ is F. |
| 78 | The polypeptide of any one of rows 20-76, wherein $X_{28}$ is M. |
| 79 | The polypeptide of any one of rows 20-78, wherein $X_{23}$ is T, $X_{24}$ is K, $X_{25}$ is E, and $X_{26}$ is N. |
| 80 | The polypeptide of any one of rows 20-78, wherein $X_{23}$ is T, $X_{24}$ is E, $X_{25}$ is E, and $X_{26}$ is N. |
| 81 | The polypeptide of any one of rows 20-78, wherein $X_{23}$ is K, $X_{24}$ is K, $X_{25}$ is D, and $X_{26}$ is S. |
| 82 | The polypeptide of any one of rows 1-81, wherein the variant has the sequence of any one of SEQ ID NOs: 158-171. |
| 83 | The polypeptide of row 82, wherein the variant has the sequence of SEQ ID NO: 159. |
| 84 | The polypeptide of row 82, wherein the variant has the sequence of SEQ ID NO: 165. |
| 85 | The polypeptide of row 82, wherein the variant has the sequence of SEQ ID NO: 168. |
| 86 | The polypeptide of row 82, wherein the variant has the sequence of SEQ ID NO: 169. |
| 87 | The polypeptide of row 82, wherein the variant has the sequence of SEQ ID NO: 170. |

| Row | Composition |
|---|---|
| 88 | The polypeptide of row 82, wherein the variant has the sequence of SEQ ID NO: 171. |
| 89 | The polypeptide of any one of rows 1-88, wherein the amino acid at position $X_{24}$ is replaced with the amino acid K. |
| 90 | The polypeptide of any one of rows 1-88, wherein the amino acid at position $X_{24}$ is replaced with the amino acid E. |
| 91 | The polypeptide of any one of rows 1-90, wherein the variant is truncated from the N-terminus by deletion of one amino acid. |
| 92 | The polypeptide of any one of rows 1-90, wherein the variant is truncated from the N-terminus by deletion of two amino acids. |
| 93 | The polypeptide of any one of rows 1-90, wherein the variant is truncated from the N-terminus by deletion of three amino acids. |
| 94 | The polypeptide of any one of rows 1-90, wherein the variant is truncated from the N-terminus by deletion of four amino acids. |
| 95 | The polypeptide of any one of rows 1-90, wherein the variant is truncated from the N-terminus by deletion of five amino acids. |
| 96 | The polypeptide of any one of rows 1-90, wherein the variant is truncated from the N-terminus by deletion of six amino acids. |
| 97 | The polypeptide of any one of rows 1-90, wherein the variant is truncated from the N-terminus by deletion of seven amino acids. |
| 98 | The polypeptide of any one of rows 1-97, further comprising an Fc domain monomer fused to the C-terminus of the polypeptide by way of a linker. |
| 99 | The polypeptide of row 98, wherein the Fc domain monomer comprises the sequence of SEQ ID NO: 97. |
| 100 | The polypeptide of row 98 or 99, wherein the polypeptide forms a dimer. |
| 101 | The polypeptide of any one of rows 1-97, further comprising a wild-type Fc domain fused to the C-terminus of the polypeptide by way of a linker. |
| 102 | The polypeptide of row 101, wherein the wild-type Fc domain comprises the sequence of SEQ ID NO: 150 or SEQ ID NO: 155. |
| 103 | The polypeptide of row 102, wherein the wild-type Fc domain comprises the sequence of SEQ ID NO: 150. |
| 104 | The polypeptide of row 102, wherein the wild-type Fc domain comprises the sequence of SEQ ID NO: 155. |

(continued)

| Row | Composition |
|---|---|
| 105 | The polypeptide of any one of rows 1-97, further comprising an Fc domain comprising amino acid substitutions fused to the C-terminus of the polypeptide by way of a linker. |
| 106 | The polypeptide of row 105, wherein the Fc domain does not form a dimer. |
| 107 | The polypeptide of any one of rows 1-97, further comprising an albumin-binding peptide fused to the C-terminus of the polypeptide by way of a linker. |
| 108 | The polypeptide of row 107, wherein the albumin-binding peptide comprises the sequence of SEQ ID NO: 151. |
| 109 | The polypeptide of any one of rows 1-97, further comprising a fibronectin domain fused to the C-terminus of the polypeptide by way of a linker. |
| 110 | The polypeptide of row 109, wherein the fibronectin domain comprises the sequence of SEQ ID NO: 152. |
| 111 | The polypeptide of any one of rows 1-97, further comprising a human serum albumin fused to the C-terminus of the polypeptide by way of a linker. |
| 112 | The polypeptide of row 111, wherein the human serum albumin comprises the sequence of SEQ ID NO: 153. |
| 113 | The polypeptide of any one of rows 98-112, wherein the linker is an amino acid spacer. |
| 114 | The polypeptide of row 113, wherein the amino acid spacer is GGG, GGGA (SEQ ID NO: 98), GGGG (SEQ ID NO: 100), GGGAG (SEQ ID NO: 130), GGGAGG (SEQ ID NO: 131), or GGGAGGG (SEQ ID NO: 132). |
| 115 | The polypeptide of row 114, wherein the amino acid spacer is GGG. |
| 116 | The polypeptide of row 103 or 115, wherein the polypeptide has the sequence of SEQ ID NO: 172. |
| 117 | The polypeptide of row 113, wherein the amino acid spacer is GA, GS, GG, GGA, GGS, GGG, GGGS (SEQ ID NO: 99), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), SGGG (SEQ ID NO: 107), GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), and GSGSGSGSGSGS (SEQ ID NO: 117), GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), GGSGGSGGSGGS (SEQ ID NO: 123), GGAGGGAG (SEQ ID NO: 124), GGSGGGSG (SEQ ID NO: 125), GGAGGGAGGGAG (SEQ ID NO: 126), and GGSGGGSGGGSG (SEQ ID NO: 127), GGGGAGGGGAGGGGA (SEQ ID NO: 128), GGGGSGGGGSGGGGS (SEQ ID NO: 129), AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACK- |

(continued)

| Row | Composition |
|---|---|
|  | IPNDLKQKVMNH (SEQ ID NO: 143), GGSAGGSGSGSGSSGASGTGTAGGTGSGSGTGSG (SEQ ID NO: 144), AAANSSIDLISVPVDSR (SEQ ID NO: 145), GGSGGGSEGGSEGGGSEGGGSEGGSEGGGSGGGS (SEQ ID NO: 146), EAAAK (SEQ ID NO: 147), or PAPAP(SEQ ID NO: 148). |
| 118 | The polypeptide of any one of rows 1-117, wherein the polypeptide has a serum half-life of at least 7 days. |
| 119 | The polypeptide of any one of rows 1-118, wherein the polypeptide binds to activin A, activin B, and/or myostatin and has reduced or weak binding to human BMP9. |
| 120 | The polypeptide of row 119, wherein the polypeptide does not substantially bind to human BMP9. |
| 121 | The polypeptide of any one of rows 1-120, wherein the polypeptide binds to human activin A with a $K_D$ of 800 pM or less. |
| 122 | The polypeptide of any one of rows 1-121, wherein the polypeptide binds to human activin B with a $K_D$ of 800 pM or less. |
| 123 | The polypeptide of any one of rows 1-122, wherein the polypeptide binds to human GDF-11 with a $K_D$ of 5 pM or higher. |
| 124 | A nucleic acid molecule encoding a polypeptide of any one of rows 1-123. |
| 125 | A vector comprising the nucleic acid molecule of row 124. |
| 126 | A host cell that expresses a polypeptide of any one of rows 1-123, wherein the host cell comprises a nucleic acid molecule of row 124 or a vector of row 125, wherein the nucleic acid molecule or vector is expressed in the host cell. |
| 127 | A pharmaceutical composition comprising a polypeptide of any one of rows 1-123, a nucleic acid molecule of row 124, or a vector of row 125, and one or more pharmaceutically acceptable carriers or excipients. |
| 128 | The pharmaceutical composition of row 127, wherein the polypeptide is in a therapeutically effective amount. |
| 129 | A construct comprising two identical polypeptides (e.g., a homodimer), each comprising an extracellular ActRIIB variant of any one of rows 1-97 (e.g., an ActRIIB variant having a sequence of any one of SEQ ID NOs: 157-171) fused (e.g., linked using an amino acid spacer) to the N- or C-terminus of an Fc domain monomer (e.g., the sequence of SEQ ID NO: 97). The two Fc domain monomers in the two polypeptides interact to form an Fc domain in the construct. |
| 130 | A construct comprising two different polypeptides (e.g., a heterodimer), each comprising an extracellular ActRIIB variant of any one of rows 1-97 (e.g., an ActRIIB variant having a sequence of any one of SEQ ID NOs: 157-171) fused (e.g., linked using an amino acid spacer) to the N- or C-terminus of an Fc domain monomer (e.g., the sequence of SEQ ID NO: 97). The two Fc domain monomers in the two polypeptides interact to form an Fc domain in the construct. |

73

Table 12

| Row | Composition |
|---|---|
| 1 | A polypeptide comprising an extracellular activin receptor type II (ActRII) chimera, the chimera having a sequence of any one of

GAILGRAETRECIYYNANWELERTNQSGLERCEGEQX$_1$KRRHCFATWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 174),

GAILGRAETRECIYYNANWELERTNQSGLERCEGEQX$_1$KRLHCFATWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 175),

GAILGRAETRECIYYNANWELERTNQSGLERCEGEQX$_1$KRLHCYASWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 176),

GAILGRAETRECIYYNANWELERTNQSGLERCEGEQX$_1$KRLHCYASWRNSSGSIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 177),

GAILGRAETRECIYYNANWELERTNQSGLERCEGEQX$_1$KRLHCYASWRNSSGTIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 178),

GAILGRAETRECIYYNANWELERTNQSGLERCEGEQX$_1$KRLHCYASWRNSSGTIELVKKGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 179),

GAILGRAETRECIYYNANWELERTNQSGLERCEGEQX$_1$KRLHCYASWRNSSGTIELVKKGCWLD DX$_2$X$_3$CYDRQECVX$_4$X$_5$X$_6$X$_7$X$_8$PX$_9$VYFCCCEGNMCNEKFSYFPEMEVTQPTS (SEQ ID NO: 180),

GRGEAETRECIYYNANWELERTNQSGLERCEGEQX$_1$KRRHCFATWKNISGSIEIVKQGCWLDDX $_2$X$_3$CYDRTDCVX$_4$X$_5$X$_6$X$_7$X$_8$PX$_9$VYFCCCEGNMCNEKFSYFPEMEVTQPTS (SEQ ID NO: 181),

GRGEAETRECIYYNANWELERTNQSGLERCEGEQX$_1$KRLHCFATWKNISGSIEIVKQGCWLDDX $_2$X$_3$CYDRTDCVX$_4$X$_5$X$_6$X$_7$X$_8$PX$_9$VYFCCCEGNMCNEKFSYFPEMEVTQPTS (SEQ ID NO: 182),

GRGEAETRECIYYNANWELERTNQSGLERCEGEQX$_1$KRLHCYASWKNISGSIEIVKQGCWLDDX $_2$X$_3$CYDRTDCVX$_4$X$_5$X$_6$X$_7$X$_8$PX$_9$VYFCCCEGNMCNEKFSYFPEMEVTQPTS (SEQ ID NO: 183),

GRGEAETRECIYYNANWELERTNQSGLERCEGEQX$_1$KRLHCYASWRNSSGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 184),

GRGEAETRECIYYNANWELERTNQSGLERCEGEQX$_1$KRLHCYASWRNSSGTIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 185), |

74

| Row | Composition |
|---|---|
|  | GRGEAETRECIYYNANWELERTNQSGLERCEGEQX$_1$KRLHCYASWRNSSGTIELVKKGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 186), and GRGEAETRECIYYNANWELERTNQSGLERCEGEQX$_1$KRLHCYASWRNSSGTIELVKKGCWLDD X$_2$X$_3$CYDRQECVX$_4$X$_5$X$_6$X$_7$X$_8$PX$_9$VYFCCCEGNMCNEKFSYFPEMEVTQPTS (SEQ ID NO: 187), wherein X$_1$ is D or R, X$_2$ is I, F, E, D, Y, S, N, Q, or T, X$_3$ is N or T, X$_4$ is A or E, X$_5$ is T or K, X$_6$ is E or K, X$_7$ is E or D, X$_8$ is N or S, and X$_9$ is Q, E, K, R, D, or N, optionally wherein the chimera is truncated from the N-terminus by deletion of 1, 2, 3, 4, 5, 6, 7, 8, or 9 amino acids, wherein the chimera retains the two amino acids before the first cysteine. |
| 2 | A polypeptide containing an extracellular ActRII chimera, the chimera having a sequence of any one of GAILGRSETQECIYYNANWELERTNQSGLERCEGEQX$_1$KRRHCFATWKNISGSIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 188), GAILGRSETQECIYYNANWELERTNQSGLERCEGEQX$_1$KRLHCFATWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 189), GAILGRSETQECIYYNANWELERTNQSGLERCEGEQX$_1$KRLHCYASWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 190), GAILGRSETQECIYYNANWELERTNQSGLERCEGEQX$_1$KRLHCYASWRNSSGSIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 191), GAILGRSETQECIYYNANWELERTNQSGLERCEGEQX$_1$KRLHCYASWRNSSGTIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 192), GAILGRSETQECIYYNANWELERTNQSGLERCEGEQX$_1$KRLHCYASWRNSSGTIELVKKGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 193), and GAILGRSETQECIYYNANWELERTNQSGLERCEGEQX$_1$KRLHCYASWRNSSGTIELVKKGCWLD DX$_2$X$_3$CYDRQECVX$_4$X$_5$X$_6$X$_7$X$_8$PX$_9$VYFCCCEGNMCNEKFSYFPEMEVTQPTS (SEQ ID NO: 194), in which X$_1$ is D or R, X$_2$ is I, F, E, D, Y, S, N, Q, or T, X$_3$ is N or T, X$_4$ is A or E, X$_5$ is T or K, X$_6$ is E or K, X$_7$ is E or D, X$_8$ is N or S, and X$_9$ is Q, E, K, R, D, or N, optionally wherein the chimera is truncated from the N-terminus by deletion of 1, 2, 3, 4, 5, 6, 7, 8, or 9 amino acids, wherein the chimera retains the two amino acids before the first cysteine. |

EP 4 674 480 A2

| Row | Composition |
|---|---|
| 3 | The polypeptide of row 1, wherein the chimera has the sequence of GAILGRAETRECIYYNANWELERTNQSGLERCEGEQX$_1$KRRHCFATWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 174). |
| 4 | The polypeptide of row 1, wherein the chimera has the sequence of GAILGRAETRECIYYNANWELERTNQSGLERCEGEQX$_1$KRLHCFATWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 175). |
| 5 | The polypeptide of row 1, wherein the chimera has the sequence of GAILGRAETRECIYYNANWELERTNQSGLERCEGEQX$_1$KRLHCYASWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 176). |
| 6 | The polypeptide of row 1, wherein the chimera has the sequence of GAILGRAETRECIYYNANWELERTNQSGLERCEGEQX$_1$KRLHCYASWRNSSGSIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 177). |
| 7 | The polypeptide of row 1, wherein the chimera has the sequence of GAILGRAETRECIYYNANWELERTNQSGLERCEGEQX$_1$KRLHCYASWRNSSGTIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 178). |
| 8 | The polypeptide of row 1, wherein the chimera has the sequence of GAILGRAETRECIYYNANWELERTNQSGLERCEGEQX$_1$KRLHCYASWRNSSGTIELVKKGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 179). |
| 9 | The polypeptide of row 1, wherein the chimera has the sequence of GAILGRAETRECIYYNANWELERTNQSGLERCEGEQX$_1$KRLHCYASWRNSSGTIELVKKGCWLD DX$_2$X$_3$CYDRQECVX$_4$X$_5$X$_6$X$_7$X$_8$PX$_9$VYFCCCEGNMCNEKFSYFPEMEVTQPTS (SEQ ID NO: 180). |
| 10 | The polypeptide of row 1, wherein the chimera has the sequence of GRGEAETRECIYYNANWELERTNQSGLERCEGEQX$_1$KRRHCFATWKNISGSIEIVKQGCWLDDX $_2$X$_3$CYDRTDCVX$_4$X$_5$X$_6$X$_7$X$_8$PX$_9$VYFCCCEGNMCNEKFSYFPEMEVTQPTS (SEQ ID NO: 181). |
| 11 | The polypeptide of row 1, wherein the chimera has the sequence of |

(continued)

| Row | Composition |
|---|---|
| | GRGEAETRECIYYNANWELERTNQSGLERCEGEQX$_1$KRLHCFATWKNISGSIEIVKQGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 182). |
| 12 | The polypeptide of row 1, wherein the chimera has the sequence of GRGEAETRECIYYNANWELERTNQSGLERCEGEQX$_1$KRLHCYASWKNISGSIEIVKQGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 183). |
| 13 | The polypeptide of row 1, wherein the chimera has the sequence of GRGEAETRECIYYNANWELERTNQSGLERCEGEQX$_1$KRLHCYASWRNSSGSIEIVKQGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 184). |
| 14 | The polypeptide of row 1, wherein the chimera has the sequence of GRGEAETRECIYYNANWELERTNQSGLERCEGEQX$_1$KRLHCYASWRNSSGTIEIVKQGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 185). |
| 15 | The polypeptide of row 1, wherein the chimera has the sequence of GRGEAETRECIYYNANWELERTNQSGLERCEGEQX$_1$KRLHCYASWRNSSGTIELVKKGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 186). |
| 16 | The polypeptide of row 1, wherein the chimera has the sequence of GRGEAETRECIYYNANWELERTNQSGLERCEGEQX$_1$KRLHCYASWRNSSGTIELVKKGCWLDDX$_2$X$_3$CYDRQECVX$_4$X$_5$X$_6$X$_7$X$_8$PX$_9$VYFCCCEGNMCNEKFSYFPEMEVTQPTS (SEQ ID NO: 187). |
| 17 | The polypeptide of row 2, wherein the chimera has the sequence of GAILGRSETQECIYYNANWELERTNQSGLERCEGEQX$_1$KRRHCFATWKNISGSIEIVKQGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 188). |
| 18 | The polypeptide of row 2, wherein the chimera has the sequence of GAILGRSETQECIYYNANWELERTNQSGLERCEGEQX$_1$KRLHCFATWKNISGSIEIVKQGCWLDDX$_2$X$_3$CYDRTDCVX$_4$X$_5$X$_6$X$_7$X$_8$PX$_9$VYFCCCEGNMCNEKFSYFPEMEVTQPTS (SEQ ID NO: 189). |
| 19 | The polypeptide of row 2, wherein the chimera has the sequence of |

EP 4 674 480 A2

| Row | Composition |
|---|---|
| | GAILGRSETQECIYYNANWELERTNQSGLERCEGEQX$_1$KRLHCYASWKNISGSIEIVKQGCWLDD X$_2$X$_3$CYDRTDCVX$_4$X$_5$X$_6$X$_7$X$_8$PX$_9$VYFCCCEGNMCNEKFSYFPEMEVTQPTS (SEQ ID NO: 190). |
| 20 | The polypeptide of row 2, wherein the chimera has the sequence of GAILGRSETQECIYYNANWELERTNQSGLERCEGEQX$_1$KRLHCYASWRNSSGSIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 191). |
| 21 | The polypeptide of row 2, wherein the chimera has the sequence of GAILGRSETQECIYYNANWELERTNQSGLERCEGEQX$_1$KRLHCYASWRNSSGTIEIVKQGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 192). |
| 22 | The polypeptide of row 2, wherein the chimera has the sequence of GAILGRSETQECIYYNANWELERTNQSGLERCEGEQX$_1$KRLHCYASWRNSSGTIELVKKGCWLD DX$_2$X$_3$CYDRTDCVX$_4$X$_5$X$_6$X$_7$X$_8$PX$_9$VYFCCCEGNMCNEKFSYFPEMEVTQPTS (SEQ ID NO: 193). |
| 23 | The polypeptide of row 2, wherein the chimera has the sequence of GAILGRSETQECIYYNANWELERTNQSGLERCEGEQX$_1$KRLHCYASWRNSSGTIELVKKGCWLD DX$_2$X$_3$CYDRQECVX$_4$X$_5$X$_6$X$_7$X$_8$PX$_9$VYFCCCEGNMCNEKFSYFPEMEVTQPTS (SEQ ID NO: 194). |
| 24 | The polypeptide of any one of rows 1-23, wherein X$_1$ is D. |
| 25 | The polypeptide of any one of rows 1-23, wherein X$_1$ is R. |
| 26 | The polypeptide of any one of rows 1-25, wherein X$_2$ is I. |
| 27 | The polypeptide of any one of rows 1-25, wherein X$_2$ is F. |
| 28 | The polypeptide of any one of rows 1-25, wherein X$_2$ is E. |
| 29 | The polypeptide of any one of rows 1-25, wherein X$_2$ is D. |
| 30 | The polypeptide of any one of rows 1-25, wherein X$_2$ is Y. |
| 31 | The polypeptide of any one of rows 1-25, wherein X$_2$ is S. |
| 32 | The polypeptide of any one of rows 1-25, wherein X$_2$ is N. |
| 33 | The polypeptide of any one of rows 1-25, wherein X$_2$ is Q. |
| 34 | The polypeptide of any one of rows 1-25, wherein X$_2$ is T. |

(continued)

| Row | Composition |
|---|---|
| 35 | The polypeptide of any one of rows 1-34, wherein $X_3$ is N. |
| 36 | The polypeptide of any one of rows 1-34, wherein $X_3$ is T. |
| 37 | The polypeptide of any one of rows 1-36, wherein $X_4$ is A. |
| 38 | The polypeptide of any one of rows 1-36, wherein $X_4$ is E. |
| 39 | The polypeptide of any one of rows 1-38, wherein $X_5$ is T. |
| 40 | The polypeptide of any one of rows 1-38, wherein $X_5$ is K. |
| 41 | The polypeptide of any one of rows 1-40, wherein $X_6$ is E. |
| 42 | The polypeptide of any one of rows 1-40, wherein $X_6$ is K. |
| 43 | The polypeptide of any one of rows 1-42, wherein $X_7$ is E. |
| 44 | The polypeptide of any one of rows 1-42, wherein $X_7$ is D. |
| 45 | The polypeptide of any one of rows 1-44, wherein $X_8$ is N. |
| 46 | The polypeptide of any one of rows 1-44, wherein $X_8$ is S. |
| 47 | The polypeptide of any one of rows 1-46, wherein $X_9$ is Q. |
| 48 | The polypeptide of any one of rows 1-46, wherein $X_9$ is E. |
| 49 | The polypeptide of any one of rows 1-46, wherein $X_9$ is K. |
| 50 | The polypeptide of any one of rows 1-46, wherein $X_9$ is R. |
| 51 | The polypeptide of any one of rows 1-46, wherein $X_9$ is D. |
| 52 | The polypeptide of any one of rows 1-46, wherein $X_9$ is N. |
| 53 | The polypeptide of any one of rows 1-52, wherein $X_5$ is T, $X_6$ is E, $X_7$ is E, and $X_8$ is N. |
| 54 | The polypeptide of any one of rows 1-52, wherein $X_5$ is T, $X_6$ is K, $X_7$ is E, and $X_8$ is N. |
| 55 | The polypeptide of any one of rows 1-54, wherein $X_2$ is E and $X_3$ is T. |
| 56 | The polypeptide of any one of rows 1-54, wherein $X_2$ is I or F and $X_3$ is N. |
| 57 | The polypeptide of row 56, wherein $X_2$ is I. |
| 58 | The polypeptide of row 56, wherein $X_2$ is F. |
| 59 | The polypeptide of row 1 or row 2, wherein the chimera has the sequence of any one of SEQ ID NOs: 195-216. |
| 60 | The polypeptide of row 59, wherein the chimera has the sequence of SEQ ID NO: 195. |

(continued)

| Row | Composition |
|---|---|
| 61 | The polypeptide of row 59, wherein the chimera has the sequence of SEQ ID NO: 196. |
| 62 | The polypeptide of row 59, wherein the chimera has the sequence of SEQ ID NO: 197. |
| 63 | The polypeptide of row 59, wherein the chimera has the sequence of SEQ ID NO: 198. |
| 64 | The polypeptide of row 59, wherein the chimera has the sequence of SEQ ID NO: 213. |
| 65 | The polypeptide of row 59, wherein the chimera has the sequence of SEQ ID NO: 214. |
| 66 | The polypeptide of row 59, wherein the chimera has the sequence of SEQ ID NO: 201. |
| 67 | The polypeptide of row 59, wherein the chimera has the sequence of SEQ ID NO: 215. |
| 68 | The polypeptide of row 59, wherein the chimera has the sequence of SEQ ID NO: 216. |
| 69 | The polypeptide of row 59, wherein the chimera has the sequence of SEQ ID NO: 210. |
| 70 | The polypeptide of any one of rows 1-69, wherein the chimera is truncated from the N-terminus by deletion of one amino acid. |
| 71 | The polypeptide of any one of rows 1-69, wherein the chimera is truncated from the N-terminus by deletion of two amino acids. |
| 72 | The polypeptide of any one of rows 1-69, wherein the chimera is truncated from the N-terminus by deletion of three amino acids. |
| 73 | The polypeptide of any one of rows 1-69, wherein the chimera is truncated from the N-terminus by deletion of four amino acids. |
| 74 | The polypeptide of any one of rows 1-69, wherein the chimera is truncated from the N-terminus by deletion of five amino acids. |
| 75 | The polypeptide of any one of rows 1-69, wherein the chimera is truncated from the N-terminus by deletion of six amino acids. |
| 76 | The polypeptide of any one of rows 1-69, wherein the chimera is truncated from the N-terminus by deletion of seven amino acids. |
| 77 | The polypeptide of any one of rows 1-69, wherein the chimera is truncated from the N-terminus by deletion of eight amino acids. |
| 78 | The polypeptide of any one of rows 1-69, wherein the chimera is truncated from the N-terminus by deletion of nine amino acids. |
| 79 | The polypeptide of any one of rows 1, 2, and 70-78, wherein the chimera has the sequence of any one of SEQ ID NOs: 221-293. |
| 80 | The polypeptide of row 79, wherein the chimera has the sequence of SEQ ID NO: 221. |
| 81 | The polypeptide of row 79, wherein the chimera has the sequence of SEQ ID NO: 226. |
| 82 | The polypeptide of row 79, wherein the chimera has the sequence of SEQ ID NO: 227. |
| 83 | The polypeptide of row 79, wherein the chimera has the sequence of SEQ ID NO: 228. |
| 84 | The polypeptide of row 79, wherein the chimera has the sequence of SEQ ID NO: 229. |
| 85 | The polypeptide of row 79, wherein the chimera has the sequence of SEQ ID NO: 230. |
| 86 | The polypeptide of row 79, wherein the chimera has the sequence of SEQ ID NO: 231. |

| Row | Composition |
| --- | --- |
| 87 | The polypeptide of row 79, wherein the chimera has the sequence of SEQ ID NO: 232. |
| 88 | The polypeptide of row 79, wherein the chimera has the sequence of SEQ ID NO: 236. |
| 89 | The polypeptide of row 79, wherein the chimera has the sequence of SEQ ID NO: 240. |
| 90 | The polypeptide of row 79, wherein the chimera has the sequence of SEQ ID NO: 252. |
| 91 | The polypeptide of row 79, wherein the chimera has the sequence of SEQ ID NO: 253. |
| 92 | The polypeptide of row 79, wherein the chimera has the sequence of SEQ ID NO: 256. |
| 93 | The polypeptide of row 79, wherein the chimera has the sequence of SEQ ID NO: 257. |
| 94 | The polypeptide of row 79, wherein the chimera has the sequence of SEQ ID NO: 260. |
| 95 | The polypeptide of row 79, wherein the chimera has the sequence of SEQ ID NO: 261. |
| 96 | The polypeptide of row 79, wherein the chimera has the sequence of SEQ ID NO: 262. |
| 97 | The polypeptide of row 79, wherein the chimera has the sequence of SEQ ID NO: 263. |
| 98 | The polypeptide of row 79, wherein the chimera has the sequence of SEQ ID NO: 264. |
| 99 | The polypeptide of row 79, wherein the chimera has the sequence of SEQ ID NO: 265. |
| 100 | The polypeptide of row 79, wherein the chimera has the sequence of SEQ ID NO: 266. |
| 101 | The polypeptide of row 79, wherein the chimera has the sequence of SEQ ID NO: 268. |
| 102 | The polypeptide of row 79, wherein the chimera has the sequence of SEQ ID NO: 269. |
| 103 | The polypeptide of row 79, wherein the chimera has the sequence of SEQ ID NO: 272. |
| 104 | The polypeptide of row 79, wherein the chimera has the sequence of SEQ ID NO: 273. |
| 105 | The polypeptide of row 79, wherein the chimera has the sequence of SEQ ID NO: 274. |
| 106 | The polypeptide of any one of rows 1-105, wherein the polypeptide (e.g., the chimera) further includes a C-terminal extension of one or more amino acids (e.g., 1, 2, 3, 4, 5, 6, or more amino acids from wild-type extracellular ActRIIA or ActRIIB). |
| 107 | The polypeptide of row 106, wherein the C-terminal extension is NP. |
| 108 | The polypeptide of row 106, wherein the C-terminal extension is NPVTPK (SEQ ID NO: 154). |
| 109 | The polypeptide of any one of rows 1-108, wherein the polypeptide further includes an Fc domain monomer fused to the C-terminus of the polypeptide (e.g., the C-terminus of the chimera) by way of a linker. |
| 110 | The polypeptide of row 109, wherein the Fc domain monomer has the sequence of SEQ ID NO: 97. |
| 111 | The polypeptide of row 109 or row 110, wherein the polypeptide forms a dimer. |

(continued)

| Row | Composition |
|---|---|
| 112 | The polypeptide of any one of rows 1-108, wherein the polypeptide further includes a wild-type Fc domain fused to the C-terminus of the polypeptide (e.g., the C-terminus of the chimera) by way of a linker. |
| 113 | The polypeptide of row 112, wherein the wild-type Fc domain has the sequence of SEQ ID NO: 150 or SEQ ID NO: 155. |
| 114 | The polypeptide of row 112 or row 113, wherein the polypeptide has the sequence of any one of SEQ ID NOs: 217-220 and SEQ ID NOs: 294-373. |
| 115 | The polypeptide of row 114, wherein the polypeptide has the sequence of SEQ ID NO: 217. |
| 116 | The polypeptide of row 114, wherein the polypeptide has the sequence of SEQ ID NO: 218. |
| 117 | The polypeptide of row 114, wherein the polypeptide has the sequence of SEQ ID NO: 219. |
| 118 | The polypeptide of row 114, wherein the polypeptide has the sequence of SEQ ID NO: 220. |
| 119 | The polypeptide of row 114, wherein the polypeptide has the sequence of SEQ ID NO: 294. |
| 120 | The polypeptide of row 114, wherein the polypeptide has the sequence of SEQ ID NO: 299. |
| 121 | The polypeptide of row 114, wherein the polypeptide has the sequence of SEQ ID NO: 300. |
| 122 | The polypeptide of row 114, wherein the polypeptide has the sequence of SEQ ID NO: 301. |
| 123 | The polypeptide of row 114, wherein the polypeptide has the sequence of SEQ ID NO: 302. |
| 124 | The polypeptide of row 114, wherein the polypeptide has the sequence of SEQ ID NO: 303. |
| 125 | The polypeptide of row 114, wherein the polypeptide has the sequence of SEQ ID NO: 304. |
| 126 | The polypeptide of row 114, wherein the polypeptide has the sequence of SEQ ID NO: 305. |
| 127 | The polypeptide of row 114, wherein the polypeptide has the sequence of SEQ ID NO: 309. |
| 128 | The polypeptide of row 114, wherein the polypeptide has the sequence of SEQ ID NO: 310. |
| 129 | The polypeptide of row 114, wherein the polypeptide has the sequence of SEQ ID NO: 323. |
| 130 | The polypeptide of row 114, wherein the polypeptide has the sequence of SEQ ID NO: 324. |
| 131 | The polypeptide of row 114, wherein the polypeptide has the sequence of SEQ ID NO: 325. |
| 132 | The polypeptide of row 114, wherein the polypeptide has the sequence of SEQ ID NO: 326. |
| 133 | The polypeptide of row 114, wherein the polypeptide has the sequence of SEQ ID NO: 327. |
| 134 | The polypeptide of row 114, wherein the polypeptide has the sequence of SEQ ID NO: 328. |
| 135 | The polypeptide of row 114, wherein the polypeptide has the sequence of SEQ ID NO: 331. |
| 136 | The polypeptide of row 114, wherein the polypeptide has the sequence of SEQ ID NO: 332. |
| 137 | The polypeptide of row 114, wherein the polypeptide has the sequence of SEQ ID NO: 335. |

| Row | Composition |
|---|---|
| 138 | The polypeptide of row 114, wherein the polypeptide has the sequence of SEQ ID NO: 336. |
| 139 | The polypeptide of row 114, wherein the polypeptide has the sequence of SEQ ID NO: 339. |
| 140 | The polypeptide of row 114, wherein the polypeptide has the sequence of SEQ ID NO: 340. |
| 141 | The polypeptide of row 114, wherein the polypeptide has the sequence of SEQ ID NO: 341. |
| 142 | The polypeptide of row 114, wherein the polypeptide has the sequence of SEQ ID NO: 342. |
| 143 | The polypeptide of row 114, wherein the polypeptide has the sequence of SEQ ID NO: 343. |
| 144 | The polypeptide of row 114, wherein the polypeptide has the sequence of SEQ ID NO: 344. |
| 145 | The polypeptide of row 114, wherein the polypeptide has the sequence of SEQ ID NO: 345. |
| 146 | The polypeptide of row 114, wherein the polypeptide has the sequence of SEQ ID NO: 346. |
| 147 | The polypeptide of row 114, wherein the polypeptide has the sequence of SEQ ID NO: 347. |
| 148 | The polypeptide of row 114, wherein the polypeptide has the sequence of SEQ ID NO: 348. |
| 149 | The polypeptide of any one of rows 1-108, wherein the polypeptide further includes an Fc domain containing an amino acid substitution fused to the C-terminus of the polypeptide (e.g., the C-terminus of the chimera) by way of a linker. |
| 150 | The polypeptide of row 149, wherein the Fc domain does not form a dimer. |
| 151 | The polypeptide of any one of rows 1-108, wherein the polypeptide further includes an albumin-binding peptide fused to the C-terminus of the polypeptide (e.g., the C-terminus of the chimera) by way of a linker. |
| 152 | The polypeptide of row 151, wherein the albumin-binding peptide has the sequence of SEQ ID NO: 151. |
| 153 | The polypeptide of any one of rows 1-108, wherein the polypeptide further includes a fibronectin domain fused to the C-terminus of the polypeptide (e.g., the C-terminus of the chimera) by way of a linker. |
| 154 | The polypeptide of row 153, wherein the fibronectin domain has the sequence of SEQ ID NO: 152. |
| 155 | The polypeptide of any one of rows 1-108, wherein the polypeptide further includes a human serum albumin fused to the C-terminus of the polypeptide (e.g., the C-terminus of the chimera) by way of a linker. |
| 156 | The polypeptide of row 155, wherein the human serum albumin has the sequence of SEQ ID NO: 153. |
| 157 | The polypeptide of any one of rows 109-156, wherein the linker is an amino acid spacer. |
| 158 | The polypeptide of row 157, wherein the amino acid spacer is GGG, GGGA (SEQ ID NO: 98), GGGG (SEQ ID NO: 100), GGGAG (SEQ ID NO: 130), GGGAGG (SEQ ID NO: 131), or GGGAGGG (SEQ ID NO: 132). |
| 159 | The polypeptide of row 157, wherein the amino acid spacer is GGS, GGGS (SEQ ID NO: 99), GGGGS (SEQ ID NO: 102), GGSG (SEQ ID NO: 105), or SGGG (SEQ ID NO: 107). |

| Row | Composition |
|---|---|
| 160 | The polypeptide of row 157, wherein the amino acid spacer is GA, GS, GG, GGA, GGS, GGGS (SEQ ID NO: 99), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), SGGG (SEQ ID NO: 107), GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), and GSGSGSGSGSGS (SEQ ID NO: 117), GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), GGSGGSGGSGGS (SEQ ID NO: 123), GGAGGGAG (SEQ ID NO: 124), GGSGGGSG (SEQ ID NO: 125), GGAGGGAGGGAG (SEQ ID NO: 126), and GGSGGGSGGGSG (SEQ ID NO: 127), GGGGAGGGGAGGGGA (SEQ ID NO: 128), GGGGSGGGGSGGGGS (SEQ ID NO: 129), AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACKIPNDLKQKVMNH (SEQ ID NO: 143), GGSAGGSGSGSSGGSSGASGTGTAGGTGSGSGTGSG (SEQ ID NO: 144), AAANSSIDLISVPVDSR (SEQ ID NO: 145), GGSGGGSEGGGSEGGGSEGGGSEGGGSGGGS (SEQ ID NO: 146), EAAAK (SEQ ID NO: 147), or PAPAP(SEQ ID NO: 148). |
| 161 | The polypeptide of any one of rows 1-160, wherein the polypeptide (e.g., an ActRII chimera-Fc fusion protein) has a serum half-life of at least seven days. |
| 162 | The polypeptide of any one of rows 1-161, wherein the polypeptide has increased binding to one or more an ActRII ligands (e.g., activin A, activin B, myostatin, and/or GDF-11) compared to wild-type ActRIIA and/or wild-type ActRIIB (e.g., wild-type extracellular ActRIIA and/or ActRIIB). |
| 163 | The polypeptide of any one of rows 1-162, wherein the polypeptide has decreased binding to bone morphogenetic protein 9 (BMP9, e.g., human BMP9) compared to wild-type ActRIIB (e.g., wild-type extracellular ActRIIB). |
| 164 | The polypeptide of any one of rows 1-163, wherein the polypeptide binds to activin A, activin B, and/or myostatin and has reduced or weak binding to human BMP9 (e.g., compared to wild-type extracellular ActRIIB). |
| 165 | The polypeptide of any one of rows 1-164, wherein the polypeptide does not substantially bind to human BMP9. |
| 166 | The polypeptide of any one of rows 1-165, wherein the polypeptide binds to human activin A with a $K_D$ of 800 pM or less. |
| 167 | The polypeptide of any one of rows 1-166, wherein the polypeptide binds to human activin B with a $K_D$ of 800 pM or less. |
| 168 | The polypeptide of any one of rows 1-167, wherein the polypeptide binds to human GDF-11 with a $K_D$ of 5 pM or higher. |
| 169 | A nucleic acid molecule encoding the polypeptide of any one of rows 1-168. |
| 170 | A vector comprising the nucleic acid molecule of row 169. |
| 171 | A host cell that expresses the polypeptide of any one of rows 1-168, wherein the host cell comprises the nucleic acid molecule of row 169 or the vector of row 170, wherein the nucleic acid molecule or vector is expressed in the host cell. |
| 172 | A pharmaceutical composition comprising the polypeptide of any one of rows 1-168, the nucleic acid molecule of row 169, or the vector of row 170 and one or more pharmaceutically acceptable carriers or excipients. |
| 173 | The pharmaceutical composition of row 172, wherein the polypeptide, nucleic acid molecule, or vector is in a therapeutically effective amount. |

EP 4 674 480 A2

| Row | Composition |
|---|---|
| 174 | A construct comprising two identical polypeptides (e.g., a homodimer), each comprising an extracellular ActRII chimera of any one of rows 1-108 (e.g., an ActRII chimera having a sequence of any one of SEQ ID NOs: 174-216 and 221-293) fused (e.g., linked using an amino acid spacer) to the N- or C-terminus of an Fc domain monomer (e.g., the sequence of SEQ ID NO: 97). The two Fc domain monomers in the two polypeptides interact to form an Fc domain in the construct. |
| 175 | A construct comprising two different polypeptides (e.g., a heterodimer), each comprising an extracellular ActRII chimera of any one of rows 1-108 (e.g., an ActRII chimera having a sequence of any one of SEQ ID NOs: 174-216 and 221-293) fused (e.g., linked using an amino acid spacer) to the N- or C-terminus of an Fc domain monomer (e.g., the sequence of SEQ ID NO: 97). The two Fc domain monomers in the two polypeptides interact to form an Fc domain in the construct. |

**EXAMPLES**

The following examples are provided to further illustrate some embodiments of the present invention, but are not intended to limit the scope of the invention; it will be understood by their exemplary nature that other procedures, methodologies, or techniques known to those skilled in the art may alternatively be used.

**Example 1** - **Effect of ActRIIA/B-Fc on erythropoietin and erythropoietin receptor levels**

*Assessment of serum erythropoietin levels*

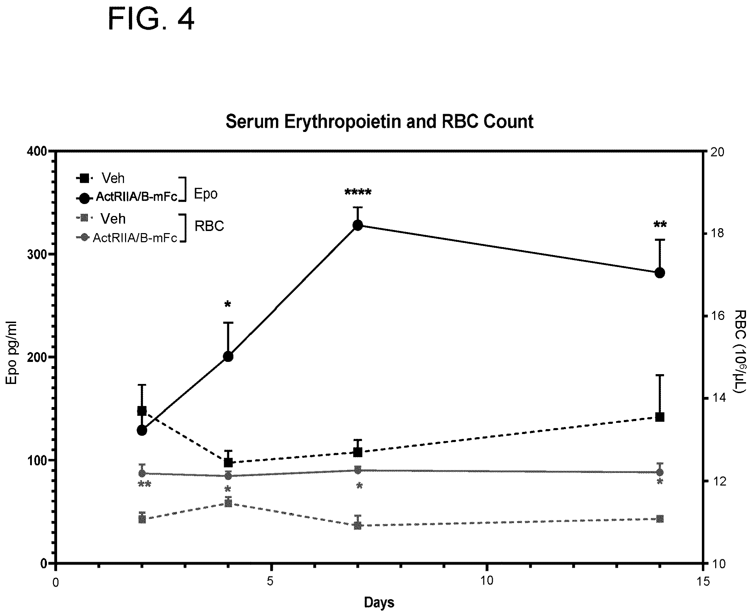

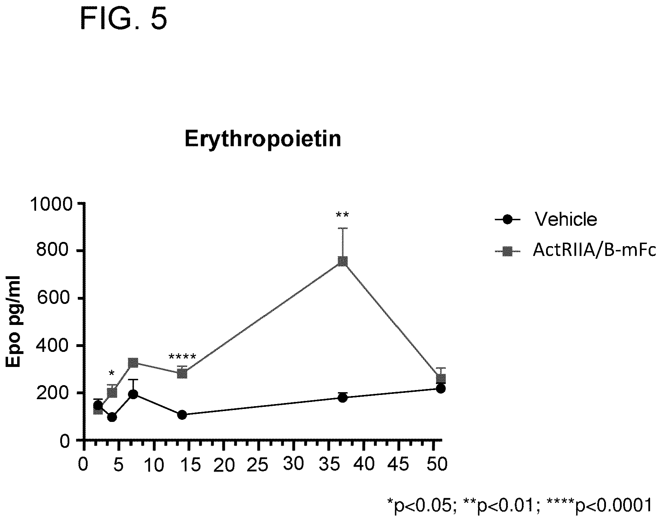

Eleven-week-old male mice (received from Taconic Biosciences) were treated with a single dose of 10 mg/kg ActRIIA/B-mFc (SEQ ID NO: 69 linked to a mouse Fc domain) by intraperitoneal (IP) injection or with vehicle. Mice were sacrificed on days 2, 4, 7, 14, 37, and 51 after treatment. Blood was sampled into EDTA tubes from a submandibular/cheek bleed and stored at 4 °C until analysis at IDEXX BioAnalytics for RBC counts. At necropsy, whole blood was drawn by cardiac bleed and collected into Microvette tubes (Sarstedt). The tubes were incubated at room temperature for two hours followed by centrifugation at 6000 RPM for 10 minutes. Serum was collected and stored at -80 $^0$C until the analysis.
Erythropoietin (EPO) levels were measured using a mouse erythropoietin immunoassay (Quantikine ELISA kit) according to manufacturer instructions. Colorimetric readings of the ELISA plate were measured using a SpectraMax M5 microplate reader at 450 nm and background was subtracted from a reading at 540 nm. Erythropoietin concentration was transformed by creating a 4-parameter logistic curve using GraphPad Prism software. As shown in **FIG. 4,** EPO levels were increased in response to ActRIIA/B-mFc treatment, even in the context of increased RBCs. $*p<0.05$, $**p<0.01$, $****p<0.0001$ between ActRIIA/B-mFc-treated and vehicle at each time point with 2-way ANOVA followed by a Sidak post test. Data are shown as the mean $\pm$ SEM. **FIG. 5** shows that EPO levels were elevated in mice treated with ActRIIA/B-mFc by day 4 through day 37 compared to vehicle-treated mice. $*p\leq0.05$; $**p\leq0.01$; $p****\leq0.0001$ between ActRIIA/B-mFc-treated and vehicle-treated at each time point by t-test. Data are shown as the mean $\pm$ SEM.

*Assessment of erythropoietin receptor expression*

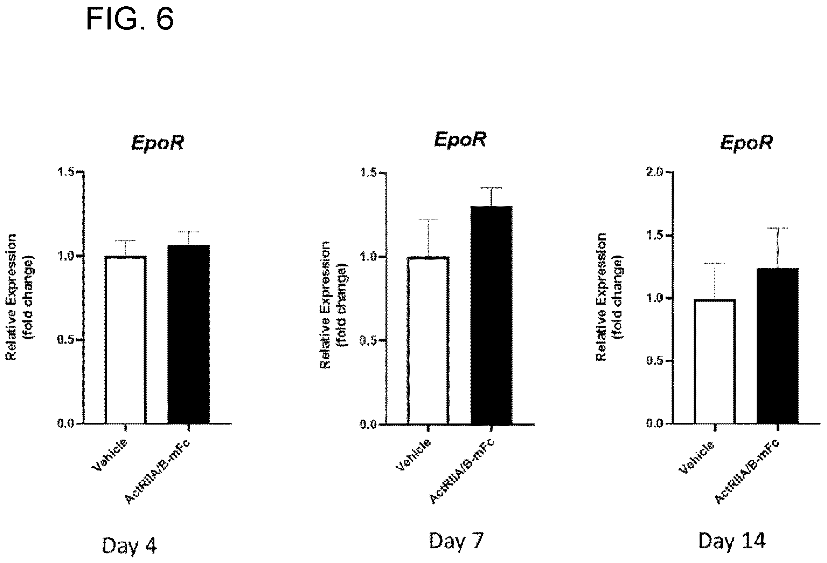

Eleven-week-old male mice (received from Taconic Biosciences) were treated with a single dose of 10mg/kg ActRIIA/B-mFc by IP injection or with vehicle. Mice were sacrificed on days 4, 7 and 14 after treatment. Bone marrow cells were isolated by flushing the bone marrow from a femur. Red blood cells were removed via lysis using an ammonium chloride solution (Stem Cell Technologies). One million cells were lysed with Trizol reagent and RNA isolated using the Direct-zol RNA MicroPrep (Zymo Research) kit. cDNA was synthesized using Reverse Transcription Kit (Qiagen) and the *EpoR* expression measured by qPCR using the ViiA 7 real-time PCR system (Applied Biosystems). Primers used: EpoR Forward: TTCAGCGGATTCTGGAGTGCCT (SEQ ID NO: 376) and EpoR Reverse: AGCAACAGCGAGATGAGGACCA (SEQ ID NO: 377). β-actin was used as a housekeeping gene. Primers used: β-actin Forward: CATCGTGGGCCGCCCTA (SEQ ID NO: 378) and β-actin Reverse: CACCCACATAGGAGTCCTTCTG (SEQ ID NO: 379). Relative gene expression was determined using the $\Delta\Delta$CT method and graphs were plotted in GraphPad Prism. Error bars represent SEM. Statistical analysis was done using Student's t-test. As shown in **FIG. 6,** EPO receptor expression was increased after ActRIIA/B-mFc treatment.

**Example 2** - **Treatment of end-stage renal disease by administration of an extracellular ActRII variant**

According to the methods disclosed herein, a physician of skill in the art can treat a subject, such as a human patient, having end-stage renal disease so as to increase EPO levels. To treat the subject, a physician of skill in the art can administer to the subject a composition containing a polypeptide including an extracellular ActRII variant (e.g., a polypeptide containing an extracellular ActRIIA variant having the sequence of any one of SEQ ID NOs: 1-72 (e.g., SEQ ID NOs: 6-72), a polypeptide containing an extracellular ActRIIB variant having the sequence of any one of SEQ ID NOs: 157-171 (e.g., SEQ ID NOs: 158-171), or a polypeptide containing an extracellular ActRII chimera having the sequence of any one of SEQ ID NOs: 174-216 (e.g., SEQ ID NOs: 195-216), such as a polypeptide containing an extracellular ActRIIa variant, an extracellular ActRIIB variant, or an extracellular ActRII chimera linked to an Fc domain). The composition containing the extracellular ActRII variant may be administered to the subject, for example, by parenteral injection (e.g., intravenous or subcutaneous injection) to treat end-stage renal disease. The polypeptide containing an extracellular ActRII variant (e.g., an extracellular ActRIIA variant having the sequence of any one of SEQ ID NOs: 1-72 (e.g., SEQ ID NOs: 6-72), an extracellular ActRIIB variant having the sequence of any one of SEQ ID NOs: 157-171 (e.g., SEQ ID NOs: 158-171), or an extracellular ActRII chimera having the sequence of any one of SEQ ID NOs: 174-216 (e.g., SEQ ID NOs: 195-216)) is administered in a therapeutically effective amount, such as from 0.01 to 500 mg/kg (e.g., 0.01, 0.1, 0.2, 0.3, 0.325, 0.35, 0.375, 0.4, 0.5, 0.75, 1, 1.25, 1.5, 1.75, 2, 2.25, 2.5, 2.75, 3, 3.25, 3.5, 3.75, 4, 4.25, 4.5, 4.75, 5, 10, 15, 20, 25, 30, 35, 40, 45, 50, 100, 150, 200, 250, 300, 350, 400, 450, or 500 mg/kg). In some embodiments, the extracellular ActRII variant is administered once every sixteen weeks, quarterly, once every twelve weeks, bimonthly, once every eight weeks, once a month, once every four weeks, once every two weeks, or at least once a week or more (e.g., 1, 2, 3, 4, 5, 6, or 7 times a week or more). The extracellular ActRII variant is administered in an amount sufficient to increase EPO levels, increase EPO receptor levels, and/or slow progression of the disease.

Following administration of the composition to a patient, a practitioner of skill in the art can monitor the patient's improvement in response to the therapy by a variety of methods. For example, a physician can monitor the patient's EPO levels using a blood test and can measure kidney function using blood tests, urine tests, and imaging tests. A finding that the patient's EPO levels are increased or that the disease is progressing more slowly following administration of the composition compared to test results prior to administration of the composition indicates that the patient is responding favorably to the treatment. Subsequent doses can be determined and administered as needed.

## Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure come within known or customary practice within the art to which the invention pertains and may be applied to the essential features hereinbefore set forth.

All publications, patents, and patent applications are herein incorporated by reference in their entirety to the same extent as if each individual publication, patent or patent application was specifically and individually indicated to be incorporated by reference in its entirety.

Other embodiments are within the following claims.

The invention further provides the following non-limiting numbered embodiments.

1. A method of treating a subject having a disease or condition that can be treated with erythropoietin or an erythropoiesis-stimulating agent, comprising administering to the subject a therapeutically effective amount of a composition of Table 10, a composition of Table 11, or a composition of Table 12.

2. A method of increasing erythropoietin levels in a subject in need thereof, comprising administering to the subject a therapeutically effective amount of a composition of Table 10, a composition of Table 11, or a composition of Table 12.

3. A method of increasing erythropoietin receptor levels in a subject in need thereof, comprising administering to the subject a therapeutically effective amount of a composition of Table 10, a composition of Table 11, or a composition of Table 12.

4. The method of any one of embodiments 1-3, wherein the subject has or is at risk of developing anemia due to dialysis or anemia of prematurity.

5. The method of any one of embodiments 1-3, wherein the subject has or is at risk of developing of end-stage renal disease, renal insufficiency, polycythemia, hemochromatosis, a disease or condition associated with dysfunction of endothelial progenitor cells, a disease or condition having an autoimmune or inflammatory component, a neurological disorder or inflammatory brain disease, gastrointestinal dysmotility, a disease of the endocrine system, a disease of the reproductive system, aging, pregnancy, a menstrual disorder, ischemia or an ischemic disorder or condition, hypoxia or a hypoxic disorder or condition, an ulcer, a burn, a wound, ischemia-reperfusion injury, asthma, hypertension, a viral disease or infection, a systemic microbial infection, a gastrointestinal disease, arterial sclerosis, cancer, psychosis, a genetic disease, an inflammatory disease, graft-versus-host disease, cardiovascular disease, an allergy, or arthritis.

6. The method of embodiment 5, wherein the ischemia is central nervous system ischemia, liver ischemia, renal ischemia, or cardiac ischemia.

7. The method of embodiment 5, wherein the ischemic disorder or condition is occlusive arterial disease, chronic venous insufficiency, circulatory shock, pulmonary embolism, myocardial infarction, ischemic stroke, acute respiratory failure, chronic heart failure, atherosclerosis, cardiac cirrhosis, macular degeneration, sleep apnea, Raynaud's disease, systemic sclerosis, nonbacterial thrombotic endocarditis, a transient ischemic attack, or ischemia resulting from general anesthesia.

8. The method of embodiment 5, wherein the hypoxic disorder or condition is a pulmonary disorder, severe pneumonia, pulmonary edema, hyaline membrane disease, liver disease, renal disease, cancer, or altitude sickness.

9. The method of embodiment 5, wherein the viral disease or infection is a Hepatitis C virus infection or an HIV infection.

10. The method of embodiment 5, wherein the disease or condition associated with dysfunction of endothelial progenitor cells is heart failure, angina pectoris, endotheliosis, reticuloendotheliosis, age-related cardiovascular disorder, coronary heart disease, atherosclerosis, myocardial ischemia, hypercholesterolemia, an ischemic disorder of the extremities, Raynaud's disease, preeclampsia, pregnancy induced hypertension, an endothelium-mediated chronic inflammatory disorder, wound healing, chronic renal failure, or acute renal failure.

11. The method of embodiment 10, wherein the disease or condition associated with dysfunction of endothelial progenitor cells is chronic renal failure.

12. The method of embodiment 5, wherein the autoimmune or inflammatory disease or condition is acute cerebrovascular injury, acute brain injury, acute cardiovascular injury, arthritis, an autoimmune disease, a stroke, a neurological injury, or immune-mediated inflammation.

13. The method of embodiment 5, wherein the neurological disorder or inflammatory brain disease is a demyelinating disease, epilepsy, spinal cord injury, a complication following traumatic brain injury,
a chronic inflammatory brain disease, or a neurological disorder associated with a surgery.

14. The method of embodiment 13, wherein the chronic inflammatory brain disease is a neurodegenerative disease.

15. The method of embodiment 14, wherein the neurodegenerative disease is Alzheimer's disease, Parkinson's disease, Huntington's disease, amyotrophic lateral sclerosis (ALS), or age-related macular degeneration (AMD).

16. The method of embodiment 13, wherein the demyelinating disease is multiple sclerosis, neuromyelitis optica, acute disseminated encephalomyelitis, or transverse myelitis.

17. The method of embodiment 5, wherein the gastrointestinal dysmotility is associated with an intestinal injury, abdominal trauma, an intestinal inflammatory condition, an intestinal infection, slow transit constipation, post-operative ileus, a neurodegenerative injury, a neurotraumatic injury, a congenital problem, or a malnutrition-malabsorption problem.

18. The method of embodiment 17, wherein the intestinal infection is a bacterial infection, peritonitis, or ascites.

19. The method of embodiment 17, wherein the intestinal inflammatory condition is inflammatory bowel disease, Crohn's disease, or ulcerative colitis.

20. The method of embodiment 17, wherein the slow transit constipation is chronic constipation, idiopathic constipation, constipation due to post-operative ileus, or constipation caused by opiate use.

21. The method of embodiment 17, wherein the congenital problem is gastroschisis, omphalocele, aganglionic megacolon, Hirschsprung's disease, chronic intestinal pseudo-obstruction, small left colon syndrome, anorectal anomalies, esophageal dysplasia and atresia, ectopic anus, congenital hernias, or internal anal sphincter achalasia.

22. The method of embodiment 17, wherein the malnutrition-malabsorption problem is associated with an intestinal injury, an abdominal trauma, an intestinal inflammatory condition, an intestinal infection, constipation, post-operative ileus, a neurodegenerative injury, a neurotraumatic injury, a congenital problem, Gaucher disease, refeeding syndrome, extremely low birth weight, cancer cachexia, infection, cancer, spinal cord dysfunction, spinal dysraphism, bifida, a tumor, central nervous system dysfunction, peripheral neuropathy, removal part of the gastrointestinal tract, hemorrhage, liver dysfunction, celiac disease, cystic fibrosis, muscular dystrophies, or cerebral palsy.

23. The method of embodiment 5, wherein the subject has or is at risk of developing of end-stage renal disease.

24. The method of embodiment 5, wherein the subject has or is at risk of developing polycythemia.

25. The method of any one of embodiments 1-24, wherein the composition of Table 10, Table 11, or Table 12 is administered to the subject prior to surgery, after stem cell transplantation, prior to or during a space flight, during or after tissue or organ transplantation, to promote the growth of new blood vessels, for granulation tissue formation, for trauma treatment, or for post-vascular graft treatment.

26. The method of any one of embodiments 1-25, wherein the subject is receiving kidney dialysis.

27. The method of any one of embodiments 1-3 and 5-26, wherein the subject does not have anemia.

28. The method of any one of embodiments 1-3 and 5-27, wherein the subject has normal hematopoiesis.

29. The method of any one of embodiments 1-28, wherein the subject has low serum erythropoietin.

30. A method of preparing a tissue or organ for transplantation, comprising contacting the tissue or organ with a therapeutically effective amount of a composition of Table 10, a composition of Table 11, or a composition of Table 12.

31. The method of any one of embodiments 1-30, wherein the method comprises administering to the subject a therapeutically effective amount of a composition of Table 10.

32. The method of any one of embodiments 1-30, wherein the method comprises administering to the subject a therapeutically effective amount of a composition of Table 11.

33. The method of any one of embodiments 1-30, wherein the method comprises administering to the subject a therapeutically effective amount of a composition of Table 12.

34. The method of any one of embodiments 1-33, wherein the composition is administered in an amount sufficient to increase EPO levels, increase EPO receptor levels, promote the growth of new blood vessels and/or the replacement of damaged vascular regions, promote granulation tissue formation, reduce infiltration of mononuclear cells into the brain of the subject, improve a neurological deficit, reduce axonal damage, reduce neuronal cell death, reduce glial cell death, affect myostatin, activin A, activin B, and/or BMP9 signaling in the subject, or reduce or inhibit the binding of activin A, activin B, and/or myostatin to their receptors.

## Claims

1. A polypeptide comprising an extracellular activin receptor type IIA (ActRIIA) variant for use in a method of treating and/or preventing cancer, an inflammatory disease, a disease or condition having an autoimmune or inflammatory component, a disease or condition associated with dysfunction of endothelial progenitor cells, or a genetic disease in a subject by increasing erythropoietin levels or erythropoietin receptor levels in the subject, wherein the method comprises administering to the subject a therapeutically effective amount of the polypeptide, wherein the variant has the sequence of SEQ ID NO: 69.

2. The polypeptide for use according to claim 1, wherein the polypeptide is administered to the subject prior to surgery, after stem cell transplantation, during or after tissue or organ transplantation, to promote the growth of new blood vessels, for granulation tissue formation, for trauma treatment, or for post-vascular graft treatment.

3. The polypeptide for use according to claim 1 or claim 2, wherein (i) the subject does not have anemia and/or (ii) the subject has normal hematopoiesis.

4. The polypeptide for use according to any one of claims 1-3, wherein the subject has low serum erythropoietin.

5. The polypeptide for use according to any one of claims 1-4, wherein the polypeptide further comprises an Fc domain monomer fused to the C-terminus of the polypeptide by way of a linker.

6. The polypeptide for use according to claim 5, wherein the linker is a peptide linker containing flexible amino acid residues.

7. The polypeptide for use according to claim 5 or claim 6, wherein the linker is an amino acid spacer, optionally wherein the amino acid spacer is GGG.

8. The polypeptide for use according to any one of claims 5-7, wherein the Fc domain monomer is an IgG1, IgG2a, IgG2b, IgG3 or IgG4 Fc domain monomer.

9. The polypeptide for use according to claim 8, wherein the Fc domain monomer is an IgG1 Fc domain monomer.

10. The polypeptide for use according to claim 8 or claim 9, wherein the Fc domain monomer comprises the amino acid sequence of SEQ ID NO: 155.

11. The polypeptide for use according to any one of claims 1-10, wherein the polypeptide comprises the amino acid sequence of SEQ ID NO: 156.

EP 4 674 480 A2

# FIG. 1

| Construct | | | SEQ ID NO. |
|---|---|---|---|
| ActRIIA | | C LFFNANWEKD RTNQTGVEPC YGDKDKRRHC FATWKNISGS IEIVKQGCWL DDINCYDRTD CVEKKDSPEV YFCCCEGNMC | 73 (portion) |
| ActRIIB | | C IYYNANWELE RTNQSGLERC EGEQDKRLHC YASWRNSSGT IELVKKGCWL DDFNCYDRQE CVATEENPQV YFCCCEGNFC | 74 (portion) |
| ActRIIA/B | GA | ILGRSETQEC LFYNANWELE RTNQTGVERC EGEKDKRLHC YATWRNISGS IEIVKKGCWL DDFNCYDRTD CVETEENPQV YFCCCEGNMC NEKFSYFPEM EVTQPTS | 69 |
| ActRIIA/Bdelta9 | GA | ILGRSETQEC LFYNANWELD RTNQTGVERC EGEKDKRLHC YATWRNISGS IEIVKKGCWL DDFNCYDRTD CVETKENPQV YFCCCEGNMC NEKFSYFPEM EVTQPTS | 58 |
| ActRIIA/Bdelta9min | GA | ILGRSETQEC LFYNANWELD RTNQTGVEPC EGEKDKRLHC FATWKNISGS IEIVKKGCWL DDINCYDRTD CVETKENPQV YFCCCEGNMC NEKFSYFPEM EVTQPTS | 6 |
| ActRIIA/B+ | GA | ILGRSETQEC LFYNANWELE RTNQTGVERC EGEKDKRLHC YATWRNISGS IEIVAKGCWL DDFNCYDRTD CVETEENPQV YFCCCEGNMC NEKFSYFPEM EVTQPTS | 150 |
| ActRIIA/b-delta-9m2[2] | GA | ILGRSETQEC LFYNANWELD RTNQTGVERC EGEKDKRLHC YATWRNISGS IEIVKKGCWL DDINCYDRTD CVETKENPQV YFCCCEGNMC NEKFSYFPEM EVTQPTS | 38 |
| ActRIIa/b-delta/9m3 | GA | ILGRSETQEC LFYNANWELD RTNQTGVERC EGEKDKRLHC FATWRNISGS IEIVKKGCWL DDFNCYDRTD CVETKENPQV YFCCCEGNMC NEKFSYFPEM EVTQPTS | 41 |
| ActRIIa/b-delta-9m4[2] | GA | ILGRSETQEC LFYNANWELD RTNQTGVEPC EGEKDKRLHC YATWRNISGS IEIVKKGCWL DDFNCYDRTD CVETKENPQV YFCCCEGNMC NEKFSYFPEM EVTQPTS | 44 |
| ActRIIa/bmax1 | GA | ILGRSETQEC LYYNANWELE RTNQTGVERC EGEQDKRLHC YATWRNISGS IEIVKKGCWL DDFNCYDRTD CVETEENPQV YFCCCEGNMC NEKFSYFPEM EVTQPTS | 70 |
| ActRIIa/bmax2 | GA | ILGRSETQEC LFYNANWELE RTNQTGVERC EGEKDKRLHC YATWRNISGS IEIVKKGCWL DDFNCYDRTD CVETEENPQV YFCCCEGNMC NEKFSYFPEM EVTQPTSNP | 71 |
| ActRIIa/bmax3 | GA | ILGRSETQEC LFYNANWELE RTNQTGVERC EGEKDKRLHC YATWRNISGS IEIVKKGCWL DDFNCYDRTD CVETEENPQV YFCCCEGNMC NEKFSYFPEM EVTQPTSNPV TPK | 72 |

EP 4 674 480 A2

# FIG. 2

| SEQ ID NO: | Construct | | | | | | | | | | | | |
|---|---|---|---|---|---|---|---|---|---|---|---|---|---|
| | Vehicle | | | | | | | | | | | | |
| SEQ ID NO: 73 (p) | ActRIIA | C LFFNANWEKD | RTNQTGVEPC | YGDKDKRRHC | FATWKNISGS | IEIVKQGCWL | DDINCYDRTD | CVEKKDSPEV | YFCCCEGNMC | | | | |
| SEQ ID NO: 74 (p) | ActRIIB | C IYYNANWELE | RTNQSGLERC | EGEQDKRLHC | YASWRNSSGT | IELVKKGCWL | DDFNCYDRQE | CVATEENPQV | YFCCCEGNFC | | | | |
| SEQ ID NO: 158 | ActRIIB/A | GRGEAETREC IFYNANWEKD | RTNQSGLEPC | YGDQDKRRHC | FASWKNSSGT | IELVKQGCWL | DDINCYDRQE | CVAKKDSPEV | YFCCCEGNFC | NERFTHLPEA | GGPEVTYEPP | PTAPT |
| SEQ ID NO: 159 | ActRIIBdelta9 | GRGEAETREC IYYNANWELD | RTNQSGLERC | EGEQDKRLHC | YASWRNSSGT | IELVKKGCWL | DDINCYDRQE | CVATKENPQV | YFCCCEGNFC | NERFTHLPEA | GGPEVTYEPP | PTAPT |
| SEQ ID NO: 160 | ActRIIB 2.01 | GRGEAETREC IYYNANWELE | RTNQSGLERC | EGEQDKRLHC | YASWRNSSGT | IELVKKGCWL | DDFNCYDRQE | CVAKKDSPEV | YFCCCEGNFC | NERFTHLPEA | GGPEVTYEPP | PTAPT |
| SEQ ID NO: 161 | ActRIIB 2.02 | GRGEAETREC IYYNANWELE | RTNQSGLERC | EGEQDKRLHC | YASWRNISGT | IELVKKGCWL | DDFNCYDRQE | CVAKKDSPEV | YFCCCEGNFC | NERFTHLPEA | GGPEVTYEPP | PTAPT |
| SEQ ID NO: 162 | ActRIIB 2.03 | GRGEAETREC IYYNANWELE | RTNQTGLERC | EGEQDKRLHC | YASWRNITGT | IELVKKGCWL | DDFNCYDRQE | CVAKKDSPEV | YFCCCEGNFC | NERFTHLPEA | GGPEVTYEPP | PTAPT |
| SEQ ID NO: 163 | ActRIIB 2.04 | GRGEAETREC IYYNANWELE | RTNQSGLEPC | EGEQDKRLHC | YASWRNSSGT | IELVKKGCWL | DDFNCYDRQE | CVAKKDSPEV | YFCCCEGNFC | NERFTHLPEA | GGPEVTYEPP | PTAPT |
| SEQ ID NO: 164 | ActRIIB 2.05 | GRGEAETREC IYYNANWELE | RTNQSGLERC | YGDKDKRLHC | YASWRNSSGT | IELVKKGCWL | DDFNCYDRQE | CVAKKDSPEV | YFCCCEGNFC | NERFTHLPEA | GGPEVTYEPP | PTAPT |
| SEQ ID NO: 165 | ActRIIB 2.06 | GRGEAETREC IYYNANWELE | RTNQSGLERC | EGEQDKRLHC | FASWKNSSGT | IELVKQGCWL | DDFNCYDRQE | CVAKKDSPEV | YFCCCEGNFC | NERFTHLPEA | GGPEVTYEPP | PTAPT |
| SEQ ID NO: 166 | ActRIIB 2.07 | GRGEAETREC IFYNANWEKD | RTNQSGLERC | EGEQDKRLHC | YASWRNSSGT | IELVKKGCWL | DDFNCYDRQE | CVAKKDSPEV | YFCCCEGNFC | NERFTHLPEA | GGPEVTYEPP | PTAPT |
| SEQ ID NO: 167 | ActRIIB 2.08 | GRGEAETREC IYYNANWELE | RTNQSGLERC | YGDQDKRRHC | YASWRNSSGT | IELVKKGCWL | DDFNCYDRQE | CVAKKDSPEV | YFCCCEGNFC | NERFTHLPEA | GGPEVTYEPP | PTAPT |
| SEQ ID NO: 168 | ActRIIB 2.09[3] | GRGEAETREC LYYNANWELE | RTNQSGVERC | EGEKDKRLHC | YASWRNSSGS | LEIVKKGCWL | DDFNCYDRTD | CVATEENPQV | YFCCCEGNMC | NERFTHLPEA | GGPEVTYEPP | PTAPT |
| SEQ ID NO: 169 | ActRIIB 2.10[3] | GRGEAETREC LYYNANWELE | RTNQSGVERC | EGEKDKRLHC | YASWRNSSGS | LEIVKKGCWL | DDFNCYDRDT | CVATEENPQV | YFCCCEGNMC | NERFTHLPEA | GGPEVTYEPP | PTAPT |

Blue = 11A residue into 11B    (p) = portion of sequence
Red = 11B into 11A
Green = novel change

# FIG. 3

```
Chimera 1                      GAILGRAETRECIYYNANWELERTNQSGLERCEGEQDKRLHCYASWRNSSGTIEIVKQGCWLDDINCYDRTDCVATEENPQVYFCCCEGNMCNEKFSYFPEMEVTQPTS
(SEQ ID NO: 195)
Chimera 2                      --GRGEAETRECIYYNANWELERTNQSGLERCEGEQDKRLHCYASWRNSSGTIEIVKQGCWLDDINCYDRTDCVEKKDSPEVYFCCCEGNMCNEKFSYFPEMEVTQPTS
(SEQ ID NO: 196)
Chimera 2 E81Q                 --GRGEAETRECIYYNANWELERTNQSGLERCEGEQDKRLHCYASWRNSSGTIEIVKQGCWLDDINCYDRTDCVEKKDSPQVYFCCCEGNMCNEKFSYFPEMEVTQPTS
(SEQ ID NO: 197)
Chimera 2 I65F_E81Q            --GRGEAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS
(SEQ ID NO: 198)
Chimera 1/2                    GAILGRAETRECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS
(SEQ ID NO: 213)
Chimera 1/2b                   GAILGRSETQECIYYNANWELERTNQSGLERCEGEQDKRLHCYASWRNSSGTIEIVKQGCWLDDFNCYDRTDCVEKKDSPQVYFCCCEGNMCNEKFSYFPEMEVTQPTS
(SEQ ID NO: 214)
Chimera 3                      --GRGEAETRECIYYNANWELERTNQSGLERCEGEQDKRRHCFATWKNISGSIEIVKQGCWLDDFNCYDRTDCVEKKDSPQVYFCCCEGNMCNEKFSYFPEMEVTQPTS
(SEQ ID NO: 201)
Chimera 4                      --GRGEAETRECIYYNANWELERTNQSGLERCEGEQDKRLHCYASWRNSSGTIELVKKGCWLDDFNCYDRQECVATKDSPEVYFCCCEGNMCNEKFSYFPEMEVTQPTS
(SEQ ID NO: 215)
Chimera 5                      --GRGEAETRECIYYNANWELERTNQSGLERCEGEQDKRLHCYASWRNSSGTIELVKKGCWLDDFNCYDRQECVATKENPQVYFCCCEGNMCNEKFSYFPEMEVTQPTS
(SEQ ID NO: 216)
Chimera I65E N66T
E81Q(SEQ ID NO: 210)          --GRGEAETRECIYYNANWELERTNQSGLERCEGEQDKRLHCYASWRNSSGTIEIVKQGCWLDDETCYDRTDCVEKKDSPQVYFCCCEGNMCNEKFSYFPEMEVTQPTS
```

ActRIIA          *ActRIIB*          ***Non-ActRIIA or ActRIIB Substitution***

EP 4 674 480 A2

FIG. 4

FIG. 5

**Erythropoietin**

*p<0.05; **p<0.01; ****p<0.0001

EP 4 674 480 A2

FIG. 6

*EpoR*

Day 4

*EpoR*

Day 7

*EpoR*

Day 14

EP 4 674 480 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5013718 A
- US 7745387 B
- US 8466172 B
- US 8729030 B
- US 10695402 B
- US 20180303903 A1
- US 20170312268 A1
- US 20060074225 A
- US 8216805 B
- US 5731168 A
- US 20120244578 A
- US 20140024111 A

### Non-patent literature cited in the description

- **TOWNSON et al.** *J. Biol. Chem.*, 2012, vol. 287, 27313
- **KABAT**. Sequences of Proteins of Immunological Interest. Public Health Service, National Institutes of Health, 1991
- **YING et al.** *J Biol Chem.*, 2012, vol. 287, 19399-19408
- **RIDGWAY et al.** *Protein Eng.*, 1996, vol. 9, 617-612
- **ATWELL et al.** *J Mol Biol.*, 1997, vol. 270, 26-35
- **MERCHANT et al.** *Nat Biotechnol.*, 1998, vol. 16, 677-681
- Recombinant Gene Expression: Reviews and Protocols (Methods in Molecular Biology). Humana Press, 2004
- Therapeutic Proteins: Methods and Protocols (Methods in Molecular Biology). Humana Press, 2012
- **WILSON et al.** *Cell*, 1984, vol. 37, 767
- Therapeutic Peptides and Proteins: Formulation, Processing and Delivery Systems. Taylor & Francis Group, CRC Press, 2015
- Remington: The Science and Practice of Pharmacy. 2012
- ASHP Handbook on Injectable Drugs. Toissel, 2014